(19) **Europäisches Patentamt European Patent Office Office européen des brevets**

(11)  **EP 4 017 281 B1**

(12)  **EUROPEAN PATENT SPECIFICATION**

(45) Date of publication and mention
of the grant of the patent:
**28.08.2024  Bulletin 2024/35**

(21) Application number: **20848776.9**

(22) Date of filing: **18.12.2020**

(51) International Patent Classification (IPC):
*A23K 50/40* (2016.01)    *A23K 50/42* (2016.01)
*A23K 50/45* (2016.01)    *A23K 50/48* (2016.01)
*A61P 3/10* (2006.01)

(52) Cooperative Patent Classification (CPC):
(C-Sets available)
**A23K 50/40; A23K 50/42; A23K 50/45;
A23K 50/48; A61P 3/10;** A23V 2002/00    (Cont.)

(86) International application number:
**PCT/US2020/070929**

(87) International publication number:
**WO 2021/127701 (24.06.2021 Gazette 2021/25)**

(54) **PET FOOD COMPOSITIONS**

HAUSTIERFUTTERMITTEL

ALIMENT POUR ANIMAUX DE COMPAGNIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **19.12.2019  US 201962950905 P**

(43) Date of publication of application:
**29.06.2022  Bulletin 2022/26**

(60) Divisional application:
**24189980.6**

(73) Proprietor: **Hill's Pet Nutrition, Inc.
Topeka, KS 66603 (US)**

(72) Inventors:
• **JACKSON, Matthew
Topeka, Kansas 66604 (US)**
• **WALDY, Christopher
Maple Hill, Kansas 66507 (US)**
• **OGLEBY, Blair
Topeka, Kansas 66617 (US)**
• **JEWELL, Dennis
Lawrence, Kansas 66049 (US)**
• **LINDECRANTZ, Jason
Topeka, Kansas 66605 (US)**
• **MONTELONGO, Luis J.
Lawrence, Kansas 66047 (US)**

(74) Representative: **Wichmann, Hendrik
Wuesthoff & Wuesthoff
Patentanwälte und Rechtsanwalt PartG mbB
Schweigerstraße 2
81541 München (DE)**

(56) References cited:
**US-A1- 2010 292 330    US-A1- 2011 081 443
US-A1- 2019 134 132    US-A1- 2019 373 917
US-A1- 2020 375 934**

(52) Cooperative Patent Classification (CPC): (Cont.)

C-Sets
A23V 2002/00, A23V 2200/32, A23V 2200/328,
A23V 2250/1944, A23V 2250/51

**Description**

**BACKGROUND**

High-Protein, low carbohydrate food composition having non-fermentable fiber is disclosed in U.S. Patent Application Publication No. US 2011/0081443 A1. A food composition was provided having high protein levels, high fat levels, low carbohydrate levels and non-fermentable fiber. Methods of using these compositions for weight management in mammals were also provided.

Nutrition blend for health benefits in animals was disclosed in U.S. Patent Application Publication No. 2019/0134132. The document taught a method of minimizing fat accumulation in a growing animal without limiting caloric intake or preventing or treating obesity in an animal, the method comprising orally administering a ketogenic diet and a nutrient blend to the animal. The nutrient blend can include at least four nutrients selected from the group consisting of walnut, maitake mushroom extract, EGCG, turmeric root powder, lycopene, taurine, EPA, and DHA to the animal.

The development of dietary therapy in cancer is discussed in U.S. Patent Application Publication No. 2018/0214410 to Hagihara et al ("Hagihara"). Hagihara provides new compositions or combinations thereof for the treatment of cancer. More specifically, Hagihara provides compositions or combinations thereof for cancer treatment including a high-fat diet. Specifically, the high-fat diet is characterized by having approximately 120 g/day or more, or approximately 70% or more of the total daily energy, from fat, based on a real bodyweight of 50 kg. The diet is preferably a carbohydrate-restricted high-fat diet, and more preferably provided by a ketone formula and/or MCT oil. The dietary therapy by a high-fat diet of the present disclosure is provided along with surgical treatment, chemotherapy or radiation therapy, or combinations thereof.

A composition for increasing ketone bodies in an animal, wherein the composition comprises about 1-15% medium-chain triglycerides, about 30-70% protein, about 20-40% fat and about 10-25% carbohydrates is described in US 2019/0373917 A1. A composition comprising long chain polyunsaturated fatty acids, nitric oxide releasing compounds and medium-chain triglycerides as well as the use of this composition for enhancing cognitive function is described in US 2010/0292330 A1. A composition comprising a therapeutically effective amount of medium chain triglycerides including caprylic acid and the use of this composition for achieving weight loss in an animal are described in US 2020/375934 A1.

Objects and method of diet adjustment in diabetes was discussed by R. T. Woodyatt in Arch Intern Med (Chic). 1921; vol. 28, iss. 2, pp. 125 to 141. In the dietetic management of diabetes the author was engaged in the effort to correlate symptoms and signs shown by the patient with the kinds and quantities of food he consumes. The success of treatment, the average of results in all types of cases, depends on the truth of our concept of the relationship existing between symptoms or signs and the food supply. During the previous few years the average of results obtained in the dietetic management of diabetes has been improved greatly through the work of Allen and Joslin, and the system they have developed is in some respects more logical and less empirical than any we have had heretofore. Yet the literature of the subject is still confused by a lack of unanimity among all writers as to the best manner of handling all cases.

A ketogenic diet is employed to reduce seizures in both dogs and humans A hallmark of current implementations of a ketogenic diet is a paucity of dietary fiber. Increased dietary fiber is associated with reductions in numerous maladies including cardiovascular disease and cancer. However, current implementations of the ketogenic diet come with stark warnings about possible detrimental consequences to the gut microbiome. For seizure remission, this is completely counterproductive. The mammalian gut microbiome mediates the effects of ketogenic diet to reduce seizures such that the ketogenic diet has reduced efficacy when a healthy, functioning microbiome is not present in the subject (The Gut Microbiota Mediates the Anti-Seizure Effects of the Ketogenic Diet. Cell. 2018 Jun 14; 173(7): 1728-1741. Thus, current ketogenic diet compositions are self-limiting in their efficacy for seizures. A better composition of the ketogenic diet would include fermentable and non-fermentable, as well as soluble and insoluble fibers to promote a healthy gut microbiome and allow for development of the full benefit of the ketogenic diet for seizure reduction.

Lipolytic activity of human gastric and duodenal juice against medium and long-chain triglycerides is discussed by M. Cohen, R. G. H. Morgan, and A. F. Hofmann in Gastroenterology 1971, vol. 60, iss. 1, pp. 1 to 15. The presence of lipase in gastric aspirates containing less than 3 % contamination with duodenal reflux (as determined by tests for bile acid) was demonstrated in all samples of apparently normal gastric juice from 73 subjects when assayed with a trioctanoin substrate emulsified in sodium taurodeoxycholate and buffered at pH 6. This enzyme, in contrast to lipase(s) in duodenal aspirate, was stable at pH 2, had a lower pH optimum, was rapidly inactivated by trypsin even in the presence of bile acid, and was moderately inhibited by added fatty acid. Like duodenal lipase(s), gastric lipase had greater activity against short-chain than long-chain triglycerides and was more active against the fatty acids in the 1 than in the 2 positions of triglyceride. It had a much smaller apparent molecular weight (40,000 to 50,000) than duodenal lipase (>500,000) by gel filtration chromatography and had only moderate esterifying properties compared with duodenal lipase(s). Synthetic triglycerides were cleaved more slowly by gastric lipase than by pancreatic lipase in pancreatic fistula juice or duodenal content. Triglycerides of human milk particles were cleaved by gastric lipase and lipases present in duodenal content, but not by pancreatic fistula juice which lacked bile acid, suggesting that milk particle triglyceride is resistant to pancreatic lipase unless bile acid is present. In healthy adults, the concentration of gastric lipase in gastric contents was much less than that of pancreatic lipase in duodenal contents, and gastric lipase did not contribute significantly to the lipolytic activity of duodenal content after a test meal. Gastric lipase was significantly decreased in the gastric contents of 3 achlorhydric patients. Medium chain triglyceride was not cleaved by 16 hr of incubation at pH 1.8 or 1 hr of incubation at pH 1, suggesting that "acid hydrolysis" does not occur. Gastric lipase probably contributes to digestion of milk triglyceride in infants, as well as to hydrolysis of administered medium chain triglyceride, especially in children with decreased pancreatic lipase concentrations. Its limited activity against long chain triglyceride suggests that gastric lipase has little role in normal fat digestion in adults.

Ketogenic response to medium-chain triglyceride load in the rat was disclosed by A. Bach et al. in J. Nutr. 1977, vol. 107, iss. 10, pp. 1863 to 1870. The authors studied ketonemia induced in rats by a single oral load of medium-chain triglycerides (MCT) (C8:0 50.5%, C10:0 48.0%, C12:0 1.0%). Medium-chain fatty acids, rather than being incorporated into the lipids synthesized by the liver, are oxidized there, with high production of ketone bodies. Severe and long-lasting hyperketonemia developed rapidly. With increased MCT loads, ketonemia also increased, although not linearly. The level of the hyperketonemia seemed equal in the two sexes. Ingestion of MCT by fasting rats caused an additional rise in ketonemia. Long-chain triglycerides were not ketogenic since their constituent fatty acids are incorporated into lipids and are thus less subject to oxidation. Lipids induce less severe ketonemia in genetically obese rats than in normal-weight rats.

Gut microbiota, metabolic health, and the potential beneficial effects of a medium chain triglyceride diet in obese individuals is discussed by S. A. Rial et al. in Nutrients 2016 vol. 8, iss. 5, p 281. Obesity and associated metabolic complications, such as non-alcoholic fatty liver disease (NAFLD) and type 2 diabetes (T2D), are in constant increase around the world. While most obese patients show several metabolic and biometric abnormalities and comorbidities, a subgroup of patients representing 3% to 57% of obese adults, depending on the diagnosis criteria, remains metabolically healthy. Among many other factors, the gut microbiota is now identified as a determining factor in the pathogenesis of metabolically unhealthy obese (MUHO) individuals and in obesity-related diseases such as endotoxemia, intestinal and systemic inflammation, as well as insulin resistance. Interestingly, recent studies suggest that an optimal healthy-like gut microbiota structure may contribute to the metabolically healthy obese (MHO) phenotype. Medium chain triglycerides (MCT), can ameliorate metabolic disease via their capacity to improve both intestinal ecosystem and permeability. MCT-enriched diets could, therefore, be used to manage metabolic diseases through modification of gut microbiota.

Functional alterations of human neutrophils by medium-chain triglyceride emulsions, evaluation of phagocytosis, bacterial killing, and oxidative activity are discussed by R. Bellinati-Pires in J. Leukoc. Biol. 1993, vol 53, iss. 4, pp 404 to 410. Medium-chain triglyceride (MCT) and long-chain triglyceride (LCT) emulsions currently used in nutritional therapy were evaluated for their in vitro effect on neutrophil oxidative metabolism, phagocytosis, and bacterial killing activities. Neutrophils from healthy adult male volunteers were assessed after blood incubation with commercially available fat emulsions containing LCT, MCT, or a mixture of 50% MCT and 50% LCT at a final triglyceride concentration of 20 mg/ml. It was observed that MCTcontaining emulsions stimulated nitroblue tetrazolium (NBT) dye reduction by neutrophils as determined by a cytochemical NBT test performed directly on whole blood. This effect was dose-dependent. However, after lipid removal by cell washing, the MCT-treated neutrophils showed decreased production of hydrogen peroxide ($H_2O_2$) and NBT reduction in response to bacterial lipopolysaccharide or phorbol myristate acetate stimuli as well as impaired phagocytosis and killing of *Staphylococcus aureus.* In contrast, the LCT emulsion did not alter any of the neutrophil functions evaluated. The present data suggest that MCTs elicit the oxidative metabolism of neutrophils, probably by phagocytosis of fat particles and, depending on the lipid concentration, this effect may not be reversible, leading to impairment of the cellular response to subsequent membrane stimuli.

The function of capric acid in cyclophosphamide-induced intestinal inflammation, oxidative stress, and barrier function in pigs are discussed by S. I. Lee and K. S. Kang. 2017. Scientific Reports vol. 7, 16530. The small intestine is not only critical for nutrient absorption, but also serves as an important immune organ. Medium-chain fatty acids have nutritional and metabolic effects and support the integrity of the intestinal epithelium. However, their roles in intestinal immunity in pigs are not fully understood. The authors investigated the effects of a medium-chain fatty acid, capric acid, on intestinal oxidative stress, inflammation, and barrier function in porcine epithelial cells and miniature pigs after treatment with the immune suppressant cyclophosphamide. Capric acid alleviated inflammatory cytokine production (TNF-$\alpha$ and IL-6) and related gene expression (NF-$\kappa$B, TNF-$\alpha$, IFN-$\gamma$), alleviated oxidative stress (GSSG/GSH ratio, H2O2, and malondialdehyde), and increased oxidative stress-related gene expression (SOD1 and GCLC) in cyclophosphamide-treated IPEC-J2 cells. The permeability of FD-4 and expression of ZO-1 and OCLN in cyclophosphamide-treated IPEC-J2 cells were reduced by capric acid. Dietary capric acid reduced TNF-$\alpha$, IL-6, and MDA levels and increased SOD, GPx, and the expression of genes related to pro-inflammatory, oxidative stress, and intestinal barrier functions in cyclophosphamide-treated miniature pigs. These results revealed that capric acid has protective effects against cyclophosphamide-induced small intestinal dysfunction in pigs.

Effects of dietary caprylic and capric acids on piglet performance and mucosal epithelium structure of the ileum have been discussed by E. Hanczakowska, A. Szewczyk, and K. Okon in *J. Anim. Feed Sci.* 2011, vol. 20, iss. 4, pp. 556 to 565. Effects of a diet supplemented with caprylic and/or capric acid on piglet performance, apparent digestibility of nutrients, intestinal microflora and small intestine (ileum) structure were investigated. The experiment was performed on 252 piglets (24 litters) allocated to 4 experimental groups (6 litters each). The animals were fed with a standard feed mixture (control) or the same mixture supplemented with 2 g of caprylic or capric acid (groups $C_8$ and $C_{10}$, respectively) per 1 kg of feed. Group $C_8+C_{10}$ received 1 g of caprylic and 1 g of capric acid. Apparent digestibility was estimated using $Cr_2O_3$ as an indicator, while microbiological analyses were performed using standard agar plates. The short-chain fatty acid (SCFA) content of the ileum and caecum digesta was analysed using Varian 340 analyzer. The piglets receiving caprylic or capric acids grew significantly (P<0.01) faster than the control ones (average daily gains during the whole experiment, i.e., between days 1 and 84 of age, were: 288, 269, 278 and 234 g, respectively). The best feed utilization (1.3 kg per kg) was found in animals receiving caprylic acid. The acids also lowered piglet mortality, while significantly increased protein digestibility (P<0.01) and, to a lesser degree (P<0.05), also fibre digestibility. There was no significant difference in acidity of the digesta between control and experimental groups. Capric acid increased the amount of aerobic bacteria as compared to the control group, but the amount of *Escherichia coli* remained unchanged. The population of *Clostridium perfringens* was reduced by both caprylic and capric acids (P<0.01). Acids had no effect on SCFA content of the ileum but lowered the acetic acid content of the caecum digesta. Capric acid had the strongest effect on villi, which were significantly higher (306 $\mu$m) than in the control group (233 $\mu$m). Differences in crypt depth were smaller but the crypts were also the deepest in piglets receiving capric acid. The results suggest that caprylic and capric acids added to the feed improve piglet performance, probably due to positive changes in the mucosal epithelium structure of the ileum.

Despite decades of production of companion animal dry foods in the commercial and research space, there are no recorded instances of a kibble, treat, or other diet meeting the criteria of ketogenic diets. Namely, a macronutrient composition exhibiting a ketogenic ratio of greater than or equal to about 1.5. Further, there are no published reports showing the relative levels of ketogenicity of state of the art ketogenic commercial dry kibbles. The ketogenic ratio (KR) is a ratio of the sum of ketogenic factors to the sum of antiketogenic factors (KR = K/AK) and was defined in the early 1900s as a rigorous, evidence based approach to determining the expected ketogenicity of a food. *Zilberter T, Zilberter Y.* Ketogenic Ratio Determines Metabolic Effects of Macronutrients and Prevents Interpretive Bias. Frontiers in Nutrition. 2018; 5:75. Calculations based on analytical data show instances of commercial products claiming or marketing to be ketogenic in fact fail to meet the scientific and medical basis for ketogenic, typically due to their high protein levels. Further, measurements of circulating blood ketones in dogs who'd consumed these state of the art commercial ketogenic foods did not manifest particularly high levels of ketones (e.g. D-beta-hydroxybutyrate; BHB) when compared to values available in published literature. It should be noted, however, that available historical examples predate the appearance of ketogenic commercial dry kibbles and typically employed a period of fasting followed by a period of feeding non-kibble semi-liquid fat feeding. Lathan A. Crandall, Jr. A Comparison Of Ketosis In Man And Dog. J. Biol. Chem. 1941, 138:123-128. Thus, current ketogenic commercial dry pet food kibbles would appear to be less than optimally ketogenic by not generating higher circulating blood BHB ketone levels. It should be noted that the ketogenic commercially available diets do not contain ketogenic fatty acids, such as medium chain triglycerides. Additionally, it should be noted that dogs are known to be particularly resistant to ketosis as a species when compared to humans. *Id.* The resistance to ketosis exhibited in canines further hinders the ability of conventional or state of the art ketogenic commercial pet food compositions to optimally induce ketosis in canines. In view of the foregoing, one having ordinary skill in the art would understand that they cannot merely or simply transfer ketogenic diet recommendations readily from humans to companion animals or pets; and thus, separate and custom solutions need to be developed that may be non-obvious and/or counter-intuitive.

Further, such diets lack sufficient dietary fiber to support gut health. Finally, such diets contain high levels of inorganic ash, specifically phosphorus, which can be harmful to renal health.

Although many advances in the art of formulating pet food composition have been made with respect to improving its ability to treat diseases, many more challenges remain. More specifically, there is an art recognized need for ketogenic pet food compositions, which are resistant to crumbling, palatable, and allow for increased inclusion of fat levels in the composition in order to better achieve ketosis in pet species known to be resistant to this physiological state. In this regard, delivery of a ketogenic pet food composition as a dry extruded kibble is known to be difficult to those skilled in the art of dry kibble extrusion. In dry kibble extrusion, use of starch-containing ingredients, including but not limited to grains, legumes and tubers, in the composition provides not only a source of dietary energy as carbohydrate, but also fulfills three critical non-nutritional roles that ensure a viable dry kibble product can and will be consumed by an end user. First, starch is viewed as an essential binding agent that allows for a dry kibble to be produced with sufficient durability that the kibble may be transferred through commercial production processes to end users without excess breakage and loss (termed "fines" in the art). Second, starch is used to ensure appropriately expanded foam cell structure in dry kibbles, which provides a pleasing aesthetic to pets and increases overall palatability. Third, the larger foam cell structure in dry kibble enabled by inclusion of starch also allows for accommodation of increased inclusion of topically added coatings (e.g. fats, palatants) through the increased surface area. An ideal solution would improve upon existing disclosed state of the art ketogenic pet food dry kibble by not only delivering enhanced ketogenicity but also by delivering enhanced or improved: 1) Durability, 2) Palatability, and 3) Fat Inclusion.

Embodiments of the present disclosure are designed to meet these and other needs.

## BRIEF SUMMARY

The present disclosure is directed to a pet food composition and medical uses thereof as defined in the claims. The composition comprises greater than 33 wt% fat, a protein source, less than 7 wt% carbohydrates, and greater than 10 wt% fiber, and has a ketogenic ratio defined as (0.9 F + 0.46 P) / (0.1 F + 0.58 P + C) of 1.5 or greater, wherein F is wt% fat, P is wt% protein and C is wt% carbohydrates. The composition can provide complete and balanced nutrition to a companion animal. The composition can further have the following characteristics: a ketogenic ratio greater than or equal to about 1.7, low levels of digestible carbohydrate, and 20% of the present fat is in the form of medium chain triglycerides. Further, under one embodiment, the composition comprises moderate protein on a per kcal basis. Further, under one embodiment, the composition comprises high microbiome-friendly fiber. Still further, under one embodiment, the composition comprises a low level of ash or a low level of phosphorus, or both.

The composition results in marked improvements over commercially available products by combining dietary ingredients that are not typically used together and overcoming significant technical challenges in the process.

Under one embodiment, the composition of the present disclosure is an adult canine or feline dry maintenance kibble has a protein level of 7.5 g/100 kcal ME or less, at least 10 wt%, at least 14 wt%, or at least 16 wt.% fiber on a dry matter basis, 6 wt% or less, 5 wt% or less, or 3.5 wt.% or less of digestible starch and/or sugar, at least 35% wt. fat on a dry matter basis, at least 20% of fat as medium chain triglycerides, 6 wt.% or less of ash, 0.7 wt.% or less of phosphorus, and a ketogenic ratio (KR) greater than 1.7

The formulations for the dry kibble and the treat forms utilize a strategic combination of proteins and fibers.

Under one embodiment, the composition of the present disclosure is a dry kibble or a treat wherein functional proteins and fibers reduce nitrogen burden on the lower gastrointestinal tract as well as feeds and nourishes the gut microbiome. Adherence to a low ash profile furthers the benefits of this reduction to practice. The ketogenic ratio KR of such a composition is greater than 1.5, which is higher than any commercially available companion animal diet.

The composition of the present disclosure comprises fat, or fats. Fats are one of the three main macronutrients, along with carbohydrates and proteins. Fat molecules consist of primarily carbon and hydrogen atoms and are therefore hydrophobic and are soluble in organic solvents and insoluble in water. Under one embodiment, the composition of the present disclosure comprises more than about 35 wt.% fat, or more than about 42 wt.% fat.

The fat is comprised of at least 20 wt.% medium chain triglycerides. Alternatively, the fat of the dietary composition is comprised of from about 10 wt.% to about 35 wt.% medium chain triglycerides. Triglyceride is an ester derived from glycerol and three fatty acids. The phrase "medium chain triglycerides" refers to triglycerides of fatty acid being 6 to 12 carbon atoms in length, including caproic acid, caprylic acid, capric acid, and lauric acid.

The composition of the present disclosure comprises protein. Crude protein may be supplied by any of a variety of sources known by those skilled in the art, including plant sources, animal sources, or both. Animal sources include, for example, meat, meat by-products, seafood, dairy, eggs, etc.

Under one embodiment, the composition of the present disclosure comprises from about 4.5 g to about 14 g of protein per 100 kcal metabolizable energy, about 6 g to about 12 g of protein per 100 kcal metabolizable energy, or from about 7 g to about 10 g of protein per 100 kcal metabolizable energy. In another embodiment, the composition of the present disclosure comprises about 4.5 g to 7.5 g of protein per 100 kcal metabolizable energy. The claimed compositions may comprise a metabolizable energy content of from about 3000 to about 5000 kcal/kg.

One of the ingredients of the dietary composition of the present disclosure is carbohydrate, including polysaccharides and sugars. Under one embodiment, the composition of the present disclosure comprises less than about 3.5 wt.% carbohydrates.

One of the ingredients of the dietary composition of the present disclosure is dietary fiber. Dietary fiber refers to components of a plant that are resistant to digestion by an animal's digestive enzymes. Dietary fiber, or total dietary fiber, consists of insoluble fiber and soluble fiber.

The present disclosure is also directed to a dietary composition for a companion animal comprising greater than 35 wt.% of fat, protein, less than 3.5 wt.% of carbohydrates, and at least 14 wt%, or at least 16 wt.% of fiber, wherein the ketogenic ratio is greater than 1.5.

The ketogenic ratio (KR) is a ratio of the sum of ketogenic factors to the sum of antiketogenic factors: KR = K/AK. The ketogenic ratio is defined by the formula

$$ KR = (0.9\,F + 0.46\,P) \,/\, (0.1\,F + 0.58\,P + C) $$

wherein F is grams of fat; P is grams of protein and C is grams of carbohydrate.

Under one embodiment, the ketogenic ratio of the composition of the present disclosure is greater than about 1.5.

In some embodiments, the compositions described herein comprise from about 30 wt.% to about 60 wt.% protein. In other embodiments, the compositions described herein comprise from about 32.5 wt.% to about 57.5 wt.% protein. In further embodiments, the compositions described herein comprise from about 35 wt.% to about 55 wt.% protein. In certain embodiments, the compositions described herein comprise from about 37.5 wt.% to about 52.5 wt.% protein. Yet other embodiments provide compositions comprising from about 40 wt.% to about 50 wt.% protein. In some embodiments, the protein comprises animal protein, plant protein or a combination thereof.

The dietary composition for a companion animal may also further comprise additional ingredients. Micronutrients occurring in the composition of the present disclosure include trace minerals, vitamins, related compounds and sources thereof, at levels that are recommended or acceptable for companion animals.

Under one embodiment, the composition of the present disclosure comprises less than about 6 wt.% ash.

Under one embodiment, the composition of the present disclosure comprises a low level of phosphorus. Reducing the level of dietary phosphorus has been shown to slow progression of kidney disease and prolong life. Under one embodiment, the composition of the present disclosure comprises less than about 0.7 wt.% of phosphorus.

The food compositions may be prepared in a canned or wet form using conventional food preparation processes known to skilled artisans. The food compositions may be prepared in a dry form using conventional processes known to skilled artisans. Typically, dry ingredients such as animal protein, plant protein, grains, and the like are ground and mixed together. Moist or liquid ingredients, including fats, oils, animal protein, water, and the like are then added to and mixed with the dry mix. The mixture is then processed into dry food pieces. Alternatively or additionally, the non-water wet ingredients can be coated onto the surface of the food pieces after drying via methods that may or may not involve application of a reduced pressure system (e.g. vacuum).

The food compositions can be in any form useful for feeding the composition to the companion animal, e.g., kibbles, treats, and toys for animal food. Kibbles are generally formed using an extrusion process in which the mixture of dry and wet ingredients is subjected to mechanical work at high pressure and temperature and forced through small openings and cut off into kibble by a rotating knife. The dried kibble is then dried and optionally coated with one or more topical coatings such as flavors, fats, oils, powders, and the like.

The dietary composition of the present disclosure can be useful for improving the health of a companion animal by feeding the animal a pet food comprising an effective amount of the dietary composition. The ketogenic diet pet food is applicable to several indications or conditions, including, but not limited to, cancer, inflammatory bowel disease, diabetes, obesity, hyperglycemia, hypertriglyceridemia, chronic inflammation, antibiotic resistance of the gut microbiome, or pathogenic dysbiosis of the gut microbiome, neurological seizures, high blood glucose, metabolic stress, or combinations thereof. The administration of the pet food comprising the dietary composition shifts the animal's metabolism from glucose-converting metabolism to fat-burning metabolism.

In the first aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively.

In the second aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively.

In the third aspect, the disclosure is directed to a dietary composition for a companion animal comprising greater than about 35 wt.% fat, protein, less than about 1.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively.

In the fourth aspect, the disclosure is directed to a dietary composition for a companion animal comprising greater than about 43 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively.

In the fifth aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, and wherein the fat is comprised of at least 20 wt.% medium chain triglycerides.

In the sixth aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, and wherein the fat is comprised of from about 10 wt.% to about 35 wt.% medium chain triglycerides.

In the seventh aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, comprising between about 4.5 g and 75 g of protein per 1000 kcal metabolizable energy.

In the eighth aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, and comprising less than 6 wt.% ash.

In the ninth aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, and further comprising less than 3 wt.% of ash.

In the tenth aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, further comprising less than 0.7 wt.% of phosphorus.

In the eleventh aspect, the disclosure is directed to a dietary composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, further comprising less than 0.3 wt.% of phosphorus.

In the twelfth aspect, the disclosure is directed a nutritionally complete pet food comprising the composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively.

In the thirteenth aspect, the disclosure is directed a nutritionally complete pet food comprising the composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, wherein the pet food is a wet pet food.

In the fourteenth aspect, the disclosure is directed a nutritionally complete pet food comprising the composition for a companion animal comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, wherein the pet food is a dry pet food.

In the fifteenth aspect, the disclosure is directed to a dietary composition for use in a method of treating high blood glucose, chronic inflammation, metabolic stress, or cancer, in a companion animal, the method comprising administering to the companion animal a pet food comprising an effective amount of the dietary composition comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively.

In the sixteenth aspect, the disclosure is directed to a dietary composition for use in a method of treating high blood glucose, chronic inflammation, metabolic stress, or cancer, in a companion animal, the method comprising administering to the companion animal a pet food comprising an effective amount of the dietary composition comprising greater than about 35 wt.% fat, protein, less than about 3.5 wt.% carbohydrates, and at least 14 wt%, or at least 16 wt.% fiber, wherein (0.9 F + 0.46 P) / (0.1 F + 0.58 P + C) is greater than 1.5, wherein F, P, and C, are wt.% of fat, protein, and carbohydrates, respectively, wherein the dietary composition is a nutritionally complete dog food.

## DETAILED DESCRIPTION

For illustrative purposes, the principles of the present disclosure are described by referencing various exemplary embodiments thereof. The terminology used herein is for the purpose of description and not of limitation.

As used herein and in the appended claims, the singular forms "a", "an", and "the" include plural references unless the context dictates otherwise. The singular form of any class of the ingredients refers not only to one chemical species within that class but also to a mixture of those chemical species; for example, the term "MCFA" in the singular form, may refer to a mixture of compounds each of which is also considered an MCFA. The terms "a" (or "an"), "one or more" and "at least one" may be used interchangeably herein. The terms "comprising", "including", and "having" may be used interchangeably. The term "include" should be interpreted as "include, but are not limited to". The term "including" should be interpreted as "including, but are not limited to".

The abbreviations and symbols as used herein, unless indicated otherwise, take their ordinary meaning.

The symbol "cP" means centipoise or millipascal-seconds. The symbol "kcal" means a kilocalorie or about 4182 J. The symbol "°" refers to a degree, including a degree of an angle and degree of Celsius.

The abbreviations "AAFCO" means the "Association of American Feed Control Officials; "NRC" means Nutrition Research Council; "ME" means metabolizable energy; "KR" means ketogenic ratio; "DMB" means dry matter basis; "MCT" means medium chain triglycerides; "MCFA" means medium chain fatty acids; and "GMO" means genetically modified organism.

The abbreviation "wt.%" means percent by weight.

The term "about" when referring to a number means any number within a range of 10% of the number. For example, the phrase "about 35 wt.%" refers to a number between and including 31.5000 wt.% and 38.5000 wt.%.

As used herein, the term or expression "digestible carbohydrate" (hereafter, "carbohydrate") may refer to any dietary monosaccharide known to provide energy calories after absorption, and further to any polymer of sugars such as oligo- or polysaccharides including starch that is capable of being hydrolyzed by mammalian digestive saccharolytic enzymes.

As used throughout, ranges are used as shorthand for describing each and every value that is within the range. Any value within the range can be selected as the terminus of the range. Thus, the phrase such as "greater than about 35 wt.%" includes the value of 35 wt.%.

The phrase "a companion animal" refers to a domesticated or domestic-bred animal whose physical, emotional, behavioral and social needs can be readily met as companions in a home, or in close daily relationships with one or more humans. Under one embodiment, species included in the definition of a companion animal include dogs, canines, cats, felines, horses, rabbits, ferrets, guinea pigs, and select other small mammals. Under another embodiment, species included in the definition of a companion animal include dogs, cats, horses, rabbits, ferrets, guinea pigs, and select other small mammals, birds, small reptiles, fish, and domestic-bred farm animals.

The definition of the term "dog" includes a companion dog, a guard dog, a hunting dog, a herding dog, and a working dog.

The definition of the term "cat" includes a domestic cat, *Felis catus,* and *Felis silvestris catus.* The definition of the term "cat" includes a house cat and a feral cat. The terms "feline" and "cat" may be used interchangeably herein.

The phrase "adult pet" as used herein refers to a subset of "pet" and includes, for example, domesticated dogs (canines) and cats (felines) that are between about 3 years old and about 8 years old.

The phrase "senior pet" as used herein refers to a subset of "pet" and includes, for example, domesticated dogs (canines) and cats (felines) that are about 9 years old and above.

The phrase "dietary composition" refers to food for consumption by a companion animal, or to food for consumption by a dog. This phrase is to be interpreted broadly; the phrase includes food that is consumed by the companion animal or by the cat on exclusive basis, food that is consumed by the companion animal or by the dog on regular basis, food by the companion animal or by the dog consumer on occasional basis, and food by the companion animal or by the dog consumer on rare basis.

Any member in a list of species that are used to exemplify or define a genus may be mutually different from, or overlapping with, or a subset of, or equivalent to, or nearly the same as, or identical to, any other member of the list of species. Further, unless explicitly stated, such as when reciting a Markush group, the list of species that define or exemplify the genus is open, and it is given that other species may exist that define or exemplify the genus just as well as, or better than, any other species listed.

In the event of a conflict in a definition in the present disclosure and that of a cited reference, the present disclosure controls.

In one embodiment, the pet food composition of the present disclosure is o a composition that provides complete and balanced nutrition to a companion animal, and has the following further characteristics: a ketogenic ratio greater than or equal to 1.7, low levels of digestible carbohydrate, and 20% of the present fat is in the form of medium chain triglycerides. Further, under one embodiment, the composition comprises moderate protein on a per kcal basis. Further, under one embodiment, the composition comprises high levels of a blend of fibers known to promote a healthy microbiome of the sort which allows for full expression of the benefits of the ketogenic diet. Still further, under one embodiment, the composition comprises a low level of ash or a low level of phosphorus or both.

The composition of the present disclosure comprises a blend of fibers and proteins that is designed to largely replace starch in a dry food form for companion animals.

One advantage of the composition of the present disclosure is that the composition exceeds the scientifically and medically validated formal definition of a ketogenic diet while offering characteristics not found in any existing ketogenic companion animal dry kibble and treats.

The composition results in marked improvements over commercially available products by combining dietary ingredients that are not typically used together and overcoming significant technical challenges in the process.

Under one embodiment, the composition of the present disclosure is an adult canine or feline dry maintenance kibble has a protein level of 7.5 g/100 kcal ME or less, at least 10 wt%, at least 14 wt%, or at least 16 wt.% fiber on a dry matter basis, 6 wt% or less, 5 wt% or less, or 3.5 wt.% of digestible starch and/or sugar, at least 35 wt.% fat on a dry matter basis, at least 20% of fat as medium chain triglycerides, 6 wt.% or less of ash, 1 wt% or less, 0.8 wt% or less, 0.7 wt.% or less, or 0.6 wt% or less of phosphorus, and a ketogenic ratio (KR) greater than 1.7 with all nutrients scaled to AAFCO and NRC levels on a ME density basis.

Under one embodiment, the composition of the present disclosure is a canine or feline dry maintenance kibble having a protein level of 7.5 g/100 kcal ME or less, at least 15 wt.% fiber on a dry matter basis, 6 wt% or less, 5 wt% or less, or 3.5 wt.% or less of digestible starch and/or sugar, at least 34 wt.% fat on a dry matter basis, at least 20% of fat as medium chain triglycerides, 7 wt.% or less of ash, 1 wt% or less, 0.8 wt% or less, 0.7 wt.% or less, or 0.6 wt% or less phosphorus, and a ketogenic ratio (KR) greater than 1.5 with all nutrients scaled to AAFCO and NRC levels on a ME density basis.

Under one embodiment, the composition of the present disclosure is a canine or feline dry baked treat having a protein level of less than 7.0 g/100 kcal ME, at least 10 wt%, at least 14 wt%, or at least 16 wt.% fiber on a dry matter basis, less than 1.5% digestible starch and/or sugar, more than 43% fat on a dry matter basis, more than 20% of fat as medium chain triglycerides, less than 6% ash, 1 wt% or less, 0.8 wt% or less, 0.7 wt.% or less, or 0.6 wt% or less phosphorus, and a ketogenic ratio (KR) greater than 2.0.

The formulations for the dry kibble and the treat forms utilize a strategic combination of proteins and fibers. The formulation employs an extensive blend of proteins that serve at least two different purposes. Firstly, the proteins are highly digestible and lead to a low nitrogen burden on the lower gastrointestinal tract. Secondly, these proteins have food production functional characteristics that aid in the formation of an aesthetically pleasing, low-starch dry kibble.

Similarly, the formulation also employs an extensive blend of fibers that serve at least two purposes. Firstly, the fibers are of diverse types which bypass digestion and nourish the gut microbiome, while concurrently providing plant polyphenols to activate the metabolism of this microbiome. These fibers and polyphenols have been shown to increase the types of bacteria which can promote the anti-seizure efficacy of a ketogenic diet (e.g. Akkermansia and Parabacteroides). Secondly, these fibers have food production functional characteristics that aid in the formation of an aesthetically pleasing, low-starch dry kibble.

The formulation is controlled for protein levels, which, alongside the use of highly digestible protein sources mentioned herein, aids in reducing nitrogen burden on the lower gastrointestinal tract. Ketogenic foods are often employed for healthy body weight management. Despite the fact that the composition of the present disclosure has 37 wt.% (DMB) protein, which is more protein than a typical exemplary commercially available weight loss canine food, the protein level is identical to this exemplary food when assessed on a grams per metabolized energy basis. The composition of the present disclosure contains 37.2 g/100 g protein DMB, 8.2 g/100 kcal ME, KR = 1.7; whereas a commercially available comparative diet contains 28.4 g/100 g protein DMB, 8.2 g/100 kcal ME, but KR = 0.4. Thus, when a companion animal fed to the same level of kilocalories of the diet, there is only a moderate amount of protein in the food. Compared to the composition of the present disclosure, commercially available examples of companion animal food utilize excessively high protein levels in their foods.

The ketogenic ratio, KR, is defined by the formula

$$KR = (0.9 \times \text{Fat\%} + 0.46 \times \text{Protein\%}) / (\text{Carb\%} + 0.58 \times \text{Protein\%} + 0.1 \times \text{Fat\%})$$

wherein Fat% is the weight percent of fat in a diet, Protein% is the weight percent of protein in the diet, and Carb% is the weight percent of carbohydrates. This equation may be rewritten as

$$KR = (0.9\,F + 0.46\,P) / (0.1\,F + 0.58\,P + C)$$

wherein F, P, and C, are wt.% of fat, protein, and carbohydrates, respectively.

The equation for ketogenic ratio, from which a scientifically and medically validated ketogenic status is derived, shows that increasing the protein level reduces the overall KR. This is evident since the cofactor for protein in the denominator is greater than the cofactor for protein in the numerator of the KR equation (numerator = 0.46 vs. denominator = 0.58).

The equation for the ketogenic ratio also shows that increasing the fat level increases the KR. This is because the cofactor in the numerator is nine times that of the cofactor in the denominator.

These observations are consistent with biochemical processes: most amino acids are glucogenic and their catabolism *in vivo* leads to the production of glucose through the process of gluconeogenesis. The ketogenic analysis of two examples of compositions of the present disclosure, and two commercially available kibble diets C and D are presented in Tables 2, 3, 4, and 5, respectively. The composition of comparative diets C and D have KR of 1.15 and 0.97, respectively, based on analytical results of macronutrients. However, the kibble form of the composition of the present disclosure has a KR of 1.7, and the treat form has a KR = 2.17.

A cut off of KR = 1.5 has been long designated for true scientifically and medically valid ketogenicity of foods (rather than a marketing gimmick of simply stating "keto" without any scientific basis). See, for example, *Woodyatt,* op. cit. Diets purporting to be ketogenic for humans have been recently reassessed according to the KR criteria and few of them truly meet the standard. See, *Zilberter,* op. cit. It is clear from this analysis that commercially available foods for companion animals claiming to be ketogenic fail to adhere to the scientific and medical standards for this designation.

One critical element to the formulation which provides synergism to achieve ketogenic status is the presence of a large portion of the formulation fat as medium chain triglycerides (MCT), which is a profoundly ketogenic form of energy. MCTs are hydrolyzed to medium chain fatty acids (MCFA) by pancreatic lipase with an avidity exceeding that for long chain fatty acidcontaining triglycerides. MCFA generally consist of saturated carboxylic acids such as caproic acid, hexanoic acid, $CH_3(CH_2)_4COOH$, C6:0, caprylic acid, octanoic acid, $CH_3(CH_2)_6COOH$, C8:0, capric acid, decanoic acid, $CH_3(CH_2)_8COOH$, and C10:0. Both caprylic and capric acids the more common forms of MCFA, because they decrease gastrointestinal upset while increasing ketone production to a much greater extent than longer chain fatty acids (*i.e.,* $\geq$ C12:0). MCFA are absorbed directly into the liver via portal circulation. During catabolic energy production, MCFA do not require carnitine to be shuttled into mitochondria and are extensively converted to ketone bodies in the liver, which are then exported to the systemic circulation, leading to ketonemia and the designation of MCT as "ketogenic". See, *e.g., Bach et al.,* op. cit. MCT are prominent in nutritional prescriptions for modifying metabolic and overweight status. Under one embodiment, MCT increase gut barrier integrity and interact with the gut microbiome to improve the health of the host. MCT modify the gut microbiome, which is a mode of action for these fats to improve the metabolic health of obese individuals. See, *e.g., Rial et al.,* op. cit. Additionally, MCT influence neutrophil functions, including phagocytosis and oxidative bacterial killing, which may impact the makeup of gut microbiota. See, *e.g., Bellinati-Pires et al.,* op. cit. MCT have been shown to positively impact gut barrier integrity in an inflammatory model, which could influence the appearance of microbial metabolites in systemic circulation. See, *e.g., Lee & Kang,* op cit. Relevant to intestinal inflammation, MCT reduce the activity of bacterialresponsive inflammatory pathways and have shown efficacy in decreasing colitis. See. *e.g., Hanczakowska et al.,* op. cit. In tandem with the use of high levels of fibers known to nourish and activate the gut microbiome, the use of MCT, under one embodiment, heighten the benefits to gastrointestinal health in a way not possible with either agent alone.

Under one embodiment, the composition of the present disclosure comprises high levels of MCT. The exemplary kibble disclosed in Table 1 comprises 3.63 wt.% caprylic acid and 2.98 wt.% capric acid based on the analytical results of nutrients. Two commercially available companion animal diets C and D each comprises less than 0.02 wt.% caprylic acid and less than 0.02 wt.% capric acid. The level of MCT in exemplary kibble of the present disclosure has more than 100 times as much of the highly ketogenic fat known to improve the gut microbiome and improve host health. Importantly, some fat escapes digestion and caprylic acid are known to be converted to butyrate and 3-hydroxybutyrate via beta-oxidation by gut microbes and colonocytes. Butyrate is the primary source of energy for colonocytes, while 3-hydroxybutyrate is the primary ketone body *in vivo.* Though not bound by any theory, it is proposed that butyrate and 3-hydroxybutyrate generated synchronously provide a synergistic health benefit, and thus the composition of the present disclosure is expected to greatly improve health through the provision of significant amount of fat as MCT. In the exemplary kibble, 7.7% fat is present as MCT (DMB) and thus with a total fat level of 36% (DMB), MCT comprise ~20% of total fat.

Under one embodiment, the composition of the present disclosure has low levels of ash (< 6%, < 7%, etc.), which when combined with the moderate protein level and high fiber content to aid in the reduction of stressor on renal function, can be used under a wide array of feeding indications for adult companion animals. For example, the composition may have less than about 7 wt% ash, less than about 6 wt% ash, or less than about 5.5 wt% ash. The exemplary kibble disclosed in Table 1 comprises 5.5 wt.% ash, and 0.67 wt.% phosphorus, based on analytical results of nutrients. Other embodiments disclosed herein may include about 6.2 wt% ash, about 5.9 wt% ash, about 7.0 wt% ash, about 6.1 wt% ash, about 6.7 wt% ash, or about 6.1 wt% ash. Other embodiments disclosed herein may also include about 0.63 wt% phosphorus, about 0.72 wt% phosphorus, about 0.58 wt% phosphorus, about 0.62 wt% phosphorus, or about 0.56 wt% phosphorus. The comparative commercially available example C comprises 8.6 wt.% ash DMB and 1.2 wt.% phosphorus DMB, as determined by analysis of nutrients. The comparative commercially available example D comprises 9.9 wt.% ash DMB, and 1.2 wt.% phosphorus DMB as determined by analysis of nutrients. Although not bound by any theory, the composition of the present disclosure is healthier to renal function of companion animals.

The formulation has micronutrients added according to the ME, such that increased levels of vitamins and minerals were included (on a DMB percent basis) to account for the fact that companion animals will consume fewer grams of this food when fed to maintain healthy body weight since this food has significantly higher energy content relative to a traditional starchbased kibble.

Under one embodiment, the composition of the present disclosure is a dry kibble or a treat wherein functional proteins and fibers reduce nitrogen burden on the lower gastrointestinal tract as well as feeds and nourish the gut microbiome. Adherence to a low ash profile furthers the benefits of this reduction to practice. The ketogenic ratio KR of such a composition is greater than 1.5, which is higher than any commercially available companion animal diet. The combination of orthogonal functionality of the ingredients (e.g., nutrition and food production) is novel and non-obvious.

The present disclosure is also directed to a dietary composition for a companion animal comprising greater than about 34 wt% of fat or greater than 35 wt.% of fat, protein, less than 6 wt % of carbohydrates or less than 3.5 wt.% of carbohydrates, and greater than 10 wt.% of fiber, wherein (0.9 F + 0.46 P) / (0.1 F + 0.58 P + C) is greater than 1.5, wherein F, P, and C, are wt.% of fat, protein, and carbohydrates, respectively.

The composition of the present disclosure comprises fat or fats. Fats are one of the three main macronutrients, along with carbohydrates and proteins. Fat molecules consist of primarily carbon and hydrogen atoms and are therefore hydrophobic and are soluble in organic solvents and insoluble in water.

The term fat should be interpreted broadly, and include, cholesterol, phospholipids, triglycerides, lipids, and dietary oils. Some fatty acids that are set free by the digestion of fats are called essential because they cannot be synthesized in the body from simpler constituents. The term includes essential fatty acids, such as alpha-linolenic acid, and linoleic acid. The term also includes lipids needed by the body that may be synthesized from essential fatty acids and other fats. The term fat also refers to fats and other lipids that are broken down in the body by lipases produced in the pancreas.

The term fat also refers to fats that are saturated fats have no double bonds between the carbons in the chain, unsaturated fats have one or more double bonded carbons in the chain, oils and fats that have multiple double bonds, and polyunsaturated fats. Unsaturated fats include cis fats, which are the most common in nature, and trans fats. Fats also include unsaturated fats that have been altered by reaction with hydrogen in the presence of a catalyst, to make saturated fat.

Under one embodiment, the fat is a crude fat. Under one embodiment, the fat is a refined fat. Crude fat or fat may be supplied by any of a variety of sources known by those skilled in the art, including meat, meat by-products, fish oil, and plants. Plant fat sources include wheat, flaxseed, rye, barley, rice, sorghum, corn, oats, millet, wheat germ, corn germ, soybeans, peanuts, and cottonseed, as well as oils derived from these and other plant fat sources. The fat content of a composition may be determined by any number of methods known by those of skill in the art. Fats may be derived from fat sources such as choice white grease and vegetable oil.

Under one embodiment, the composition of the present disclosure comprises more than about 34 wt% fat, or more about 35 wt.% fat. Under one embodiment, the composition of the present disclosure comprises more about 37 wt.% fat. Under one embodiment, the composition of the present disclosure comprises more about 40 wt.% fat. Under one embodiment, the composition of the present disclosure comprises more about 42 wt.% fat. Under one embodiment, the composition of the present disclosure comprises more about 45 wt.% fat. Under one embodiment, the composition of the present disclosure comprises more about 50 wt.% fat. Under one embodiment, the composition of the present disclosure comprises more about 55 wt.% fat. Under one embodiment, the composition of the present disclosure comprises more about 60 wt.% fat. Under one embodiment, the composition of the present disclosure comprises more about 65 wt.% fat.

Under one embodiment, the composition of the present disclosure comprises between about 35 wt.% (i.e., 31.5 wt%) to about 38.5 wt%) and about 70 wt.% of fat (i.e., 63 wt% to 77 wt%). Under one embodiment, the composition of the present disclosure comprises between about 40 wt.% and about 70 wt.% of fat. Under one embodiment, the composition of the present disclosure comprises between about 45 wt.% and about 70 wt.% of fat. Under one embodiment, the composition of the present disclosure comprises between about 50 wt.% and about 70 wt.% of fat. Under one embodiment, the composition of the present disclosure comprises between about 60 wt.% and about 70 wt.% of fat. Under one embodiment, the composition of the present disclosure comprises between about 35 wt.% and about 60 wt.% of fat. Under one embodiment, the composition of the present disclosure comprises between about 40 wt.% and about 60 wt.% of fat. Under one embodiment, the composition of the present disclosure comprises between about 45 wt.% and about 60 wt.% of fat. Under one embodiment, the composition of the present disclosure comprises between about 50 wt.% and about 60 wt.% of fat. Under one embodiment, the composition of the present disclosure comprises between about 35 wt.% and about 50 wt.% of fat. Under one embodiment, the composition of the present disclosure comprises between about 40 wt.% and about 50 wt.% of fat. Under one embodiment, the composition of the present disclosure comprises between about 45 wt.% and about 50 wt.% of fat. Under one embodiment, the composition of the present disclosure comprises between about 35 wt.% and about 45 wt.% of fat. Under one embodiment, the composition of the present disclosure comprises between about 40 wt.% and about 45 wt.% of fat. Under one embodiment, the composition of the present disclosure comprises between about 35 wt.% and about 40 wt.% of fat.

Under one embodiment, the fat is comprised of at least 20 wt.% medium chain triglycerides. Triglyceride, triacylglycerol, or triacylglyceride is an ester derived from glycerol and three fatty acids. Triglycerides are the main constituents of body fat in mammals and other vertebrates, and in vegetable fats. Triglycerides are also present in the blood of enable the transference of fat from muscle, adipose tissue liver and other body tissues.

The term "triglyceride" is defined broadly. Included in the definition of triglycerides is saturated triglycerides, and unsaturated triglycerides. Saturated triglycerides have a higher melting point and are more likely to be solid at room temperature. Unsaturated fats have double bonds between some of the carbon atoms. The unsaturated triglycerides have a lower melting point and are more likely to be liquid at room temperature.

The phrase "medium chain triglycerides" refers to triglycerides of fatty acid being 6 to 12 carbon atoms in length, including caproic acid, caprylic acid, capric acid and lauric acid. Additional examples of fatty acids of 6 to 12 carbons include hexanoic acid, $CH_3(CH_2)_4COOH$, enanthic acid, heptanoic acid, $CH_3(CH_2)_5COOH$, enantic acid, octanoic acid, $CH_3(CH_2)_6COOH$, pelargonic acid, nonanoic acid, $CH_3(CH_2)_7COOH$, decanoic acid, $CH_3(CH_2)_8COOH$, undecylic acid, undecanoic acid, $CH_3(CH_2)_9COOH$, and mixtures thereof.

Medium chain triglycerides may be either naturally derived or synthetic. Medium chain triglyceride oil is commercially available as Miglyol 812 from SASOL Limited (Sandton, South Africa), CRODAMOL GTCC-PN from Croda Int'l. (Snaith, Yorkshire, United Kingdom), Neobees M-5 oil from Stepan Co. (Northfield, Illinois, USA). Other low-melting medium chain oils may also be used in the present disclosure.

Under one embodiment, the fat of the dietary composition is comprised of at least 20 wt.%, at least 21 wt%, at least 22 wt%, or at least 23 wt% medium chain triglycerides. Under one embodiment, the fat of the dietary composition is comprised of at least 25 wt.% medium chain triglycerides. Under one embodiment, the fat of the dietary composition is comprised of at least 30 wt.% medium chain triglycerides. Under one embodiment, the fat of the dietary composition is comprised of at least 35 wt.% medium chain triglycerides. Under one embodiment, the fat of the dietary composition is comprised of at least 40 wt.% medium chain triglycerides.

Under one embodiment, the fat of the dietary composition is comprised of from about 10 wt.% to about 35 wt.% medium chain triglycerides. Under one embodiment, the fat of the dietary composition is comprised of from about 15 wt.% to about 35 wt.% medium chain triglycerides. Under one embodiment, the fat of the dietary composition is comprised of from about 20 wt.% to about 35 wt.% medium chain triglycerides. Under one embodiment, the fat of the dietary composition is comprised of from about 25 wt.% to about 35 wt.% medium chain triglycerides. Under one embodiment, the fat of the dietary composition is comprised of from about 10 wt.% to about 25 wt.% medium chain triglycerides. Under one embodiment, the fat of the dietary composition is comprised of from about 15 wt.% to about 25 wt.% medium chain triglycerides. Under one embodiment, the fat of the dietary composition is comprised of from about 20 wt.% to about 25 wt.% medium chain triglycerides. Under one embodiment, the fat of the dietary composition is comprised of from about 10 wt.% to about 20 wt.% medium chain triglycerides. Under one embodiment, the fat of the dietary composition is comprised of from about 15 wt.% to about 20 wt.% medium chain triglycerides. Under one embodiment, the fat of the dietary composition is comprised of from about 10 wt.% to about 15 wt.% medium chain triglycerides.

One of the ingredients of the dietary composition of the present disclosure is crude protein. Crude protein may be supplied by any of a variety of sources known by those skilled in the art, including plant sources, animal sources, or both. Animal sources include, for example, meat, meat by-products, seafood, dairy, eggs, etc. Meats include, for example, the flesh of poultry, fish, and mammals (e.g., cattle, pigs, sheep, goats, and the like). Meat by-products include, for example, lungs, kidneys, brain, livers, and stomachs and intestines (freed of all or essentially all their contents). Plant protein includes vegetable proteins such as soybean, cottonseed, and peanut. The protein can be intact, almost completely hydrolyzed, or partially hydrolyzed. The protein content of foods may be determined by any number of methods known by those of skill in the art, for example, as published by the Association of Official Analytical Chemists in Official Methods of Analysis ("OMA"), method 988.05. The amount of "crude protein" in a composition disclosed herein may be determined based on the amount of nitrogen in the composition according to methods familiar to one of skill in the art.

The term "protein" means a polypeptide, or a peptide, or a polymer of amino acids. The term encompasses naturally occurring and non-naturally occurring (synthetic) polymers and polymers in which artificial chemical mimetics are substituted for one or more amino acids. The term also encompasses fragments, variants, and homologs that have the same or substantially the same properties and perform the same or substantially the same function as the original sequence. The term encompasses polymers of any length, including polymers containing from about 2 to 1000, from 4 to 800, from 6 to 600, and from 8 to 400 amino acids. The term includes amino acid polymers that are synthesized and that are isolated and purified from natural sources. Under some embodiments, the terms "polypeptide", "peptide" or "protein" are used interchangeably.

The protein is present in the composition of the present disclosure in non-negligible amounts. Under one embodiment, the protein level is more than about 1 wt.%. Under one embodiment, the composition of the present disclosure comprises between about 1 wt.% and about 30 wt.% of protein.

Under one embodiment, the composition of the present disclosure comprises between about 4.5 g and 75 g of protein per 1000 kcal metabolizable energy. The claimed compositions may comprise a metabolizable energy content of from about 3000 to about 5000 kcal/kg.

Metabolizable energy (ME) of a diet is the energy available to an animal upon consumption of the diet after subtracting the energy excreted in feces, urine, and combustible gases. Metabolizable energy values may be determined by methods known by those skilled in the art, such as detailed by Association of American Feed Control Officials: Official Publication, Atlanta, Ga., pages 160-165 (2006).

One of the ingredients of the dietary composition of the present disclosure is carbohydrate. The term "carbohydrate" as used herein includes polysaccharides (*e.g.*, starches and dextrins) and sugars (*e.g.*, sucrose, lactose, maltose, glucose, and fructose) that are metabolized for energy when hydrolyzed. Examples of carbohydrate sources suitable for inclusion in the compositions disclosed herein include but are not limited to, corn, grain sorghum, wheat, barley, and rice.

Under one embodiment, the carbohydrate is obtained from a carbohydrate source selected from the group consisting of corn, wheat, distiller's dried grain, corn starch, rice, corn gluten meal, and mixtures thereof.

Under one embodiment, the carbohydrate component comprises a mixture of one or more carbohydrate sources. Suitable carbohydrate sources include, for example, carbohydrates selected from the group consisting of oat fiber, cellulose, peanut hull, beet pulp, parboiled rice, corn starch, corn gluten meal and mixtures thereof. By properly balancing carbohydrate sources, one skilled in the art can manipulate the texture of the final product. For example, short chain polysaccharides tend to be sticky and gluey, and longer chain polysaccharides are less sticky and gluey than the shorter chain; the desired texture of this hybrid food is achieved by longer chain polysaccharide and modified starches such as native or modified starches, cellulose and the like.

The carbohydrate mixture may additionally comprise optional components such as added salt, spices, seasonings, vitamins, minerals, flavorants, colorants, and the like. The amount of the optional additives is at least partially dependent on the nutritional requirements for different life stages of animals.

Under one embodiment, the composition of the present disclosure comprises less than about 6 wt% carbohydrates, less than about 5 wt% carbohydrates, less than about 4 wt% carbohydrates, or less than about 3.5 wt.% carbohydrates. Under one embodiment, the composition of the present disclosure comprises less than about 2.5 wt.% carbohydrates. Under one embodiment, the composition of the present disclosure comprises less than about 2 wt% or less than 1.5 wt.% carbohydrates. Under one embodiment, the composition of the present disclosure comprises less than about 0.5 wt.% carbohydrates.

Under one embodiment, the composition comprises between about 0.5 wt% and about 6 wt% carbohydrates. Under one embodiment, the composition of the present disclosure comprises between about 0.5 wt.% and about 3.5 wt.% of carbohydrates. Under one embodiment, the composition of the present disclosure comprises between about 1.5 wt.% and about 3.5 wt.% of carbohydrates. Under one embodiment, the composition of the present disclosure comprises between about 2.5 wt.% and about 3.5 wt.% of carbohydrates. Under one embodiment, the composition of the present disclosure comprises between about 0.5 wt.% and about 2.5 wt.% of carbohydrates. Under one embodiment, the composition of the present disclosure comprises between about 1.5 wt.% and about 2.5 wt.% of carbohydrates. Under one embodiment, the composition of the present disclosure comprises between about 0.5 wt.% and about 1.5 wt.% of carbohydrates.

One of the ingredients of the dietary composition of the present disclosure is dietary fiber. Dietary fiber refers to components of a plant which are resistant to digestion by an animal's digestive enzymes. Dietary fiber, or total dietary fiber, consists of insoluble fiber and soluble fiber.

As used herein, the phrase "soluble fiber" refers to dietary fiber that attracts water during digestion and slows the rate of nutrient absorption. Soluble fiber is resistant to digestion and absorption in the small intestine and undergo complete or partial fermentation in the large intestine, and is typically found in various plant sources, including oat bran, seeds, beans, and certain fruits and vegetables such as beet pulp, guar gum, chicory root, psyllium, pectin, blueberry, cranberry, squash, apples, oats, beans, citrus, barley and peas. The phrase encompasses any source of soluble fiber suitable for the compositions disclosed herein as would be evident to one of skill in the art.

Insoluble fiber may be supplied by any of a variety of sources, including cellulose, whole wheat products, wheat oat, corn bran, flaxseed, grapes, celery, green beans, cauliflower, potato skins, fruit skins, vegetable skins, peanut hulls, and soy fiber.

Crude fiber includes indigestible components contained in cell walls and cell contents of plants such as grains, e.g., hulls of grains such as rice, corn, and beans.

The composition of the present disclosure comprises more than 10 wt.% fiber. Under one embodiment, the composition of the present disclosure comprises more than about 13 wt.% fiber. Under one embodiment, the composition comprises more than about 15 wt% fiber. Under one embodiment, the composition of the present disclosure comprises more than about 16 wt.% fiber. Under one embodiment, the composition of the present disclosure comprises more than about 20 wt.% fiber. Under one embodiment, the composition of the present disclosure comprises more than about 25 wt.% fiber. Under one embodiment, the composition of the present disclosure comprises more than about 30 wt.% fiber. Under one embodiment, the composition of the present disclosure comprises more than about 40 wt.% fiber.

Under one embodiment, the composition of the present disclosure comprises between 10 wt.% and about 40 wt.% of fiber. Under one embodiment, the composition of the present disclosure comprises between about 13 wt.% and about 40 wt.% of fiber. Under one embodiment, the composition of the present disclosure comprises between about 16 wt.% and about 40 wt.% of fiber. Under one embodiment, the composition of the present disclosure comprises between about 20 wt.% and about 40 wt.% of fiber. Under one embodiment, the composition of the present disclosure comprises between about 25 wt.% and about 40 wt.% of fiber. Under one embodiment, the composition of the present disclosure comprises between about 30 wt.% and about 40 wt.% of fiber. Under one embodiment, the composition of the present disclosure comprises between 10 wt.% and about 30 wt.% of fiber. Under one embodiment, the composition of the present disclosure comprises between about 13 wt.% and about 30 wt.% of fiber. Under one embodiment, the composition of the present disclosure comprises between about 16 wt.% and about 30 wt.% of fiber. Under one embodiment, the composition of the present disclosure comprises between about 20 wt.% and about 30 wt.% of fiber. Under one embodiment, the composition of the present disclosure comprises between about 25 wt.% and about 30 wt.% of fiber.

Under one embodiment, the composition of the present disclosure comprises between 10 wt.% and about 25 wt.% of fiber. Under one embodiment, the composition of the present disclosure comprises between about 13 wt.% and about 25 wt.% of fiber. Under one embodiment, the composition of the present disclosure comprises between about 16 wt.% and about 25 wt.% of fiber. Under one embodiment, the composition of the present disclosure comprises between about 20 wt.% and about 25 wt.% of fiber. Under one embodiment, the composition of the present disclosure comprises between 10 wt.% and about 20 wt.% of fiber. Under one embodiment, the composition of the present disclosure comprises between about 13 wt.% and about 20 wt.% of fiber. Under one embodiment, the composition of the present disclosure comprises between about 16 wt.% and about 20 wt.% of fiber. Under one embodiment, the composition of the present disclosure comprises between 10 wt.% and about 16 wt.% of fiber. Under one embodiment, the composition of the present disclosure comprises between about 13 wt.% and about 16 wt.% of fiber. Under one embodiment, the composition of the present disclosure comprises between about 10 wt.% and about 13 wt.% of fiber.

The present disclosure is also directed to a dietary composition for a companion animal comprising greater than 35 wt.% (i.e., 31.5 wt% to 38.5 wt%) of fat, protein, less than 3.5 wt.% of carbohydrates, and greater than 10 wt.% of fiber, wherein the ketogenic ratio is greater than 1.5.

The ketogenic ratio (KR) is a ratio of the sum of ketogenic factors to the sum of antiketogenic factors: KR = K/AK. The antiketogenic part of the equation equals 1 so the KRs are always expressed as 2:1, 4:1, etc., or simply by the number without ":1" part. Hence, a ketogenic ratio of 1.7 means a ketogenic ratio of 1.7 : 1.

The maximal ratio compatible with the oxidation of the ketogenic molecules becomes possible at the KR = 1, making KRs below 1 antiketogenic and KRs above 2 ketogenic. The KR of a food in terms of times the fat content exceeds the amount of carbohydrate and protein combined, roughly.

The ketogenic ratio is defined by the formula

$$KR = (0.9\,F + 0.46\,P) \,/\, (0.1\,F + 0.58\,P + C)$$

wherein F is grams of fat; P is grams of protein and C is grams of carbohydrate.

Under one embodiment, the ketogenic ratio of the composition of the present disclosure is greater than 1.5. Under one embodiment, the ketogenic ratio of the composition of the present disclosure is greater than about 1.7. Under one embodiment, the ketogenic ratio of the composition of the present disclosure is greater than about 2. Under one embodiment, the ketogenic ratio of the composition of the present disclosure is greater than about 2.5. Under one embodiment, the ketogenic ratio of the composition of the present disclosure is greater than about 3. Under one embodiment, the ketogenic ratio of the composition of the present disclosure is greater than about 4.

Under one embodiment, the ketogenic ratio of the composition of the present disclosure is between 1.5 and about 4. Under one embodiment, the ketogenic ratio of the composition of the present disclosure is between about 1.7 and about 4. Under one embodiment, the ketogenic ratio of the composition of the present disclosure is between about 2 and about 4. Under one embodiment, the ketogenic ratio of the composition of the present disclosure is between about 2.5 and about 4. Under one embodiment, the ketogenic ratio of the composition of the present disclosure is between about 3 and about 4. Under one embodiment, the ketogenic ratio of the composition of the present disclosure is between 1.5 and about 3. Under one embodiment, the ketogenic ratio of the composition of the present disclosure is between about 1.7 and about 3. Under one embodiment, the ketogenic ratio of the composition of the present disclosure is between about 2 and about 3. Under one embodiment, the ketogenic ratio of the composition of the present disclosure is between about 2.5 and about 3. Under one embodiment, the ketogenic ratio of the composition of the present disclosure is between 1.5 and about 2.5. Under one embodiment, the ketogenic ratio of the composition of the present disclosure is between about 1.7 and about 2.5. Under one embodiment, the ketogenic ratio of the composition of the present disclosure is between about 2 and about 2.5. Under one embodiment, the ketogenic ratio of the composition of the present disclosure is between 1.5 and about 2. Under one embodiment, the ketogenic ratio of the composition of the present disclosure is between about 1.7 and about 2. Under one embodiment, the ketogenic ratio of the composition of the present disclosure is between 1.5 and about 1.7.

The present disclosure is further directed to a dietary composition for a companion animal comprising greater than 35 wt.% (i.e., 31.5 wt% to 38.5 wt%) of fat, protein, less than 3.5 wt.% of carbohydrates, and at least 14 wt%, or at least 16 wt.% of fiber, wherein (0.9 F + 0.46 P) / (0.1 F + 0.58 P + C) is greater than 1.5, wherein F, P, and C, are wt.% of fat, protein, and carbohydrates, respectively, further comprising additional ingredients. Whereas fat, protein, carbohydrates, and fiber are considered macronutrients, these additional ingredients are micronutrients.

The present disclosure is also directed to a nutritionally complete pet food comprising the dietary composition.

Micronutrients occurring in the composition of the present disclosure include trace minerals, vitamins, related compounds and sources thereof, at levels that are recommended or acceptable for companion animals. Examples of trace minerals include boron, cobalt, chromium, copper, iodine, iron, manganese, molybdenum, selenium, and zinc. Examples of vitamin and related compounds include Vitamin B complex, Vitamin B1, thiamin, Vitamin B2, riboflavin, Vitamin B3, niacin, Vitamin B5, pantothenic acid, Vitamin B6, pyridoxine, pyridoxal-5-phosphate, pyridoxamine, Vitamin B7, biotin, Vitamin B9, folate, Vitamin B 12, cobalamin, choline, Vitamin A, retinol, Vitamin C, ascorbic acid, Vitamin E, tocopherols, tocotrienols, Vitamin K, Vitamin K1, phylloquinone, Vitamin K2, menaquinone, carotenoids, alpha carotene, beta carotene, cryptoxanthin, lutein, lycopene, zeaxanthin,

Under one embodiment, the composition of the present disclosure comprises ash. "Ash" consists of compounds that are not organic or water, generally produced by combustion of biological materials. Ash may be determined by any number of methods known by those of skill in the art.

Under one embodiment, the composition of the present disclosure comprises more than about 6 wt.% ash. Under one embodiment, the composition of the present disclosure comprises less than about 7% ash or less than about 6 wt.% ash. Under one embodiment, the composition of the present disclosure comprises less than about 4 wt.% ash. Under one embodiment, the composition of the present disclosure comprises less than about 2 wt.% ash. Under one embodiment, the composition of the present disclosure comprises less than about 1 wt.% ash. Under one embodiment, the composition of the present disclosure comprises less than about 0.5 wt.% ash.

Under one embodiment, the composition of the comprises between about 0.5 wt% and about 7 wt% of ash. Under one embodiment, the composition of the present disclosure comprises between about 0.5 wt% and about 6 wt.% of ash. Under one embodiment, the composition of the present disclosure comprises between about 1 wt.% and about 6 wt.% or about 7 wt% of ash. Under one embodiment, the composition of the present disclosure comprises between about 2 wt.% and about 6 wt.% or about 7 wt% of ash. Under one embodiment, the composition of the present disclosure comprises between about 4 wt.% and about 6 wt.% or about 7 wt% of ash. Under one embodiment, the composition of the present disclosure comprises between about 0.5 wt.% and about 4 wt.% of ash. Under one embodiment, the composition of the present disclosure comprises between about 1 wt.% and about 4 wt.% of ash. Under one embodiment, the composition of the present disclosure comprises between about 2 wt.% and about 4 wt.% of ash. Under one embodiment, the composition of the present disclosure comprises between about 0.5 wt.% and about 2 wt.% of ash. Under one embodiment, the composition of the present disclosure comprises between about 1 wt.% and about 2 wt.% of ash. Under one embodiment, the composition of the present disclosure comprises between about 0.5 wt.% and about 1 wt.% of ash.

Under one embodiment, the composition of the present disclosure comprises a low level of phosphorus. Reducing the level of dietary phosphorus has been shown to slow progression of kidney disease and prolong life. Under one embodiment, the composition of the present disclosure comprises less than about 0.7 wt.% (i.e. less than about 0.63 wt% to less than about 0.77 wt%) of phosphorus. For example, the composition may comprise less than about 0.77 wt%, less than about 0.7 wt%, or less than about 0.63 wt% of phosphorus. Under one embodiment, the composition of the present disclosure comprises less than about 0.5 wt.% of phosphorus. Under one embodiment, the composition of the present disclosure comprises less than about 0.3 wt.% of phosphorus.

Moisture is the amount of water in the dietary composition. Dry kibble tends to have a moisture content of between 6 and 10 percent, semi-moist foods between 15 and 30 percent, and wet foods around 75 percent.

The nutritionally complete pet food contains additional ingredients such as vitamins, minerals, fillers, palatability enhancers, binding agents, flavors, stabilizers, emulsifiers, sweeteners, colorants, buffers, salts, coatings, and the like known to skilled artisans. Stabilizers include substances that tend to increase the shelf life of the composition such as preservatives, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches. Specific amounts for each composition component, food ingredient, and other ingredients will depend on a variety of factors such as the particular components and ingredients included in the composition; the species of patient; the patient's age, body weight, general health, sex, and diet; the animal's consumption rate; the type of disease being treated; and the like. Therefore, the component and ingredient amounts may vary widely and may deviate from the preferred proportions described herein.

The food compositions may be prepared in a canned or wet form using conventional food preparation processes known to skilled artisans. Typically, ground animal proteinaceous tissues are mixed with the other ingredients such as fish oils, cereal grains, balancing ingredients, special purpose additives (e.g., vitamin and mineral mixtures, inorganic salts, cellulose, and beet pulp, bulking agents, and the like) and water in amounts sufficient for processing. These ingredients are mixed in a vessel suitable for heating while blending the components. The heating of the mixture is effected using any suitable manner, e.g., direct steam injection or using a vessel fitted with a heat exchanger. Following the addition of the last ingredient, the mixture is heated to a temperature of from about 10 °C to about 100 °C. Temperatures outside this range are acceptable but may be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. Sterilization is usually accomplished by heating to temperatures of greater than about 110 °C for an appropriate time depending on the temperature used, the composition, and similar factors. The compositions of the present disclosure can be added to the food compositions before, during, or after preparation.

The food compositions may be prepared in a dry form using conventional processes known to skilled artisans. Typically, dry ingredients such as animal protein, plant protein, grains, and the like are ground and mixed together. Moist or liquid ingredients, including fats, oils, animal protein, water, and the like are then added to and mixed with the dry mix. The mixture is then processed into dry food pieces.

The food compositions can be in any form useful for feeding the composition to the companion animal, *e.g.*, kibbles, treats, and toys for animal food. Kibbles are generally formed using an extrusion process in which the mixture of dry and wet ingredients is subjected to mechanical work at high pressure and temperature and forced through small openings and cut off into kibble by a rotating knife. The wet kibble is then dried and optionally coated with one or more topical coatings such as flavors, fats, oils, powders, and the like. Kibble also can be made from the dough using a baking process, rather than extrusion, wherein the dough is placed into a mold before dry-heat processing. Treats include compositions that are given to an animal to entice the animal to eat during a non-meal time, *e.g.*, dog bones or biscuits for canines. Treats may be nutritional wherein the composition comprises one or more nutrients or and may have a food-like composition. Non-nutritional treats encompass any other treats that are non-toxic. The composition or components are coated onto the treat, incorporated into the treat, or both. Treats of the present disclosure can be prepared by an extrusion or baking process similar to those used for dry food. Other processes also may be used to either coat the composition on the exterior of existing treat forms or inject the composition into an existing treat form. Toys include chewable toys such as artificial bones and food compositions shaped to resemble natural foods that are appealing to the animal. The food composition of the present disclosure can comprise the toy or can form a coating on the surface of the toy or on the surface of a component of the toy. The composition can be incorporated partially or fully throughout the toy or both. In one embodiment, the composition is orally accessible by the intended user. The present disclosure encompasses partially consumable toys, *e.g.*, toys comprising plastic components, and fully consumable toys, *e.g.*, various artificial bones and similar foods. Further, the disclosure encompasses toys for both human and non-human use, particularly toys for companion, farm, and zoo animal use, and more particularly for feline and canine use.

The present disclosure is also directed to a method of improving the health of a companion animal by feeding the animal a pet food comprising an effective amount of the dietary composition. The ketogenic diet pet food is applicable to several indications or conditions, including high blood glucose, chronic inflammation, metabolic stress, and cancer. The administration of the pet food comprising the dietary composition shifts the animal's metabolism from glucose-converting metabolism to fat-burning metabolism.

In at least one embodiment, any one or more components or ingredients of the pet food composition disclosed herein may provide multiple functions or purposes, particularly selected were components which provided both a nutritional advantage to increase ketogenicity, or enhance the health of a subject in dietary ketosis, and also to aid in the formation of a durable, palatable kibble with high fat inclusion levels.

In one embodiment the composition includes sources of proteins which are both beneficial to nutrition in that they are low ash, highly bioavailable, and contain all essential amino acids in sufficient amounts for companion animal nutrition. Further, a second group of these proteins are predigested hydrolysates which speeds delivery of amino acid nutrition after ingestion. From a ketogenic dry kibble production standpoint, selected proteins may firstly be those which are gauged in the industry to be water active; specifically that they are sufficiently hydrophilic and hygroscopic to absorb water and steam introduced during processing. Secondly, proteins may provide an element of binding to the finished dried kibble such that the resultant kibble product is durable for transport and consumption. Thirdly, the source of protein must not contain appreciable amounts of dietary digestible carbohydrate; preferably the digestible carbohydrate level in the protein source will be less than 5%, even more preferably less than 3% and even more preferably less than 2%. With regards to the third point, this preferably involves the use of protein isolates rather than protein concentrates when those protein sources are not meat (e.g. are from dairy or vegetable). To provide both the aforementioned nutritional and production benefits, one embodiment may include one or more intact (non-hydrolyzed) proteins of the following: 1) Meat protein isolate (sourced from pork, beef, sheep, goat, avian, fish, shell fish) comprising less than 10% ash, less than 10% fat; 2) Dried Meat (sourced from pork, beef, sheep, goat, avian, fish, shell fish) comprising less than 15% ash, less than 20% fat; 3) Dried whole avian egg or egg white (less than 10% ash, less than 50% fat) from genera (Gallus gallus domesticus, Meleagris, Struthio camelus); 4) Milk, Casein or Whey protein isolate (less than 10% ash, less than 10% fat) from genera (Bos taurus, Capra aegagrus hircus, Ovis aries, Camelus spp., Bison bison, Bubalus bubalis) and related genera; 5) Glutinous proteins extracts from grains of the genus Triticum spp.; 6) Collagenous animal proteins including gelatin from pork, beef, sheep, goat, chicken or fish; and/or 7) Plant-based Protein Isolates (less than 10% ash, less than 10% fat, less than 10% fiber), which may be from peas, lentils, beans, grains, tubers or other sources provided they have less than the aforementioned levels of dietary digestible carbohydrate. This embodiment may include one or more predigested protein hydrolysates having: 1) a degree of hydrolysis of greater than or equal to about 30% (greater than around 30% of peptide bonds between neighboring amino acids in the original protein have been hydrolyzed to free amino terminal ends; and/or 2) an average molecular weight of from about 1000 Daltons to about 100,000 daltons. These hydrolysates may be selected from the following: 1) Hydrolyzed Meat protein isolate (sourced from pork, beef, sheep, goat, avian, fish, shell fish) comprising less than 10% ash, less than 10% fat; 2) Hydrolyzed Dried Meat (sourced from pork, beef, sheep, goat, avian, fish, shell fish) comprising less than 15% ash, less than 20% fat; 3) Hydrolyzed Dried Whole Avian Egg or Egg White (less than 10% ash, less than 50% fat) from genera (Gallus gallus domesticus, Meleagris, Struthio camelus); 4) Hydrolyzed Milk, Casein or Whey protein isolate (greater than 30% degree of hydrolysis, averless than 10% ash, less than 10% fat) from genera (Bos taurus, Capra aegagrus hircus, Ovis aries, Camelus spp., Bison bison, Bubalus bubalis) and related genera; and/or 5) Hydrolyzed Collagenous Animal Protein including gelatin from pork, beef, sheep, goat, chicken or fish.

In another embodiment the composition includes sources of dietary non-digestible fibers that provide benefits to both companion animal nutrition in that they nourish the gut microbiome and promote acceptable stool quality. These fibers also provide a dual benefit to ketogenic dry kibble production in that they act in concert with the aforementioned water active proteins to form a matrix capable of supporting a truly ketogenic macronutrient formulation. Ingredients may be selected which provide one or more of the following fibers: cellulose, lignin, hemicellulose, pectin, alginate, modified cellulose (including methylcellulose, hydroxyisopropylcellulose), or combinations thereof. As well, preferably there may be inclusion of fiber having an average polysaccharide degree of polymerization of greater than of around 1000 and composed of monosaccharides from the following: glucose, arabinose, xylose, mannose, galactose, and/or related sugars or sugar acids (e.g. galacturonic acid). To provide the dual functional fibers, ingredients may be used alone in or in combination from the following sources, or related sources: purified celluloses, citrus pulp, pecan shell powder, beet pulp, cranberry pulp, apple pulp, flax seed, psyllium, oat bran).

In one embodiment the composition includes sources of dietary medium chain triglycerides (MCT). These MCT increase post prandial ketonemia by being avidly converted to ketones instead of being stored as fat. Additionally, they provide a benefit to production of a ketogenic kibble due to their unique properties: low viscosity, not susceptible to autoxidation. These MCT are liquid at low temperatures (including at standard refrigeration temperatures of 4 degrees Celsius). Low viscosity facilitates and allows for the fat energy to better soak into the dry kibble. Further, the oxidative stability of the MCT lend themselves to increased taste preservation and reduced rancidity, which should improve food intake, particularly in pets with poor appetites due to disease or preference. Ingredients providing MCT may be derived from fractionated palm, coconut or butter fats. These sources preferably supply greater than 50% fat energy as MCT having as constituent fatty acids C8:0 octanoate and/or C10:0 decanoate. The C8:0 and the C10:0 will be preferably in a ratio of C8:0/C10: from 0.99 to 0.01, more preferably from 0.99 to 0.2 and most preferably from 0.99 to 0.4.

In one embodiment the composition includes sources of digestible carbohydrate starch in the form of amylopectin. Although this is a digestible starch whose inclusion decreases the ketogenicity of the diet, pure amylopectin that does not contain appreciable amylose is a profoundly water active starch that can improve dry kibble aesthetics. If the appropriate formulation level of amylopectin inclusion is selected, then the ketogenic ratio of the overall dry kibble may be preserved above 1.5 while enhancing aesthetics to improve palatability and food acceptance to companion animals. In instances where a ketogenic dry kibble formulation is preferred to be low in dietary fiber, amylopectin inclusion at low levels, such as less than about 3 wt%, less than about 2 wt%, or less may synergize with the residual water active fibers and proteins to produce a pleasing and palatable product. Ingredients providing amylopectin may be derived from potatoes (or related tubers) or rice (or other grains). These sources preferably supply greater than 90% amylopectin and less than 10% amylose.

It should be appreciated that ketogenic diets may often induce an acidic metabolic burden on the circulatory and urinary systems. As such, potassium citrate may be incorporated into the pet food foods or compositions disclosed herein to reduce kidney-stone incidence or formation. Potassium citrate may provide benefits to urinary healthy including dissolution of uroliths. It is noted that there are currently no ketogenic companion animal foods that contain potassium citrate as the inclusion of potassium citrate into pet food compositions is not trivial or obvious. For example, the incorporation of potassium citrate requires a non-obvious balancing of various factors/variables including, but not limited to, cations and anions, residual potassium, the resultant urinary pH, overall palatability, or combinations thereof. The present disclosure overcomes those significant hurdles to including potassium citrate and then further documents than an example increases circulating blood citrate and modifies urine pH. Thus in some embodiments the composition may contain potassium citrate in an amount sufficient to preferably provide between 1 to 6 Milliequivalents (mEq) potassium citrate per kilogram metabolic bodyweight (1 to 6 mEq $K_3$Citrate/kg$^{0.75}$). The composition may more preferably provide between 2 to 4 mEq $K_3$Citrate/kg$^{0.75}$). The composition may most preferably provide 3 mEq $K_3$Citrate/kg$^{0.75}$).

The ketogenic diet induces a metabolic burden for catabolism of dietary fat due to the large percentage of energy intake as fat. Continued carbohydrate restriction leads to changes in gene expression and enzyme activity that result in a metabolic shift toward fat metabolism and increased energy expenditure. These changes in gene expression and enzyme activity to increase fat metabolism would be optimally served if the fat-metabolizing co-factors that they require were provided in amounts greater than might be present in a non-supplemented diet. In fact, it may be that a ketogenic diet is not maximally valuable unless sufficient ancillary factors are available to allow increased fat metabolism. Despite this, the current state of the art ketogenic companion animal foods do not contain added fat-metabolizing factors. These labels for these commercial foods were surveyed for added carnitine, taurine, betaine, and lipoic acid. Further, the foods were chemically analyzed for these fat-metabolizing supplements. Neither labels nor analysis indicated the presence of added carnitine, added taurine, added betaine, and/or added lipoic acid. In recognition of the value of maximizing fat metabolism, in some embodiments the composition may contain added carnitine between 0.01 and 0.1%, added taurine between 0.1 and 0.5%, added betaine between 0.25 and 1% and/or added lipoic acid between 0.005 and 0.05 %. Due to species differences, lipoic acid is not included in companion animal feline formulations.

The ketogenic diet of the present disclosure may also be used to improve the health of an adult pet or a senior pet.

The pet food compositions of the present disclosure may be ketogenic pet food compositions including restricted amounts of starch while maintaining a relatively greater durability as compared to conventional pet food compositions. For example, the pet food compositions disclosed herein may have durability of greater than 70, greater than 75, greater than 80, greater than 85, or greater than 90, based on a pellet durability index score (PDI).

The pet food compositions of the present disclosure may also be ketogenic pet food compositions having relatively greater durability while maintaining or exhibiting improved palatability over conventional pet food compositions. For example, the ketogenic pet food compositions disclosed herein may exhibit improved durability that does not adversely impact palatability.

The pet food compositions of the present disclosure may be ketogenic pet food compositions in the form of kibble. In another embodiment, the ketogenic pet food compositions disclosed herein may be in the form of a wet loaf. For example, the ketogenic pet food composition may be a wet loaf disposed or packaged in a container, such as an airtight container. Illustrative containers may be or include, but are not limited to, a retort pouch, a sachet, a TETRA PAK®, a bottle, a tray, a metal can (e.g., tin can), or the like, or combinations thereof. The wet loaf prepared according the embodiments disclosed herein may include one or more of a non-digestible gum fiber, an emulsifier, gelatin, or combinations thereof. As used herein, non-digestible gum fiber may refer to any gum derived from a vegetable and/or bacterial fermentation. Illustrative non-digestible gum fibers may include, but are not limited to, guar gum, tara gum, acacia gum, carob gum, or the like, or combinations thereof. The emulsifier may include nutritional fat based emulsifiers including at least one fatty acid group. Illustrative emulsifiers may be or include, but are not limited to, lecithin, monoglycerides, diglycerides, or combinations thereof. The wet loafs may not exhibit any apparent fat or oil separation after removing from a container, cutting, and pressing. In a preferred implementation, the wet loaf ketogenic pet food compositions disclosed herein may include a non-digestible gum fiber in an amount of from about 0.5 wt% to about 3 wt%, an emulsifier compatible with the companion nutrition, or combinations thereof. In at least one embodiment, the wet loaf ketogenic pet food compositions include gelatin. In another embodiment, the wet loaf ketogenic pet food compositions are free or substantially free of gelatin.

The pet food composition of the present disclosure may be a ketogenic pet food composition for felines in the form of a dried kibble. For example, the pet food composition of the present disclosure may be a ketogenic pet food composition that meets and exceeds the protein requirements for felines while maintaining a Ketogenic Ratio of at least 1.5. The ketogenic pet food composition for felines may be complete and balanced nutrition for domesticated cats while also providing a Ketogenic Ratio of at least 1.5. The ketogenic pet food composition for felines may generally be represented by ketogenic dry kibble Ex. VIII of Example 5.

The pet food compositions of the present disclosure may be used in a method for treating or inhibiting cancer, inflammatory bowel disease, diabetes, obesity, hyperglycemia, hypertriglyceridemia, chronic inflammation, antibiotic resistance of the gut microbiome, or pathogenic dysbiosis of the gut microbiome in a companion animal, or any combination thereof. The method may include administering any one or more of the pet food compositions disclosed herein to a companion animal in need thereof.

The pet food composition of the present disclosure may be a ketogenic pet food composition for decreasing the formation of odd-chain short chain fatty acids that may inhibit or prevent the maintenance of a ketogenic state. Specifically, the ketogenic foods disclosed herein may decrease the production of odd-chain SCFA (C3:0, C5:0) from gut microbes as compared to conventional canine foods and conventional or state-of-the art commercial ketogenic foods. Illustrative odd-chain short chain fatty acids may be or include, but are not limited to, propionate (C3:0) and valerate (C5:0). The method may include feeding or administering any of the pet food compositions disclosed herein to a companion animal.

The pet food compositions of the present disclosure may be used in a method for improving gut health (e.g., gastrointestinal health) and/or providing oncology support with the ketogenic pet food and compositions disclosed herein. For example, the pet food composition of the present disclosure may be a ketogenic pet food composition that improves gut health and/or provides oncology support. For example, the ketogenic pet food composition disclosed herein may provide the following: decrease virulence factors in dogs fed the ketogenic pet food composition; decrease levels of genes coding for several bacterial enzymes known to breakdown and modify host colon epithelial saccharides; decrease levels of bacteria known to be pathogenic; inhibits the growth of pathogenic bacteria while concurrently promoting the growth of beneficial bacteria. The virulence factors may include one or more of *Enterococcus faecalis* GI 48190, *Clostridium perfringens* GENE nagK, *Clostridium perfringens* GENE nagH, *Escherichia coli* GENE stbA, *Clostridium perfringens* GENE cloSI, *Escherichia coli* GENE lacY, *Clostridium perfringens* GENE nagJ, *Clostridium perfringens* GENE pfoA, *Clostridium perfringens* GENE nagI, *Clostridium perfringens* GENE nagL, *Clostridium perfringens* GENE nanI, *Clostridium perfringens* GENE nanH, *Clostridium perfringens* GENE nanJ, *Escherichia coli* GENE ompA, *Clostridium perfringens* GENE plc, *Streptococcus pyogenes* GENE msrD, *Enterococcus faecium* GI 21886745, *Enterococcus faecium* GI 21886747, *Clostridium perfringens* GENE colA, or combinations thereof.

The beneficial bacteria may include one or more of *Clostridium hiranonis, Faecalibacterium prausnitzii,* butyrate producing bacterium, *Butyricicoccus pullicaecorum,* or combinations thereof. The pathogenic bacteria may include one or more of *Clostridium perfringens, Paeniclostridium sordellii, Clostridium baratii,* or combinations thereof. The ketogenic pet food composition surprisingly and unexpectedly promotes the growth of good bacteria and inhibits the growth of pathogenic bacteria in the same genus. For example, *C. perfringens* (pathogenic) decreased and *C. hiranonis* (beneficial) increased after feeding the dogs the exemplary pet food composition (Ex. IV) developed according to the embodiments disclosed herein. The ketogenic pet food composition may decrease virulence factors to undetectable levels.

The pet food compositions of the present disclosure may be used in a method for reducing antibiotic resistance genes (ABr) and/or multidrug resistance efflux proteins (MDR) in a gut microbiome of a companion animal to thereby improve overall pet health, support cancer recovery, and/or treat or inhibit chronic gastrointestinal disease (e.g., inflammatory bowel disease (IBD). For example, the ketogenic pet food compositions disclosed herein may significantly reduce ABr and/or MDR in animal fed the ketogenic pet food composition. The ketogenic pet food composition may aid against cachexia as well as decreases infection rates and improves the efficacy of antibiotics to treat infections in cancer and IBD patients. The ABr may include one or more of Macrolide mefA Membrane-fusion-protein emrA, Macrolide m 2021 Branch, Sensor-protein phoQ, Sensor-protein evgS, Involved-in-polymyxin-resistance pmrB, Bacitracinresistance bacA, polymyxin-and-cationic-antimicrobial-peptides arnA, Sensor-kinase cpxA, Macrolide lnuA, Macrolide mel, Sensor-kinase baeS, Involved-in-polymyxin-resistance pmrC, Membrane-fusion-protein emrK, Regulator cpxR, Membrane-fusion-protein acre, Innermembrane-transporter acrF, Beta-lactam pbp2 Ecoli, or combinations thereof. In at least one implementation, the ABr may not include one or more of Aminoglycoside aph2 Ib, Aminoglycoside aac6' Im, Aminoglycoside aph3' III, tetW, tet40, or combinations thereof. In yet another implementation, the ABr incudes tetM. The MDR may include one or more of efflux-pump mdtB, efflux-pump mdtA, efflux-pump mdtC, efflux-pump mdtD, transporter-for-efflux-complex mdtF, efflux-pump baeR, efflux-pump emrR, efflux-pump mdtN, repressor-for-mdr-efflux-pump crp, efflux-pump mdtO, efflux-pump acrD, repressor-of-efflux-complex acrS, or combinations thereof

The pet food compositions of the present disclosure may also be used in a method for inhibiting gut bacterial proteolysis and/or putrefaction to thereby reduce circulating phenols and indoles for supporting renal and gastrointestinal health. For example, the ketogenic pet food composition disclosed herein may reduce or inhibit gut bacterial proteolysis and/or putrefaction. The phenols may include one or more of 3-methoxycatechol sulfate (2), 3-methyl catechol sulfate (1), 4-acetylcatechol sulfate (1), 4-allylcatechol sulfate, 4-ethylcatechol sulfate, 4-hydroxycatechol sulfate, 4-methylcatechol sulfate, 4-vinylcatechol sulfate, catechol sulfate, 4-acetylphenyl sulfate, 4-aminophenol sulfate (2), 4-ethylphenyl sulfate, 4-hydroxyphenylacetate, 4-hydroxyphenylacetylglycine, 4-hydroxyphenylpyruvate, 4-methoxyphenol sulfate, 4-vinylphenol sulfate, or combinations thereof. In one example, the indoles may include one or more of 7-hydroxyindole sulfate, 5-hydroxyindole sulfate, 5-hydroxyindole glucuronide, 3-hydroxyindolin-2-one sulfate, indoxyl glucuronide, 3-formylindole, 6-hydroxyindole sulfate, indolin-2-one, 3-indoxyl sulfate, 5-hydroxyindoleacetate, indoleacetylglutamine, indoleacetate, 3-indoleglyoxylic acid, methyl indole-3-acetate, indoleacrylate, indolepropionate, indoleacetylglycine, indolelactate, 2-oxindole-3-acetate, indoleacetylalanine, or combinations thereof. In another example, the indoles may include one or more of indoxyl glucuronide, 3-formylindole, 6-hydroxyindole sulfate, indolin-2-one, 3-indoxyl sulfate, 5-hydroxyindoleacetate, indoleacetylglutamine, indoleacetate, 3-indoleglyoxylic acid, methyl indole-3-acetate, indoleacrylate, indolepropionate, indoleacetylglycine, indolelactate, 2-oxindole-3-acetate, indoleacetylalanine, or combinations thereof.

The pet food compositions of the present disclosure may be a ketogenic pet food composition capable of or configured to decrease proteinogenic amino acids, dipeptides formed from the proteinogenic amino acids, or combinations thereof. The dipeptides formed from the proteinogenic amino acids may include one or more of alanylleucine, glycylisoleucine, glycylleucine, glycylvaline, isoleucylglycine, leucylalanine, leucylglutamine, leucylglycine, lysylleucine, phenylalanylalanine, phenylalanylglycine, threonylphenylalanine, tryptophylglycine, tyrosylglycine, valylglutamine, valylglycine, valylleucine, or combinations thereof. The proteinogenic amino acids may include one or more of alanine, arginine, asparagine, aspartate, cysteine, cystine, glutamate, glutamine, glycine, histidine, isoleucine, leucine, lysine, methionine, phenylalanine, proline, serine, taurine, threonine, tryptophan, tyrosine, valine, or combinations thereof.

The pet food compositions of the present disclosure may be a ketogenic pet food composition that is more energy dense for supporting companion animals undergoing treatment for cancer. For example, the ketogenic pet food composition disclosed herein may have a metabolizable energy content that is about 10% to about 13% greater than existing ketogenic dry kibble diets.

In at least one implementation, the ketogenic pet food compositions disclosed herein may include one or more of chicken fat, dried eggs, chicken fat, dried eggs, meat protein isolate (pork), dried meat (chicken), met oil (c8:0, c10:0), hydrolyzed dried chicken, pecan shell fiber, whole flax seed, cellulose fiber, hydrolyzed casein protein isolate, hydrolyzed whey protein isolate, palatant, minerals, gelatin, potato amylose, amino acids, beet pulp fiber, citrus pulp fiber, lactic acid, soluble oat fiber, potassium citrate, apple pulp fiber, fish oil, psyllium husk fiber, vitamins, betaine, carnitine, alginate fiber, cranberry pulp fiber, methyl cellulose fiber, pectin fiber, taurine, antioxidant, or combinations thereof.

In at least one implementation, the ketogenic pet food compositions disclosed herein may include one or more of chicken fat, meat protein isolate (pork), dried eggs, met oil (c8:0, c10:0), dried meat (chicken), whole flax seed, palatant, pecan shell fiber, cellulose fiber, hydrolyzed casein protein isolate, hydrolyzed whey protein isolate, minerals, gelatin, potato amylose, beet pulp fiber, citrus pulp fiber, soluble oat fiber, lactic acid, potassium citrate, psyllium husk fiber, apple pulp fiber, fish oil, vitamin, betaine, alginate fiber, methyl cellulose fiber, pectin fiber, cranberry pulp fiber, taurine, carnitine, antioxidant, lipoic acid, or combinations thereof.

In another implementation, the ketogenic pet food composition disclosed herein may include one or more of chicken fat, meat protein isolate (pork), dried eggs, met oil (c8:0, c10:0), dried meat (chicken), whole flax seed, palatant, hydrolyzed dried chicken, pecan shell fiber, cellulose fiber, hydrolyzed casein protein isolate, hydrolyzed whey protein isolate, minerals, gelatin, potato amylose, beet pulp fiber, citrus pulp fiber, soluble oat fiber, lactic acid, potassium citrate, psyllium husk fiber, apple pulp fiber, fish oil, vitamin, betaine, alginate fiber, methyl cellulose fiber, pectin fiber, cranberry pulp fiber, taurine, carnitine, antioxidant, lipoic acid, or combinations thereof.

In another implementation, the ketogenic pet food composition disclosed herein may be a wet loaf including one or more of water, chicken necks, pork liver, wheat gluten, cellulose fiber, chicken fat, met (c8:0, c10:0), palatant, beef lung, non-digestible gum fiber #1, potassium citrate, betaine, carnitine, minerals, lipoic acid, vitamins, taurine, nutritional emulsifier #1, gelatin, or combinations thereof.

In yet another implementation, the ketogenic pet food composition disclosed herein may include one or more of chicken fat, dried eggs, meat protein isolate (pork), dried meat (chicken), met oil (c8:0, c10:0), hydrolyzed dried chicken, pecan shell fiber, whole flax seed, cellulose fiber, hydrolyzed casein protein isolate, hydrolyzed whey protein isolate, palatant, minerals, gelatin, potato amylose, amino acids, beet pulp fiber, citrus pulp fiber, lactic acid, soluble oat fiber, potassium citrate, apple pulp fiber, fish oil, psyllium husk fiber, vitamins, betaine, carnitine, alginate fiber, cranberry pulp fiber, methyl cellulose fiber, pectin fiber, taurine, antioxidant, or combinations thereof.

EXAMPLES

*Example 1*

Table 1 describes two exemplary compositions according to the present disclosure. The ingredients were obtained from commercial sources. An exemplary kibble diet was formulated according to AAFCO and NRC nutrition recommendations for adult canine maintenance (Ex. I). Each of the protein, fiber, and fat- type ingredients in the formulations adhere to the principles laid out above for specifications of ash, fat, carbohydrate levels for proteins, and fiber type for fibers. The compositions were formulated and produced via extrusion-based on the proposition that an optimized blend of proteins and fibers which would be able to largely replace starch in a dry food form for companion animals, and also provide enhanced benefits to companion animal health when consuming a ketogenic diet. The extruded kibble was dried before using vacuum enrobing to saturate the base kibble with fats and with palatants.

An exemplary treat diet (Ex. II) with nutritional characteristics appropriate for either domesticated canines or felines was formulated as shown in Table 1. The treat was produced via mixing, rolling and steam-convection baking. The baked treat was dried before consumption in a 100 °C oven for 6 hours to reduce moisture to approximately 8%.

The blend of fibers and proteins is envisioned to largely replace starch in a dry food form for companion animals.

**Table 1**

| Composition of Canine Dry Kibble Ex. I and Treat Ex. II | | |
|---|---|---|
| Ingredient | Ex. I (wt%) | Ex. II (wt%) |
| Dried Meat (Chicken) | 17 | 14 |
| Chicken Fat | 14.45 | 20 |
| Meat Protein Isolate (Pork) | 10 | 8 |
| Dried Eggs | 8.53 | 4 |
| MCT Oil (C8:0, C10:0) | 8 | 7 |

(continued)

| Composition of Canine Dry Kibble Ex. I and Treat Ex. II | | |
|---|---|---|
| Ingredient | Ex. I (wt%) | Ex. II (wt%) |
| Whole Flax Seed | 6 | 1.1 |
| Cellulose Fiber | 5 | 11 |
| Hydrolyzed Dried Chicken | 5 | 4 |
| Palatant | 5 | 3 |
| Insoluble Oat Fiber | 4 | 3 |
| Gelatin | 2 | 2 |
| Potato Amylopectin | 2 | 2 |
| Soybean Oil | 2 | 0 |
| Minerals | 1.85 | 0.6 |
| Beet Pulp Fiber | 1.5 | 1.1 |
| Citrus Pulp Fiber | 1.5 | 1.1 |
| Soluble Oat Fiber | 1.5 | 1.1 |
| Lactic acid | 1.2 | 1.1 |
| Psyllium Husk Fiber | 1 | 0.8 |
| Vitamins | 0.8 | 0.1 |
| Alginate Fiber | 0.5 | 0.4 |
| Methyl Cellulose Fiber | 0.5 | 0.4 |
| Pectin Fiber | 0.5 | 0.4 |
| Taurine | 0.13 | 0.1 |
| Antioxidant | 0.05 | 0 |
| Soybean Protein Isolate | 0 | 9 |
| Sodium Bicarbonate | 0 | 2 |
| Hydrolyzed Casein Protein Isolate | 0 | 2 |
| Hydrolyzed Whey Protein Isolate | 0 | 2 |

The summary of ketogenic potentials is shown in Table 2 for the Ex. I (Kibble), and Table 3 for Ex. II (Treat). The values for Ex. I are analytically determined for the final product kibble. These values are analytically determined through standard analyses for crude protein, crude fat, starch and total non-starch sugars. Total dietary fiber was measured as well, and comprises the sum of soluble and insoluble fibers. Values for Ex. II are predicted based on analysis of the ingredients in the formulation. For both tables, as well as for Tables 4 and 5, the wt.% of each ingredient is one-tenth of the column Macro g/kg.

**Table 2**

| Macronutrients and Ketogenic Potential for Canine Dry Kibble Ex. I | | | | |
|---|---|---|---|---|
| Macronutrient | Macro g/100 g | Macro kcal/100 g | Macro g/100 kcal | Macro kcal/100 kcal |
| Protein | 34.3 | 119.9 | 8.3 | 28.9 |
| Fat | 33.3 | 283.3 | 8.03 | 68.3 |
| Digestible Carbohydrate | 3.3 | 11.6 | 0.8 | 2.7 |
| Kcal | | | | 4147 |

(continued)

| Macronutrients and Ketogenic Potential for Canine Dry Kibble Ex. I | | | | |
|---|---|---|---|---|
| Macronutrient | Macro g/100 g | Macro kcal/100 g | Macro g/100 kcal | Macro kcal/100 kcal |
| Ketogenic ratio | | | | 1.73 |
| Total dietary fiber | | | | 14.9 |

**Table 3**

| Macronutrients and Ketogenic Potential for Canine or Feline Treat/Supplement Ex. II | | | | |
|---|---|---|---|---|
| Macronutrient | Macro g/100 g | Macro kcal/100 g | Macro g/100 kcal | Macro kcal/100 kcal |
| Protein | 33.8 | 118.4 | 6.9 | 24.3 |
| Fat | 43.5 | 369.6 | 8.9 | 75.7 |
| Digestible Carbohydrate | 1.3 | 4.4 | 0.3 | 0.9 |
| Kcal | | | | 4880 |
| Ketogenic ratio | | | | 2.17 |
| Total dietary fiber | | | | 10.8 |

The summaries of ketogenic potential of the only two currently commercially available comparative diets C and D are summarized in Tables 4 and 5. These values are analytically determined through standard analyses for crude protein, crude fat, starch and total non-starch sugars. Total dietary fiber was measured as well, and comprises the sum of soluble and insoluble fibers.

**Table 4**

| Macronutrients and Ketogenic Potential of Comparative Diet C | | | | |
|---|---|---|---|---|
| Macronutrient | Macro g/100 g | Macro kcal/100 g | Macro g/100 kcal | Macro kcal/100 kcal |
| Protein | 57.3 | 200.5 | 15.8 | 55.1 |
| Fat | 18.3 | 155.5 | 5.0 | 42.7 |
| Digestible Carbohydrate | 2.1 | 7.3 | 0.6 | 2 |
| Kcal | | | | 3635 |
| Ketogenic ratio | | | | 1.15 |
| Total dietary fiber | | | | 6.4 |

**Table 5**

| Macronutrients and Ketogenic Potential of Diet D | | | | |
|---|---|---|---|---|
| Macronutrient | Macro g/100 g | Macro kcal/100 g | Macro g/100 kcal | Macro kcal/100 kcal |
| Protein | 49.3 | 172.6 | 15.1 | 52.4 |
| Fat | 15.1 | 128.3 | 4.6 | 38.9 |
| Digestible Carbohydrate | 7.2 | 25.2 | 2.2 | 8.5 |
| Kcal | | | | 3689 |
| Ketogenic ratio | | | | 0.97 |
| Total dietary fiber | | | | 13.6 |

*Example 2*

Starch contributes significantly to durability so that kibble does not manifest breakage. Broken pieces of kibble, termed "fines", are aesthetically displeasing to the extent that they decrease customer acceptance of a product. It should be appreciated that a segment of the companion animal food customer base will not repurchase dog foods which contain a significant burden of fines. With starch nearly exclusively restricted from ketogenic dry kibbles, concerns arise about poor durability of these specialty foods as they are suspected to be prone to breakage. The durability of the exemplary dry kibble pet food composition (Ex. I) of Example 1 as well as an additional exemplary pet food composition (Ex. III; composition summarized in Table 6; Ketogenic potential summarized in Table 7) was compared with the two comparative pet food compositions (Diets C and D).

**Table 6**

| Composition of Canine Dry Kibble Ex. III | |
|---|---|
| **Ingredient** | **Ex.III (wt%)** |
| Chicken Fat | 15.09 |
| Meat Protein Isolate (Pork) | 11.25 |
| Dried Eggs | 10.5 |
| MCT Oil (C8:0, C10:0) | 8 |
| Dried Meat (Chicken) | 8 |
| Whole Flax Seed | 6 |
| Palatant | 5.2 |
| Pecan Shell Fiber | 5 |
| Cellulose Fiber | 4 |
| Hydrolyzed Casein Protein Isolate | 3 |
| Hydrolyzed Whey Protein Isolate | 3 |
| Minerals | 2.49 |
| Gelatin | 2 |
| Potato Amylose | 2 |
| Beet Pulp Fiber | 1.5 |
| Citrus Pulp Fiber | 1.5 |
| Soluble Oat Fiber | 1.5 |
| Lactic acid | 1.5 |
| Potassium citrate | 0.325 |
| Psyllium Husk Fiber | 1 |
| Apple Pulp Fiber | 1 |
| Fish Oil | 1 |
| Vitamin | 0.85 |
| Betaine | 0.75 |
| Alginate Fiber | 0.5 |
| Methyl Cellulose Fiber | 0.5 |
| Pectin Fiber | 0.5 |
| Cranberry Pulp Fiber | 0.5 |
| Taurine | 0.25 |

*Example 2*

(continued)

| Composition of Canine Dry Kibble Ex. III | |
|---|---|
| Ingredient | Ex.III (wt%) |
| Carnitine | 0.075 |
| Antioxidant | 0.05 |
| Lipoic Acid | 0.025 |

**Table 7**

| Macronutrients and Ketogenic Potential of Canine Dry Kibble Ex. III | | | | |
|---|---|---|---|---|
| Macronutrient | Macro g/100 g | Macro kcal/100 g | Macro g/100 kcal | Macro kcal/100 kcal |
| **Protein** | 33.2 | 116.2 | 8.0 | 28.1 |
| **Fat** | 33.0 | 280.5 | 8.0 | 67.8 |
| **Digestible Carbohydrate** | 4.7 | 16.45 | 1.1 | 3.9 |
| **Kcal** | | | | 4132 |
| **Ketogenic ratio** | | | | 1.65 |
| **Total dietary fiber** | | | | 14.7 |

Durability was tested with a standard assay whose implementation, context and relevancy of results would be apparent to someone skilled in the art of dry kibble extrusion. In brief, a weighed amount of dry kibble from a given diet was sieved on a screen with a mesh size selected to retain the intact kibbles. This released any broken fines through the screen and ensured that the durability test began with only intact kibbles. A weighed portion of intact kibbles was selected as 100%. Subsequently, the intact portion of kibbles was subjected to impact damage by shaking in a closed vessel with a standard metal weight for a standardized length of time and standardized intensity. After the induction of damage, the kibble was removed from the vessel and disposed onto the same screen as before, sieved and then the portion of intact kibble which had been retained on the screen were weighed. The percent of starting mass of kibbles remaining on the screen after induction of damage was termed the "Pellet Durability Index." It should be appreciated that this is omnibus assessment that can mimic the harsh conditions that dry kibbles are subjected to as they are transited in soft, collapsible bags. The results of this durability comparison are summarized in Table 8.

**Table 8**

| Durability of Ex. I and Ex. III relative to Comparative Diets C and D. | |
|---|---|
| Food | Pellet Durability Index Score (PDI) |
| Ex. I | 90.3 |
| Ex. III | 90.0 |
| Diet C | 52.3 |
| Diet D | 68.6 |

As illustrated by the data described in Table 8, the two separate exemplary kibble compositions (Ex. I) and (Ex. III) were significantly more durable than the comparative kibble Diets C and D. This demonstrates that the conceptualization and reduction to practice selected for Ex. I provided durability superior to the current state of the art, and that this improvement would be expected to add value by reducing waste from unusable broken kibble and improving customer acceptance.

*Example 3*

One having ordinary skill in the art would recognize that increased durability can be inversely related to appeal and palatability to animals. For example, durability may be driven to an extreme such that the dry kibble is no longer deemed appealing or palatable to dogs. Texture is an important contributing factor to palatability of foods for companion animals, and exceedingly increased durability could make the kibble difficult to eat. Durability improvements at the expense of palatability are not desirable and may even be counterproductive. Since Ex. I exhibited strongly increased durability compared to Diets C and D, it was suspected to be of lower palatability than the commercial diets. Thus, palatability testing in standardized format was performed which involved repeated testing of pet food compositions (Ex. 1) and (Ex. III), as well as an additional (Ex. IV, composition summarized in Table 9; Ketogenic potential summarized in Table 10), including testing multiple production lots for pet food composition (Ex. III) against Diet C and Diet D.

**Table 9**

| Composition of Canine Dry Kibble Ex. IV | |
|---|---|
| Ingredient | Ex. IV (wt%) |
| Chicken Fat | 13.26 |
| Meat Protein Isolate (Pork) | 10 |
| Dried Eggs | 10 |
| MCT Oil (C8:0, C10:0) | 8 |
| Dried Meat (Chicken) | 7 |
| Whole Flax Seed | 6 |
| Palatant | 5.2 |
| Hydrolyzed Dried Chicken | 5 |
| Pecan Shell Fiber | 5 |
| Cellulose Fiber | 4 |
| Hydrolyzed Casein Protein Isolate | 2.5 |
| Hydrolyzed Whey Protein Isolate | 2.5 |
| Minerals | 2.09 |
| Gelatin | 2 |
| Potato Amylose | 2 |
| Beet Pulp Fiber | 1.5 |
| Citrus Pulp Fiber | 1.5 |
| Soluble Oat Fiber | 1.5 |
| Lactic acid | 1.5 |
| Potassium citrate | 1.3 |
| Psyllium Husk Fiber | 1 |
| Apple Pulp Fiber | 1 |
| Fish Oil | 1 |
| Vitamin | 0.85 |
| Betaine | 0.75 |
| Alginate Fiber | 0.5 |
| Methyl Cellulose Fiber | 0.5 |
| Pectin Fiber | 0.5 |
| Cranberry Pulp Fiber | 0.5 |
| Taurine | 0.25 |
| Carnitine | 0.075 |

(continued)

| Composition of Canine Dry Kibble Ex. IV | |
|---|---|
| **Ingredient** | **Ex. IV (wt%)** |
| Antioxidant | 0.05 |
| Lipoic Acid | 0.025 |

**Table 10**

| Macronutrients and Ketogenic Potential of Canine Dry Kibble Ex. IV | | | | |
|---|---|---|---|---|
| **Macronutrient** | **Macro g/100 g** | **Macro kcal/100 g** | **Macro g/100 kcal** | **Macro kcal/100 kcal** |
| **Protein** | 30.9 | 108.15 | 7.6 | 26.6 |
| **Fat** | 32.8 | 278.8 | 8.06 | 68.5 |
| **Digestible Carbohydrate** | 5.6 | 19.6 | 1.37 | 4.8 |
| **Kcal** | | | | 4066 |
| **Ketogenic ratio** | | | | 1.63 |
| **Total dietary fiber** | | | | 14.2 |

Palatability was assessed in a standardized test. 25 dogs known not to exhibit sidedness to their bowl preference were offered two bowls with each bowl containing the day's caloric requirement of food. This was done on two successive days with the position of the bowls reversed on the second day. The weight of the bowls was recorded before and after the dogs were given access for 30 minutes to eat the foods. The weight of the Test food consumed divided of the weight of all food (Test and Control) consumed was termed the "Intake Ratio" and takes the form of = T/(T+C). An Intake Ratio of 0.5 indicates that there was no preference for either food. An Intake Ratio greater than 0.5 indicates the Test food was preferred over the Control food, with the implication that texture and taste were contributing factors to the increased appeal of the Test versus Control food. Even more informative is the percent of subjects in the palatability trial who ate more (prefer) the examples, prefer the comparative diets or have no preference.

In summary, the data described herein demonstrated that the improved durability provided by the pet food compositions according to the present disclosure did not adversely impact palatability, which was both surprising and unexpected. In contrast, the results show that the pet foods disclosed herein (represented by Ex. I, Ex. III, Ex, IV) provide remarkably improved palatability when compared to existing dry ketogenic kibbles (represented by Diets C and D). The pet foods disclosed herein (Ex. I, Ex. III, Ex, IV) did not lose a single palatability trial to either Diets C or D. Further, the wins were large in magnitude and statistically strong. Specifically, in three of five tests 100% of dogs preferred the exemplary pet foods (Ex. I, Ex. III, Ex, IV). In all tests greater than 90% of dogs preferred the exemplary pet foods (Ex. I, Ex. III, Ex, IV).

**Table 11**

| Palatability of Ex. I, Ex. III, Ex. IV relative to Diets C and D, | | | | |
|---|---|---|---|---|
| **Test Food** | **Control Food** | **Taste Preference % Dogs preferring Test/ Control/No Pref** | **PAL Test Intake Ratio** | **PAL Test p value** |
| Ex. III (lot 1) | Diet C | 92/8/0 | 0.77 | 0.0002 |
| Ex. III (lot 2) | Diet C | 100/0/0 | 0.95 | 0.0001 |
| Ex. I | Diet D | 100/0/0 | 0.83 | 0.0002 |
| Ex. IV | Diet D | 96/0/4 | 0.76 | 0.0002 |
| Ex. III (lot 1) | Diet D | 100/0/0 | 0.87 | 0.0002 |

(continued)

| Palatability of Ex. I, Ex. III, Ex. IV relative to Diets C and D, | | | | |
|---|---|---|---|---|
| Test Food | Control Food | Taste Preference % Dogs preferring Test/ Control/No Pref | PAL Test Intake Ratio | PAL Test p value |
| Ex. III (lot 2) | Diet D | 100/0/0 | 0.80 | 0.0001 |

## Example 4

Challenges associated with producing ketogenic companion animal foods that are severely restricted in digestible carbohydrate (starch) content are not limited to the production of dry extruded kibble. There are also widely recognized challenges associated with production of wet loaf forms of food that are typically sold in hermetically sealed cans. The challenge associated with producing ketogenic wet loaf food is different than the challenge of restricting starch in extruded dry kibble. In wet loaf products the high fat content tends to separate out from the bulk solids (e.g. proteins, fibers) during the cooking process of retort. While wet loaf companion animal foods may vary in texture from a dense loaf to a more watery stew, the commonality of preferred aesthetic is that the fats and oils are well integrated into the loaf and its associated liquids. Here we describe additional exemplary compositions of the present disclosure (Ex. V), (Ex. VI), and (Ex. VII). Specifically, wet forms of pet food compositions according to the embodiments disclosed herein. The composition of the wet pet food compositions (Ex. V), (Ex. VI), and (Ex. VII) is summarized in Table 12. As further demonstrated below, each of these wet loaf compositions were successfully prepared at various consistencies. The macronutrient makeup and Ketogenic Potential of the wet forms of the pet food compositions (Ex. V) and (Ex. VI) is summarized in Tables 13 and 14, respectively. The pet food compositions (Ex. VII) was prepared solely for purposes of comparing aesthetics and texture and was not analyzed for macronutrients or Ketogenic Potential Values. Tables 13 and 14 were analytically determined through standard analyses for crude protein, crude fat, starch and total non-starch sugars.

It is noted that there are currently no commercially available companion animal wet loaf form ketogenic foods, making it so that the only possible comparative example for palatability purposes is a standard canine adult maintenance formula. The macronutrient makeup and Ketogenic Potential of a comparative wet loaf diet (Diet E) is summarized shown in Table 15. Comparative Diet E was produced on the same day and at the same facility as pet food compositions Ex. V, Ex. VI, and Ex. VII. which reduced processing variations.

### Table 12

| Composition of Canine Wet Loaf (Ex. V), (Ex. VI), and (Ex. VII) | | | |
|---|---|---|---|
| Ingredient | Ex. V | Ex. VI | Ex. VII |
| | (Wt.%) | | |
| Water | 52.5 | 52.8 | 53.8 |
| Chicken Necks | 23.8 | 22.6 | 21.6 |
| Pork Liver | 8.6 | 9 | 8.3 |
| Wheat Gluten | 4 | 4 | 4 |
| Cellulose Fiber | 2.3 | 2 | 2.5 |
| Chicken Fat | 2.1 | 1.8 | 2 |
| MCT (C8:0, C10:0) | 2 | 2 | 2 |
| Palatant | 1.3 | 1.3 | 1.3 |
| Beef Lung | 1 | 1.23 | 1 |
| Non-Digestible Gum Fiber #1 | 1 | 2 | 0 |
| Potassium Citrate | 0.33 | 0.33 | 0.33 |
| Betaine | 0.2 | 0.2 | 0.2 |
| Carnitine | 0.2 | 0.2 | 0.2 |
| Minerals | 0.18 | 0.17 | 0.37 |

(continued)

| Composition of Canine Wet Loaf (Ex. V), (Ex. VI), and (Ex. VII) | | | |
|---|---|---|---|
| Ingredient | Ex. V | Ex. VI | Ex. VII |
| | (Wt.%) | | |
| Lipoic Acid | 0.15 | 0.15 | 0.15 |
| Vitamins | 0.11 | 0.10 | 0.10 |
| Taurine | 0.07 | 0.07 | 0.07 |
| Nutritional Emulsifier #1 | 0.01 | 0.01 | 0.01 |
| Gelatin | 0 | 0 | 2 |

**Table 13**

| Macronutrients and Ketogenic Potential of Ex. V | | | |
|---|---|---|---|
| Macronutrient | Macro g/100 g | Macro kcal/100 g | Macro g/100 kcal | Macro kcal/100 kcal |
| Protein | 38.5 | 134.8 | 7.9 | 28.0 |
| Fat | 39.7 | 337.4 | 8.1 | 70.3 |
| Digestible Carbohydrate | 4.4 | 15.4 | 0.9 | 1.5 |
| Kcal | | | | 4876 |
| Ketogenic ratio | | | | 1.74 |

**Table 14**

| Macronutrients and Ketogenic Potential of Ex. VI | | | |
|---|---|---|---|
| Macronutrient | Macro g/100 g | Macro kcal/100 g | Macro g/100 kcal | Macro kcal/100 kcal |
| Protein | 36.73 | 128.6 | 7.39 | 26.2 |
| Fat | 41.32 | 351.2 | 8.32 | 71.6 |
| Digestible Carbohydrate | 4.78 | 16.7 | 0.96 | 2 |
| Kcal | | | | 4965 |
| Ketogenic ratio | | | | 1.79 |

**Table 15**

| Macronutrients and Ketogenic Potential of Diet E | | | |
|---|---|---|---|
| Macronutrient | Macro g/100 g | Macro kcal/100 g | Macro g/100 kcal | Macro kcal/100 kcal |
| Protein | 25.9 | 90.6 | 11.1 | 22.6 |
| Fat | 18.1 | 153.9 | 6.5 | 38.5 |
| Digestible Carbohydrate | 44.3 | 155.1 | 4.5 | 38.8 |
| Kcal | | | | 3996 |
| Ketogenic ratio | | | | 0.46 |

After retort canning process was completed for Ex. V, Ex. VI, and Ex. VII, the cans were cooled for 3 days, opened into a plastic dish and assessed for the presence of oil and fat that had separated from the bulk solids.
It was readily apparent that the wet loaf pet food compositions (Ex. V) and (Ex. VI) had no apparent fat or oil separation from the bulk solids before cutting. No apparent fat or oil separation was observed after cutting and pressed either. Wet loaf pet food composition (Ex. VII), however, produced a fragile gel that collapsed to a watery stew upon cutting and pressing. The fat, however, was largely emulsified into the bulk solids for the wet loaf pet food composition (Ex. VII) and some separation was observed. Regarding pet food composition (Ex. VII), without being bound by theory, it is believed that the selected binder, namely gelatin, and the elimination of a nutritional gum fiber as binding agent produced the fragile gel. In view of the foregoing, it was surprisingly and unexpectedly discovered that wet loaf pet food compositions according to the embodiments disclosed herein may be prepared with a non-digestible gum fiber in the amount of from about 0.5 wt% to about 3 wt% and an emulsifier (i.e., lecithin) compatible with the companion nutrition and providing calories as fat. It was further surprisingly and unexpectedly discovered that wet loaf pet food compositions according to the embodiments disclosed herein may be prepared with or without gelatin.

The pet food compositions (Ex. V) and (Ex. VI) were tested in the same standardized palatability trials as described above for the dry kibble, except that Comparative Diet E was used as the control food. Table 16 summarizes the results of the palatability study.

**Table 16**

| Palatability of Wet Loaf Ex. V, Ex. VI relative to Diet E | | | | |
|---|---|---|---|---|
| Test Food | Control Food | Taste Preference % Dogs preferring Test/ Control/No Pref | PAL Test Intake Ratio | PAL Test p value |
| Ex. V | Diet E | 88/8/4 | 0.74 | 0.0002 |
| Ex. VI | Diet E | 88/12/0 | 0.70 | 0.0002 |

As indicated in Table 16, wet loafs (Ex. V) and (Ex. VI) exhibited improved palatability over Diet E. Without being bound by theory, it is believed that the inclusion of gum fiber and the emulsifier improved not only the texture, but also contributed to the increased preference, as texture is well recognized as an important factor in perceived palatability.

*Example 5*

It should be appreciated that treats and supplements are, by definition, not complete and balanced nutrition, and the Treat (Ex. II) of Example 1 was not designed, or required by companion animal nutrition governing body regulations, to be a complete and balanced nutrition. The expectation is that the companion animal will be receiving complete and balanced nutrition apart from the provision of the treats and supplements. Thus a dog consuming a ketogenic treat or supplement in the form of Treat (Ex. II) would still require a balanced nutritional food (e.g. Ex I, which is complete and balanced nutrition). However, the canine ketogenic compositions described above (Ex. I, Ex. III, Ex. IV, Ex. V, and Ex. VI) cannot simply be fed to domesticated felines as complete and balanced nutrition due to the special nutritional needs of cats as a species. This is similar to the concept that human-targeted ketogenic diets cannot simply be fed to companion animals due to species differences in nutritional requirements. As discussed above, it is non-trivial to adapt a ketogenic diet that fits the rigorous definition of a Ketogenic Ratio of at least 1.5, due the anti-ketogenic nature of dietary protein and the high requirement for protein in the diet of felines, which are obligate carnivores. For dogs, the American Association of Feed Control Officials (AAFCO) designates a dietary protein minimum of 4.5 g crude protein/100 kcal metabolizable energy (ME). While the canine pet food compositions (Ex. 1), (Ex. III), and (Ex. IV) are all significantly higher than this (between 7.6-8.3 g crude protein/100 kcal ME) and thus provide plenty of dietary proteins for nearly every possible feeding scenario, they are not appreciably larger than the feline minimum dietary protein requirement (6.5 crude protein/100 kcal ME) that has been assigned by the same governing body. Special care and novel approaches need to be taken when formulating a ketogenic diet for felines to provide adequate protein nutrition while still maintaining a Ketogenic Ratio of at least 1.5. Particularly, if foods are used to feed invalid pets recovering from disease, or as weight loss foods, or to sedentary pets, then reduced total intake of energy for the day in these situations puts cats at risk for protein malnourishment. However, the antiketogenic nature of protein prevents protein level increases at some point. There is only one existing commercially available feline nutrition-appropriate ketogenic dry kibble (summarized as Comparative Diet C in Table 4), which is a diet sold as appropriate for both dogs and cats. Diet C is high in protein at 15.8 crude protein/100 kcal ME, a value which is more than double the dietary requirement and prevents the cats eating this food from maximally expressing ketosis. Thus, the only existing commercial feline ketogenic dry kibble can be improved upon by incorporating the concept of the Ketogenic Ratio. Particularly, with such a high protein level, Diet C is necessarily limited in its Ketogenic Ratio, which is only 1.15 (Table 4), about 24% less ketogenic than a KR of 1.5.

A ketogenic dry kibble (Ex. VIII) according to the embodiments disclosed herein that provides a complete and balanced nutrition for domesticated cats while also providing a Ketogenic ratio of at least 1.5 was prepared. The composition of the ketogenic dry kibble (Ex. VIII) is summarized in Table 17. Analytical values for the macronutrient makeup and Ketogenic Ratio of this feline dry kibble Ex. VIII is summarized in Table 18. Total dietary fiber was measured as well, and comprises the sum of soluble and insoluble fibers.

**Table 17**

| Composition of Feline Dry Kibble (Ex. VII) | |
|---|---|
| Ingredient | Ex. IV (wt%) |
| Chicken Fat | 12.3 |
| Dried Eggs | 11 |
| Meat Protein Isolate (Pork) | 10 |
| Dried Meat (Chicken) | 7.2 |
| MCT Oil (C8:0, C10:0) | 6 |
| Hydrolyzed Dried Chicken | 5 |
| Pecan Shell Fiber | 5 |
| Whole Flax Seed | 5 |
| Cellulose Fiber | 4 |
| Hydrolyzed Casein Protein Isolate | 4 |
| Hydrolyzed Whey Protein Isolate | 4 |
| Palatant | 3.59 |
| Minerals | 2.09 |
| Gelatin | 2 |
| Potato Amylose | 2 |
| Amino Acids | 1.87 |
| Beet Pulp Fiber | 1.5 |
| Citrus Pulp Fiber | 1.5 |
| Lactic acid | 1.5 |
| Soluble Oat Fiber | 1.5 |
| Potassium citrate | 1.3 |
| Apple Pulp Fiber | 1 |
| Fish Oil | 1 |
| Psyllium Husk Fiber | 1 |
| Vitamins | 0.85 |
| Betaine | 0.75 |
| Carnitine | 0.075 |
| Alginate Fiber | 0.5 |
| Cranberry Pulp Fiber | 0.5 |
| Methyl Cellulose Fiber | 0.5 |
| Pectin Fiber | 0.5 |
| Taurine | 0.25 |
| Antioxidant | 0.05 |

**Table 18**

| Macronutrients and Ketogenic Potential of Feline Dry Kibble Ex. VIII | | | | |
|---|---|---|---|---|
| **Macronutrient** | **Macro g/100 g** | **Macro kcal/100 g** | **Macro g/100 kcal** | **Macro kcal/100 kcal** |
| **Protein** | 36.6 | 128.0 | 9.27 | 32.4 |
| **Fat** | 29.4 | 250.2 | 7.46 | 63.4 |
| **Digestible Carbohydrate** | 4.6 | 16.1 | 1.16 | 4 |
| **Kcal** | | | | 3943 |
| **Ketogenic ratio** | | | | 1.51 |
| **Total dietary fiber** | | | | 15.3 |

*Example 6*

The canine pet food composition (Ex. IV) of Example 4 was evaluated for its efficacy in decreasing odd-chain short chain fatty acids, namely, propionate (C3:0) and valerate (C5:0). The exemplary pet food composition (Ex. IV) was formulated according to AAFCO and NRC nutrition recommendations for adult maintenance. The composition was formulated and produced via extrusion based on the proposition that an optimized blend of fibers and proteins would be able to largely replace starch in a dry food form for companion animals. The extruded kibble (Ex. IV) was dried before using vacuum enrobing to saturate the base kibble with fats and subsequently coat with palatants. The exemplary pet food composition (Ex. IV) had a ketogenic ratio of about 1.71. It should be appreciated that the exemplary pet food composition (Ex. IV) included a combination of MCT and fish oil. The exemplary pet food composition (Ex. IV) further included an optimal fiber blend that provided gut microbes substrates that do not support odd-chain SCFA production. Specifically, the optimal fiber blend included sources of dietary non-digestible fibers that provide benefits to both companion animal nutrition, in that they nourish the gut microbiome, and promote acceptable stool quality. Ingredients were selected such that they provided the following fibers: cellulose, lignin, hemicellulose, pectin, alginate, and modified cellulose (including methylcellulose, hydroxyisopropylcellulose). As well, fiber having an average polysaccharide degree of polymerization of greater than 1000 and composed of glucose, arabinose, xylose, mannose, galactose monomers and sugar acid galacturonic acid. To provide the dual functional fibers, ingredients were a combination of purified celluloses, citrus pulp, pecan shell powder, beet pulp, cranberry pulp, apple pulp, flax seed, psyllium, and oat bran.

A preclinical randomized design crossover feeding trial was performed with canine subjects (n =35) having fecal collections in the 6th week of feeding the exemplary pet food composition as well as both a standard canine food and an existing commercial ketogenic food. The standard canine food is as described in Table 19, and the existing commercial ketogenic food was Diet D.

A dependent sample paired t-test was performed on log-base 2 transformed fecal short chain fatty acid levels when dogs consumed Ex. IV relative to when those dogs consumed Ketogenic Diet D and Standard Diet F. The study is summarized in Table 20.

A ketogenic state is often hampered by endogenous production of glucose through the process of gluconeogenesis (GNG). Glucose can be generated from several non-carbohydrate substrates for GNG, including glucogenic amino acids (e.g. alanine) and odd-chain fatty acids (e.g. propionate; C3:0 or valerate; C5:0). The odd-chain fatty acids that are most potent at producing glucose to prevent ketosis are C3:0 and C5:0. That is because the additional carbons (beyond C3) in longer odd-chain fatty acids (C7:0-C23:0, etc) are not converted to glucose in GNG, but instead are sequentially converted to acetyl-CoA via beta oxidation until only C3:0 remains. The C5:0 fatty acid is the immediate precursor to C3:0 after one round of beta oxidation.

Odd chain fatty acids are metabolized to glucose via the intermediacy of C3:0 entry into the Krebs' Cycle (TCA) and subsequently GNG. That C3:0 provision produces glucose in vivo from the liver and kidney, both canonical gluconeogenic tissues, is well documented. The flux of propionate into GNG has been quantified.

Whereas it has been proposed that production of glucose from amino acids through GNG is "demand-driven" rather than "supply-driven", such that additional dietary protein above amounts required for lean mass maintenance may not lead to untoward glucose production, the same cannot be said for GNG from C3:0. There is no available evidence indicating that C3:0 is diverted away from GNG when demand for glucose is low, rather glucose production from C3:0 proceeds unimpeded to the detriment of maintenance of ketosis.

Rigorous control of dietary protein:fat ratio, along with limiting dietary glucogenic amino acids can minimize the availability of substrates for GNG and thus minimize endogenous glucose production that prevents maintenance of ketosis. Odd-chain fatty acids are negligible in food; and thus, overt methods for decreasing odd-chain fatty acid availability for production of glucose through GNG by simply decreasing dietary levels are not likely to be successful. Aside from food as a source of odd-chain fatty acids, both C3:0 and C5:0 are generated by gut microbes in the colon irrespective of host status. Thus, C3:0 production is unregulated from a host standpoint; namely, no mechanism exists to hormonally decrease C3:0 production to protect ketosis.

Taken as a whole, it is apparent that current nutritional state of the art for achieving and maintaining ketosis is sorely lacking a mechanism to dampen production of glucose from gut microbe-produced C3:0 and C5:0 with the outcome that current technologies cannot maximize the level of ketosis that would occur if C3:0 and C5:0 production was decreased. The pet food compositions or pet food described herein is a ketogenic food which decreases odd-chain SCFA (C3:0, C5:0) production from gut microbes compared to both a standard canine food as well as an existing commercial ketogenic food.

**Table 19**

| Macronutrients and Ketogenic Potential of Diet F | | | | |
|---|---|---|---|---|
| **Macronutrient** | **Macro g/100 g** | **Macro kcal/100 g** | **Macro g/100 kcal** | **Macro kcal/100 kcal** |
| **Protein** | 23.69 | 82.9 | 7.06 | 24.7 |
| **Fat** | 14.63 | 124.4 | 4.36 | 37 |
| **Digestible Carbohydrate** | 36.6 | 128.1 | 10.91 | 38.1 |
| **Kcal** | | | | 3354 |
| **Ketogenic ratio** | | | | 0.46 |
| **Total dietary fiber** | | | | 10.6 |

Table 20

| Fecal Levels of Microbiome Odd-chain Fatty Acid GNG Precursor from Ex. IV vs. Diet D and Diet F | | | | |
|---|---|---|---|---|
| **Food** | **Fecal Propionate (C3:0)** | | **Fecal Valerate (5:0)** | |
| | **mean ± standard error ppm*** | **p value for difference from Ex. IV** | **mean ± standard error ppm*** | **p value for difference from Ex. IV** |
| Diet F | 3402.5 ± 149.9 | 0.015 | 345.5 ± 67.5 | < 0.0001 |
| Diet D | 3649.7 ± 157.9 | < 0.0001 | 124.4 ± 20.6 | 0.012 |
| Ex. IV | 2744.7 ± 138.2 | NA | 73.5 ± 12 | NA |
| * Fecal odd-chain fatty acid values are presented as ug fatty acid/g wet feces (parts per million, ppm) | | | | |

As illustrated in Table 20, dogs fed Ex. IV exhibited decreased fecal levels of both C3:0 and C5:0 odd-chain SCFA as compared to dogs consuming both the standard canine food (Diet F) and the commercial ketogenic food (Diet D). This was surprising and unexpected. As such, the exemplary pet food composition (Ex. IV) suppressed production of gluconeogenic C3:0 and C5:0 odd-chain SCFA in a manner that reduces circulating glucose and supports increased nutritional ketosis.

***Example 7***

The exemplary pet food composition (Ex. IV) was evaluated for its efficacy for reducing levels of gut bacterial virulence to provide benefits to oncology support and gastrointestinal health.

A randomized design crossover clinical feeding trial was performed with canine subjects (n = 35) having blood and fecal collections in the 6th week of feeding the exemplary pet food composition (Ex. IV), the existing commercial ketogenic food (Diet D), and the standard food (Diet F). Blood was assessed for clinical measures and circulating ketones to assess the degree to which the foods induced ketosis in dogs. Blood and fecal samples were analyzed by metabolomics profiling (high pressure liquid chromatography mass spectrometry; HPLC-MS) and feces were further analyzed for short chain fatty acid (also by HPLC-MS) and gut microbiome gene content (shotgun metagenomics sequencing).

The features in the exemplary pet food composition Ex. IV are compared to Diet D and Diet F in Table 21 relative to the ketogenic Diet D and the standard Diet F. The Percent Change from Diet D that was exhibited by Ex. IV is calculated as "(Ex. IV-Diet D)/Diet D", and equivalently for the percent change of Ex. IV from Diet F "(Ex. IV-Diet F)/Diet F".

Table 21

| Features between of Pet Food (IV) vs. Diet D and Diet F | | | | | |
|---|---|---|---|---|---|
| Nutrient (% weight) | Ex. IV | Diet D | Ex. IV Chg from Diet D | Diet F | Ex. IV Chg from Diet F |
| Fat Crude | 32.76 | 15.12 | 117% | 14.63 | 124% |
| Protein Crude | 30.94 | 49.31 | -37% | 23.69 | 31% |
| Starch | 4.6 | 6.4 | -28% | 35.5 | -87% |
| Sugars - Total | 0.97 | 0.74 | 31% | 1.1 | -12% |
| Ash | 5.66 | 8.55 | -34% | 4.89 | 16% |
| Acid Detergent Fiber | 3.7 | 6.2 | -40% | 3.7 | 0% |
| Fiber Crude | 5.8 | 5.3 | 9% | 2.2 | 164% |
| Fiber Insoluble | 12.5 | 12.9 | -3% | 8.5 | 47% |
| Fiber Neutral Detergent | 7.1 | 10.3 | -31% | 11.5 | -38% |
| Fiber Soluble | 1.7 | 0.7 | 143% | 2.1 | -19% |
| Fiber Total Dietary | 14.2 | 13.6 | 4% | 10.6 | 34% |
| C08:0 Octanoic (Caprylic) | 9.4% | 0.13% | 6985% | 0.14% | 6755% |
| C10:0 Decanoic (Capric) | 8.9% | 0.13% | 6662% | 0.14% | 6442% |
| C16:0 Hexadecanoic (Palmitic) | 16% | 20% | -18% | 19% | -14% |
| C16:1 Hexadecenoic (Palmitoleic) | 4% | 4% | -12% | 4% | -3% |
| C18:0 Octadecanoic (Stearic) | 5% | 7% | -28% | 5% | 0% |
| C18:1 Octadecenoic (Oleic + isomers) | 27% | 32% | -15% | 31% | -12% |
| C18:2 Omega 6 (Linoleic) | 12% | 19% | -35% | 24% | -49% |
| C18:3 Omega 3 (alpha-Linolenic) | 4% | 3% | 21% | 3% | 41% |
| C20:5 EPA Omega 3 | 0.55% | 0.13% | 315% | 0.14% | 302% |
| C22:6 DHA Omega 3 | 0.43% | 0.13% | 223% | 0.14% | 213% |
| Saturated Fatty Acids | 40% | 28% | 46% | 25% | 64% |
| Monounsaturated Fatty Acids | 32% | 37% | -15% | 35% | -11% |
| Polyunsaturated Fatty Acids | 19% | 24% | -22% | 28% | -32% |
| Omega 6 Sum | 13% | 20% | -35% | 25% | -46% |
| Omega 3 Sum | 5% | 4% | 43% | 3% | 76% |
| Omega 6/Omega 3 Ratio | 2.46 | 4.4 | -44% | 8.02 | -69% |
| * Fatty acids are presented as the percent of total fat only. All other analytes presented as percent by weight of total diet. | | | | | |

Ex. IV produced higher circulating blood ketone (BHB) levels compared to either the standard (non-ketogenic) Diet F or the existing commercial ketogenic dry kibble Diet D. The values for blood BHB in mmol/L were (mean $\pm$ standard error) Ex. IV 0.144 $\pm$ 0.014 mmol/L BHB; Standard Diet F 0.94 $\pm$ 0.003 mmol/L BHB; Ketogenic Diet D 0.0.101 $\pm$ 0.06 mmol/L BHB. When assessed by dependent sample t-test, the levels of BHB were statistically significantly higher (p = 0.001 vs Diet F and p = 0.006 vs Diet D). Thus pet food Ex IV produced circulating ketone levels that were on average 40% higher than the current state of the art ketogenic canine dry kibble.

While gut bacteria including the colonic microbiome provides essential functions to host health, they can also provoke disease through their virulence factors. Additionally, detrimental bacteria harbor genes which code for enzymes which can break down or modify host colonic epithelial cell surface saccharides to increase pathogenicity and virulence.

Although it is uniformly beneficial to decrease these virulence factors and cell surface modifying genes, a wholesale decrease of bacterial genera is decidedly not always beneficial. For example, Clostridium genus contains species which are harmful (perfringens sp) and also species which are beneficial (hiranonis sp).

While the ketogenic diet has proven efficacious for cancer support to prevent tumor burden/growth and aid in the management of cachexia, it is also criticized in lay and some scientific circles as detrimental to gut health since it may decrease beneficial bacteria or increase the pathogenicity of resident microbes.

A decrease in beneficial bacteria or an increase in virulence can be especially harmful to cancer patients who are undergoing radio or chemotherapy due to weakened immune system. An optimal ketogenic diet solution would not only provide high energy and preserve lean mass, but would also decrease virulence to prevent opportunistic infection in this at-risk population. The fecal microbiome shotgun metagenomics data provides information on the levels of microbial factors known to impact health and disease.

In this example, the levels of gut microbiome factors were evaluated from the following classes of markers: 1) virulence factors; 2) bacterial enzymes which modify host colon epithelial saccharides; and 3) bacteria which are pathogenic or beneficial. Pairwise t-tests were performed on the difference between fecal samples of dogs after consuming the exemplary pet food composition (Ex. IV) or the commercial ketogenic food (Diet D). The results are summarized in Tables 22-24. Values are presented as Reads Per Kilobase Million (RPKM) and differences expressed as the level in Ex. IV fed dogs as a percentage of the levels found when the dogs were eating the Commercial Ketogenic Diet D.

Table 22

| Levels of Microbial Virulence Factors in Feces | | | | |
|---|---|---|---|---|
| Virulence Factor | Ex. IV | Diet D | Ex. IV (%Chg) from Diet D | p value for Difference |
| Enterococcus faecalis GI 48190 | 0.008 | 0.147 | -95% | 2.50996E-05 |
| Clostridium perfringens GENE nagK | 3.75E-04 | 0.010 | -96% | 0.001 |
| Clostridium perfringens GENE nagH | 0 | 0.019 | -100% | 0.001 |
| Escherichia coli GENE stbA | 0 | 0.002 | -100% | 0.047 |
| Clostridium perfringens GENE cloSI | 0 | 0.005 | -100% | 0.002 |
| Escherichia coli GENE lacY | 1.42E-04 | 0.002 | -91% | 0.029 |
| Clostridium perfringens GENE nagJ | 0 | 0.013 | -100% | 0.001 |
| Clostridium perfringens GENE pfoA | 0 | 0.004 | -100% | 0.001 |
| Clostridium perfringens GENE nagI | 0 | 0.005 | -100% | 0.001 |
| Clostridium perfringens GENE nagL | 4.46E-04 | 0.011 | -96% | 0.002 |
| Clostridium perfringens GENE nanI | 0 | 0.006 | -100% | 0.001 |
| Clostridium perfringens GENE nanH | 0 | 0.005 | -100% | 0.001 |
| Clostridium perfringens GENE nanJ | 0 | 0.002 | -100% | 0.030 |
| Escherichia coli GENE ompA | 1.32E-04 | 0.004 | -97% | 0.010 |
| Clostridium perfringens GENE plc | 0.001 | 0.027 | -98% | 0.001 |
| Streptococcus pyogenes GENE msrD | 0 | 0.004 | -100% | 0.002 |
| Enterococcus faecium GI 21886745 | 0.019 | 0.054 | -66% | 0.013 |
| Enterococcus faecium GI 21886747 | 0.006 | 0.025 | -75% | 0.032 |
| Clostridium perfringens GENE colA | 0 | 0.009 | -100% | 2.38E-04 |
| Streptococcus pyogenes GI 52345264 | 0.601 | 0.252 | 139% | 3.26303E-07 |

**Table 23**

| Levels of Fecal Microbial Factors That Degrade Host Intestinal Cell Surface Saccharides | | | | |
|---|---|---|---|---|
| Host Cell Surface Saccharide Degrading Factor | Ex. IV | Diet D | Ex. IV (% Chg) from Diet D | p value for Difference |
| Hyaluronoglucosaminidase | 0.3 | 25.8 | -99% | 0.004 |
| Hyaluronoglucosaminidase 2 | 0.3 | 25.8 | -99% | 0.004 |
| Hyaluronoglucosaminidase 3 | 0.3 | 25.8 | -99% | 0.004 |
| superpathway of N-acetylneuraminate degradation | 343.7 | 2665.3 | -87% | 2.07E-11 |
| N-acylneuraminate-9-phosphatase | 0 | 0.3 | -100% | 0.021 |
| N-acylneuraminate-9-phosphatase 2 | 0 | 0.3 | -100% | 0.021 |
| N-acetylneuraminate epimerase | 0.3 | 3.0 | -90% | 0.040 |
| Exo-alpha-sialidase | 0 | 28.6 | -100% | 0.001 |
| mannosyltransferase activity | 116.1 | 273.5 | -58% | 7.22E-05 |

**Table 24**

| Levels of Pathogenic Microbes and Beneficial Microbes in Feces | | | | | |
|---|---|---|---|---|---|
| Bacteria | Bacterial Type | Ex. IV | Diet D | %Chg | P value |
| 1502_g_Clostridium(1485); s_Clostridium perfringens (1502) | Pathogen | 0.001 | 0.007 | -83% | 0.001 |
| 1505_g_Paeniclostridium(1849828); s_Paeniclostridium sordellii(1505) | Pathogen | 4.00E-05 | 1.41E-04 | -72% | 0.008 |
| 1561_g_Clostridium(1485); s_Clostridium baratii(1561) | Pathogen | 0 | 2.55E-04 | -100% | 0.045 |
| 89152_g _Peptostreptococcaceae_u_g(186804); s_ [Clostridium] hiranonis(89152) | Beneficial | 0.074 | 0.033 | 122% | 0.004 |
| 853_g_Faecalibacterium(216851); s_Faecalibacterium prausnitzii(853) | Beneficial | 0.004 | 0.001 | 215% | 0.000 |
| 245012_g_Clostridiales_u_g(186802); s_butyrate-producing bacterium SM4/1(245012) | Beneficial | 1.75E-04 | 0 | Increased from 0 | 0.002 |
| 501571_g_Butyricicoccus(580596); s_Butyricicoccus pullicaecorum(501571) | Beneficial | 0.002 | 3.25E-04 | 365% | 0.007 |

As illustrated in Table 22, there was a near uniform decrease in virulence factors in dogs fed the exemplary pet food composition. Specifically, 19 of 20 significant reductions were observed in the dogs fed the exemplary pet food composition. 11 of 20 virulence factors were eliminated. The mean$\pm$SD decrease in virulence factors by the exemplary pet food composition was about $95\pm9\%$.

The exemplary pet food composition (Ex. IV) also decreased levels of genes coding for several bacterial enzymes known to break down and modify host colon epithelial saccharides, as illustrated in Table 23. For 6 of 9 modifying enzymes, the exemplary pet food composition decreased the gene levels by 99% or more. The mean$\pm$SD decrease in virulence factors by the exemplary pet food composition was $92\pm14\%$.

The exemplary pet food composition further decreased levels of bacteria known to be pathogenic, as summarized in Table 24. Specifically, *C. Perfringens, C. baratii,* and *P. sordellii* were decreased by 83%, 72%, and 100% respectively. Table 24 further illustrated that the exemplary pet food composition increased beneficial bacteria by up to about 2.65 times higher than the comparative commercial ketogenic food (Diet D).

It was both surprising and unexpectedly discovered that two species within the same genus which are opposite

in their health effects on host physiology, *C. perfringens* (pathogenic) and *C. hiranonis* (beneficial) were significantly changed by the exemplary pet food composition (Ex. IV) in directions that are expected to benefit host or pet GI health. Specifically, *C. perfringens* decreased and *C. hiranonis* increased after feeding the dogs the exemplary pet food composition (Ex. IV) developed according to the embodiments disclosed herein.

**[0205]** In summary, the exemplary pet food composition (Ex. IV) dramatically decreased virulence factors, and the decrease was to undetectable levels of greater than 50% of changed virulence factors. The exemplary pet food composition (Ex. IV) further reduced the levels of genes coding for several bacterial enzymes known to break down and modify host colon epithelial saccharides. Further, the exemplary pet food composition (Ex. IV) decreased levels of bacteria known to be pathogens, and beneficial bacteria were increased, including opposing bacterial species within the same genus.

**[0206]** An impact of the exemplary pet food composition (Ex. IV) is an improvement for cancer therapy. For example, cancer therapy can depress immunocompetence and result in opportunistic infection from gut bacteria. As such, a pet food composition that can not only provide high energy food for lean body mass maintenance as well as decrease pathogenicity of gut bacteria would be optimal from a therapeutic standpoint. Accordingly, the exemplary pet food composition (Ex. IV) would not only aid the fight against cachexia, but also decrease infection rates in cancer patients.

***Example 8***

**[0207]** The exemplary pet food composition (Ex. IV) was evaluated for its efficacy for reducing levels of microbial antibiotic resistance genes and multidrug resistance efflux pump genes to provide benefits to oncology support and gastrointestinal health while concurrently providing a complete and balanced ketogenic nutrition.

**[0208]** While certain gut bacteria comprising the colonic microbiome provide essential functions to host or animal health, other bacteria that take up residence in the gut provoke disease; these latter bacteria are termed pathobionts. Modern medical drug regimens designed to thwart the growth and activities of pathobionts are often stymied by genetic elements existing in the pathobionts genome that prevent drug efficacy. Generally, drugs targeting pathobionts are called 'antibiotics', while the genetic elements housed in pathobionts that resist antibiotic action are called "antibiotic resistance genes" (hereafter, ABr). A special class of ABr that is particularly problematic for pet health due to the generality of their capacity to induce drug resistance are proteins which pump antibiotics out of a bacterial cell so that the intracellular concentration of the antibiotics decreases to a level where it is no longer efficacious against the pathobionts; these pumps are called multidrug resistance efflux proteins (hereafter, MDR).

**[0209]** There are at least two disease conditions whereby ABr and more specifically MDR can detrimentally impact pet health, and decrease chance of recovery/survival. In cancer, a weakened immune surveillance can lead to infiltration of pathobionts beyond the gut into pet tissues and lead to septicemia. Additionally, chronic gastrointestinal disease, e.g. inflammatory bowel disease (IBD), leads to a reduction in gut barrier integrity and increased invasiveness of pathobionts.

**[0210]** Metagenomics analysis of the ABr and MDR abundances was performed through sequencing of homogenized stool samples. A randomized design crossover clinical feeding trial was performed with canine subjects (n = 35) having fecal collections in the 6th week of feeding the exemplary pet food composition (Ex. IV) and the commercial ketogenic food (Diet D). A comparison of the features between the exemplary pet food composition (Ex. IV) and the commercial ketogenic food (Diet D) is shown in Table 21. Pairwise t-tests were performed on the difference between fecal samples of dogs after consuming the exemplary pet food composition (Ex. IV) or the commercial ketogenic food (Diet D) for the following classes of markers: 1) ABr; 2) MDR. The results are summarized in Table 25. Values for levels of ABR and MDR gene content in Table 25 are presented as RPKM and differences expressed as the level in Ex. IV fed dogs as a percentage of the levels found when the dogs were eating the Commercial Ketogenic Diet D.

**Table 25**

| Levels of Microbial Antibiotic Resistance and Multidrug Resistance Genes in Feces | | | | |
|---|---|---|---|---|
| **Antibiotic Resistance or Multi-Drug Resistance Gene** | **Ex. IV** | **Diet D** | **Ex. IV vs Diet D (%Chg)** | **p value** |
| Macrolide mefA | 0 | 4.1E-03 | -100% | 7E-04 |
| Membrane-fusion-protein emrA | 0 | 2.8E-03 | -100% | 2E-03 |
| MDR-Efflux-pump mdtB | 0 | 2.7E-03 | -100% | 0.01 |
| Macrolide m 2021 Branch | 0 | 2.3E-03 | -100% | 0.01 |
| Sensor-protein phoQ | 0 | 1.9E-03 | -100% | 3E-03 |
| Sensor-protein evgS | 0 | 1.8E-03 | -100% | 0.01 |

(continued)

| Levels of Microbial Antibiotic Resistance and Multidrug Resistance Genes in Feces | | | | |
|---|---|---|---|---|
| Antibiotic Resistance or Multi-Drug Resistance Gene | Ex. IV | Diet D | Ex. IV vs Diet D (%Chg) | p value |
| Involved-in-polymyxin-resistance pmrB | 0 | 1.6E-03 | -100% | 5E-03 |
| MDR-transporter emrY | 0 | 1.2E-03 | -100% | 0.03 |
| MDR-translocase mdfA | 0 | 7.9E-04 | -100% | 0.01 |
| Bacitracin-resistance bacA | 8.2E-05 | 2. 1E-03 | -96% | 0.01 |
| polymyxin-and-cationic-antimicrobialpeptides arnA | 8.8E-05 | 1.7E-03 | -95% | 0.02 |
| Sensor-kinase cpxA | 1.1E-04 | 2.8E-03 | -96% | 5E-03 |
| Repressor-of-efflux-complex acrS | 1.5E-04 | 2.0E-03 | -92% | 0.01 |
| MDR-Efflux-pump mdtA | 1.6E-04 | 2.1E-03 | -92% | 0.01 |
| MDR-Efflux-pump mdtC | 1.7E-04 | 2.7E-03 | -94% | 0.01 |
| Macrolide lnuA | 2.1E-04 | 3.6E-03 | -94% | 0.01 |
| MDR-transporter mdtK | 2.2E-04 | 2.6E-03 | -91% | 0.01 |
| MDR-Efflux-pump mdtD | 2.7E-04 | 3.6E-03 | -93% | 4E-03 |
| Macrolide mel | 2.7E-04 | 2.6E-03 | -90% | 0.03 |
| Sensor-kinase baeS | 2.9E-04 | 3.0E-03 | -90% | 0.01 |
| Transporter-for-efflux-complex mdtF | 3.2E-04 | 3.5E-03 | -91% | 3E-03 |
| Efflux-pump baeR | 3.6E-04 | 2.6E-03 | -86% | 0.02 |
| Involved-in-polymyxin-resistance pmrC | 3.6E-04 | 3.7E-03 | -90% | 3E-03 |
| Membrane-fusion-protein emrK | 4.5E-04 | 2.4E-03 | -81% | 0.03 |
| Regulator cpxR | 5.0E-04 | 3.6E-03 | -86% | 0.01 |
| Membrane-fusion-protein acrE | 5.1E-04 | 3.0E-03 | -83% | 0.02 |
| Repressor-for-mdr-efflux-pump emrR | 5.1E-04 | 2.3E-03 | -78% | 0.04 |
| Inner-membrane-transporter acrF | 5.3E-04 | 4.3E-03 | -88% | 5E-03 |
| MDR-Efflux-pump mdtN | 6.1E-04 | 3.1E-03 | -80% | 0.03 |
| Repressor-for-mdr-efflux-pump crp | 7.2E-04 | 4.0E-03 | -82% | 0.02 |
| MDR-Efflux-pump mdtO | 8.6E-04 | 3.4E-03 | -75% | 0.02 |
| MDR-Efflux-pump acrD | 8.7E-04 | 3.6E-03 | -76% | 0.02 |
| Beta-lactam pbp2 Ecoli | 1.7E-03 | 4.3E-03 | -60% | 0.05 |
| Tetracycline tetM | 3.2E-03 | 8.8E-02 | -96% | 6E-06 |
| Aminoglycoside aph2 Ib | 1.8E-02 | 1.2E-02 | 47% | 0.05 |
| Aminoglycoside aac6' Im | 2.4E-02 | 1.7E-02 | 46% | 0.02 |
| Aminoglycoside aph3' III | 4.5E-02 | 4.7E-03 | 844% | 1E-04 |
| Tetracycline tet40 | 4.9E-02 | 2.4E-02 | 106% | 9E-04 |
| Tetracycline tetW | 1.1E-01 | 5.3E-02 | 102% | 2E-03 |

In the analysis of ABr and MDR, 39 genetic elements were significantly different as determined by paired t-test in dogs that consumed both the commercial ketogenic food (Diet D) and the exemplary pet food composition (Ex. IV). Of these, about 87% (34 of 39) were statistically significantly decreased when the dogs consumed the exemplary pet food composition (Ex. IV) compared to when those same dogs consumed the conventional high fiber ketogenic food (Diet D). Further, about 26% (9 of 34) of those ABr/MDR elements reduced by the exemplary pet food composition (Ex. IV) were reduced to undetectable levels such that there was zero abundance of those ABr/MDR elements. Additionally, considering the 25 ABr/MDR elements reduced by the pet food composition (Ex. IV), but not reduced to undetectable levels, the average mean reduction in abundance by the exemplary pet food composition (Ex. IV) was 87% below the conventional high fiber ketogenic food (Diet D).

It must be noted that that Ex. IV was more effective in reducing levels of those enzymes which were not of the aminoglycoside/tetracycline class. In fact, levels of those genes increased when dogs consumed Ex. IV relative to Diet D. This novel and surprising finding makes it evident that pet foods prepared according to the embodiments disclosed herein are able to serve as a composition to follow on the results of veterinary testing for the type of antibiotic resistance that a companion animal patient is exhibiting. In this setting, the results of a fecal test for presence of ABr and MDR would inform a veterinarian practitioner on the appropriateness of intervening with the ketogenic kibble. In instances where veterinarians observe that a companion animal exhibits the presence of ABr in the aminoglycoside class (Aminoglycoside aph2 Ib, Aminoglycoside aac6' Im, Aminoglycoside aph3' III) or in the tetracycline class (tetW and tet40 but NOT tetM), then that veterinarian will consider other factors and solutions before providing the ketogenic kibble to that dog. Where ABr manifests in a companion animal that is not in the list of Aminoglycosides aph2 Ib, aac6' Im, aph3' III, or the two tetracycline genes tetW and tet40, then that veterinarian can personalize the nutrition for the companion animal by providing a ketogenic kibble that will aid in the management of that ABr.

In summary, the exemplary pet food composition dramatically decreased ABr and MDR, and the decrease was to undetectable levels for more than 25% of the ABr/MDR elements. It should be appreciated that the exemplary pet food composition supports or improves therapies for cancer and IBD. For example, cancer therapy can depress immunocompetence and result in opportunistic infection from gut bacteria. As such, an exemplary pet food composition that not only provides a high energy food for lean body mass maintenance as well as decreases pathogenic potential of gut bacteria would be optimal therapeutically. Further, since IBD results in loss of gut barrier integrity, an exemplary pet food composition that prevents resistance of invasive species to drug therapies would be a significant improvement on conventional high fiber ketogenic diets. Accordingly, the exemplary pet food compositions disclosed herein aid against cachexia, but also decreases infection rates and improving the efficacy of antibiotics to treat infections in cancer and IBD patients.

***Example 9***

The exemplary pet food composition (Ex. IV) was evaluated for its efficacy for inducing nutritional ketosis while inhibiting gut bacterial proteolysis and putrefaction to reduce circulating phenols and indoles with benefit for renal and gastrointestinal health.

Metabolomics analysis of the peptides and amino acids (proteolysis) as well as phenols and indoles (putrefactive uremic toxins) was performed by shotgun metabolomics of homogenized stool samples from dogs fed the exemplary pet food composition (Ex. IV) and the commercial ketogenic food (Diet F) in a cross-over design. The data was analyzed by Mixed-Model in JMP 15.0 with Diet as the independent variable and Subject as the random factor. Individual p values were assessed by Tukey's post hoc evaluation on least-squared means.

A randomized design crossover clinical feeding trial was performed with canine subjects (n = 35) having fecal collections in the 6th week of feeding the exemplary pet food composition (Ex. IV) as well as the existing commercial standard protein canine maintenance food (Diet F). A comparison of the protein, fat and non-digestible fiber features in the exemplary pet food composition and the commercial protein canine maintenance food is summarized in Table 21.

Statistical tests were performed on the difference between fecal samples from dogs after consuming the exemplary pet food composition (Ex. IV) or the canine maintenance food (Diet F) for the following classes of markers: 1) Incomplete proteolysis (fecal dipeptides) Table 26; 2) Complete proteolysis (fecal amino acids) Table 27; 3) Phenolic uremic toxins (serum phenols) Table 28; and 4) Indolic uremic toxins (serum indoles) Table 29. Values in these tables are presented as within-metabolite median centered relative fold concentration; unitless and directly comparable in magnitude.

**Table 26**

| Levels of Incomplete Microbial Proteolysis (fecal dipeptides) in Feces | | | | |
|---|---|---|---|---|
| Incomplete Microbial Proteolysis (fecal dipeptides) | Ex. IV | Diet F | Ex. IV % Chg from Diet F | p value for Difference |
| Alanylleucine | 0.67896 | 2.339348 | -71% | 6.82E-11 |
| Glycylisoleucine | 0.35017 | 1.192877 | -71% | 7.02E-09 |

(continued)

| Levels of Incomplete Microbial Proteolysis (fecal dipeptides) in Feces | | | | |
|---|---|---|---|---|
| Incomplete Microbial Proteolysis (fecal dipeptides) | Ex. IV | Diet F | Ex. IV % Chg from Diet F | p value for Difference |
| Glycylleucine | 0.599019 | 1.661004 | -64% | 7.56E-11 |
| Glycylvaline | 0.650996 | 1.598034 | -59% | 7.22E-11 |
| Isoleucylglycine | 0.843481 | 1.216579 | -31% | 0.000761 |
| Leucylalanine | 0.734504 | 1.917952 | -62% | 1.23E-06 |
| Leucylglutamine* | 0.755476 | 1.947234 | -61% | 6.43E-10 |
| Leucylglycine | 0.712905 | 1.40744 | -49% | 3.39E-07 |
| Lysylleucine | 0.642238 | 2.019659 | -68% | 6.67E-11 |
| Phenylalanylalanine | 0.83819 | 2.370833 | -65% | 6.4E-09 |
| Phenylalanylglycine | 0.673042 | 2.455475 | -73% | 6.56E-11 |
| Threonylphenylalanine | 0.653653 | 2.030109 | -68% | 6.46E-08 |
| Tryptophylglycine | 0.396095 | 1.884926 | -79% | 7.8E-11 |
| Tyrosylglycine | 0.543392 | 2.105668 | -74% | 6.58E-11 |
| Valylglutamine | 0.658899 | 1.667346 | -60% | 1.24E-10 |
| Valylglycine | 0.767937 | 1.220479 | -37% | 5.26E-06 |
| Valylleucine | 0.600566 | 1.912143 | -69% | 7.02E-11 |

One hundred percent of all detected fecal dipeptides, markers of incomplete proteolysis, were statistically significantly decreased after dogs consumed the exemplary pet food composition (Ex. IV) as compared to when they ate the standard maintenance food (Diet F). Further, the percent changes from Diet F indicated that these decreases in proteolysis had a large effect size. Dipeptides that can lead to uremic toxin production include phenylalanylalanine, phenylalanylglycine, threonylphenylalanine, tryptophylglycine, and tyrosylglycine.

Table 27

| Levels of Complete Microbial Proteolysis (fecal amino acids) in Feces | | | | |
|---|---|---|---|---|
| Incomplete Microbial Proteolysis (fecal amino acids) | Ex. IV | Diet D | Ex. IV % Chg from Diet D | p value for Difference |
| Alanine | 0.583305 | 1.70393 | -66% | 6.56E-11 |
| Arginine | 0.803655 | 2.492025 | -68% | 7.13E-11 |
| Asparagine | 0.825151 | 2.839506 | -71% | 5.33E-07 |
| Aspartate | 0.803006 | 1.686122 | -52% | 5.15E-08 |
| Cysteine | 0.930812 | 1.29131 | -28% | 5.43E-05 |
| Cystine | 0.177716 | 0.577658 | -69% | 1.27E-05 |
| Glutamate | 0.784302 | 1.78718 | -56% | 9.3E-11 |
| Glutamine | 0.423948 | 1.876374 | -77% | 6.56E-11 |
| Glycine | 0.895101 | 1.631457 | -45% | 7.47E-07 |
| Histidine | 0.510845 | 1.545362 | -67% | 2.42E-09 |
| Isoleucine | 0.322393 | 1.514294 | -79% | 6.56E-11 |
| Leucine | 0.364502 | 1.561918 | -77% | 6.56E-11 |
| Lysine | 0.720446 | 1.580469 | -54% | 6.56E-11 |

(continued)

| Levels of Complete Microbial Proteolysis (fecal amino acids) in Feces | | | | |
|---|---|---|---|---|
| Incomplete Microbial Proteolysis (fecal amino acids) | Ex. IV | Diet D | Ex. IV % Chg from Diet D | p value for Difference |
| Methionine | 0.554141 | 1.471399 | -62% | 6.56E-11 |
| Phenylalanine | 0.405673 | 1.697102 | -76% | 6.56E-11 |
| Proline | 0.741544 | 1.350273 | -45% | 7.46E-10 |
| Serine | 0.48052 | 1.599832 | -70% | 6.58E-11 |
| Taurine | 1.036436 | 1.956825 | -47% | 0.013045 |
| Threonine | 0.542342 | 1.734223 | -69% | 6.56E-11 |
| Tryptophan | 0.393755 | 2.382869 | -83% | 6.56E-11 |
| Tyrosine | 0.377214 | 1.646328 | -77% | 6.56E-11 |
| Valine | 0.406347 | 1.704691 | -76% | 6.57E-11 |

One hundred percent of all detected fecal amino acids, markers of complete proteolysis, were statistically significantly decreased after consuming the exemplary pet food composition (Ex. IV) as compared to when they ate the Diet F. Further, the percent change from Diet F indicated that these decreases in proteolysis had a large effect size. Amino acids which can lead to uremic toxin production include phenylalanine, tryptophan, and tyrosine.

**Table 28**

| Blood Levels of Phenol Microbial Uremic Toxins | | | | |
|---|---|---|---|---|
| Phenol Microbial Uremic Toxins | Ex. IV | Diet F | Ex. IV % Chg from Diet F | p value for Difference |
| 3-methoxycatechol sulfate (2) | 0.218312 | 1.62721 | -87% | 6.56E-11 |
| 3-methyl catechol sulfate (1) | 0.590691 | 0.745586 | -21% | 0.071318 |
| 4-acetylcatechol sulfate (1) | 0.704497 | 1.757119 | -60% | 5.26E-05 |
| 4-allylcatechol sulfate | 1.119384 | 1.350748 | -17% | 0.494836 |
| 4-ethylcatechol sulfate | 0.437517 | 1.604374 | -73% | 1.62E-09 |
| 4-hydroxycatechol sulfate | 0.883842 | 2.671948 | -67% | 6.56E-11 |
| 4-methylcatechol sulfate | 0.855452 | 0.684982 | 25% | 0.597498 |
| 4-vinylcatechol sulfate | 0.232509 | 1.775083 | -87% | 6.56E-11 |
| catechol sulfate | 0.739455 | 1.866309 | -60% | 3.88E-09 |
| 4-acetylphenyl sulfate | 0.907574 | 2.316639 | -61% | 7.27E-11 |
| 4-aminophenol sulfate (2) | 1.246978 | 1.027365 | 21% | 0.124098 |
| 4-ethylphenyl sulfate | 0.975327 | 2.383841 | -59% | 6.56E-11 |
| 4-hydroxyphenylacetate | 0.908592 | 1.025882 | -11% | 0.820699 |
| 4-hydroxyphenylacetylglycine | 0.955467 | 1.743548 | -45% | 0.000648 |
| 4-hydroxyphenylpyruvate | 0.928503 | 1.10299 | -16% | 0.035233 |
| 4-methoxyphenol sulfate | 0.864013 | 2.062473 | -58% | 6.78E-11 |
| 4-vinylphenol sulfate | 0.728018 | 2.766088 | -74% | 6.56E-11 |

Of the 17 uremic phenols related to kidney health (markers of putrefaction), 12 of 17 were significantly different between the exemplary pet food composition Ex. IV and the comparative Diet F. The 12 of 17 that were significantly different were significantly decreased after dogs consumed Ex. IV. Further, the percent changes from Diet F indicated that these decreases in phenolic putrefaction had a large effect size.

**Table 29**

| Levels of Indole Microbial Putrefaction in Feces | | | | |
|---|---|---|---|---|
| Indole Microbial Uremic Toxins | Ex. IV | Diet F | Ex. IV % Chg from Diet F | p value for Difference |
| 7-hydroxyindole sulfate | 1.232026 | 0.650907 | 89% | 6.56E-11 |
| 5-hydroxyindole sulfate | 1.058398 | 0.76465 | 38% | 9.44E-06 |
| 5-hydroxyindole glucuronide | 1.231713 | 0.9095 | 35% | 0.003812 |
| 3-hydroxyindolin-2-one sulfate | 0.705409 | 0.608328 | 16% | 0.35436 |
| indoxyl glucuronide | 1.222712 | 1.331629 | -8% | 0.669526 |
| 3-formylindole | 0.962064 | 1.086344 | -11% | 0.094407 |
| 6-hydroxyindole sulfate | 0.979632 | 1.135773 | -14% | 0.088118 |
| indolin-2-one | 0.95696 | 1.11455 | -14% | 0.132961 |
| 3-indoxyl sulfate | 0.916944 | 1.097317 | -16% | 0.015412 |
| 5-hydroxyindoleacetate | 1.076971 | 1.295932 | -17% | 0.014551 |
| indoleacetylglutamine | 1.451935 | 1.8999 | -24% | 0.145298 |
| indoleacetate | 1.063637 | 1.475397 | -28% | 0.146924 |
| 3-indoleglyoxylic acid | 0.833846 | 1.234187 | -32% | 5.99E-05 |
| methyl indole-3-acetate | 0.710019 | 1.064788 | -33% | 0.058879 |
| indoleacrylate | 0.851507 | 1.340905 | -36% | 2.61E-06 |
| indolepropionate | 0.98072 | 1.562922 | -37% | 8.78E-05 |
| indoleacetylglycine | 0.906647 | 1.674919 | -46% | 8.62E-06 |
| indolelactate | 0.643043 | 1.343272 | -52% | 7.54E-11 |
| 2-oxindole-3-acetate | 0.351962 | 1.480242 | -76% | 5.35E-05 |
| indoleacetylalanine | 0.216026 | 1.025889 | -79% | 6.56E-11 |

Of the 20 uremic indoles detected (markers of putrefaction), 12 were significantly different between the exemplary pet food composition and the control. 9 of the 12 were statistically significantly decreased after dogs ate the exemplary pet food composition (Ex. IV) compared to when they ate the maintenance food (Diet F) (with 3 of 12 increased). Further, the percent change from Diet F indicated that these decreases in indole putrefaction products had a large effect size.

It should be appreciated that the inventors surprisingly and unexpectedly discovered that the exemplary pet food composition disclosed herein which have 30% more protein and eight (8) times less carbohydrate still provide profoundly less proteolysis and putrefaction. The exemplary pet food composition may be utilized to improve renal health. The exemplary pet food composition allows for feeding relatively higher dietary protein while concurrently providing reduction in microbial putrefaction uremic toxins.

It should be appreciated by those skilled in the art of companion animal nutrition that increasing dietary protein leads to increased transit of undigested protein to the colon where it is subject to putrefaction by resident colon microbes to produce putrefactive metabolites which can be reabsorbed into the animal to decrease health and longevity. Generally, the higher the protein level in the diet, the higher the level of microbial putrefaction of undigested proteins to produce uremic toxins from the breakdown of aromatic amino acids (phenylalanine, tyrosine, tryptophan). However, we unexpectedly found that the pet food compositions disclosed herein reduce the levels of gut microbiome metabolites of aromatic amino acids breakdown (phenols and indoles). Without being bound by theory, it is believed that the selective inclusion of protein-containing ingredients that have high bioavailability, and the appropriate balancing with dietary fiber that led to this effect.

*Example 10*

Clearly the exemplary pet foods disclosed herein may be expected to offer benefits to a companion animal undergoing treatment for cancer. This is due to the following reasons- The exemplary pet foods disclosed herein are more energy dense, with a metabolizable energy content that is 10-13% greater than existing ketogenic dry kibble diets Diet C and Diet D. Cancer patients require increased energy to ameliorate the cachexia and sarcopenic muscle loss that accompanies the disease and it's treatment protocols. With the pet foods having 10-13% more energy density in the kibble, it would be expected that the pet foods improve on the ability of existing ketogenic dry kibbles to delivery adequate dietary energy to cancer patients.

Also, the exemplary pet foods disclosed herein are highly palatable and was preferred to the existing available ketogenic dry canine kibbles. This is important because the cancer patients often experience reduced appetite. As well, the molecular benefits to decrease microbial virulence factors, antibiotic resistance, multidrug resistance are expected to provide benefits to patients who are immune compromised and undergoing cancer therapy.

However, many cancer patients who are experiencing head and neck cancers will have difficulty chewing and eating food. Even if a food is highly palatable, the physical constraints of chewing can be a barrier to adequate energy intake in companion animal patients.

In view of the foregoing, the pet food composition (Ex. IV) along with ketogenic dry kibble Diet D and standard (non-ketogenic) dry kibble Diet F, were subjected to a Texture Probe Analysis to evaluate hardness, which may impact the ability of cancer patients to consume the food. In brief, Texture Probe Analysis was carried out on an instrument that applied force in a concentrated area on the respective dry kibble using a probe that mimicked a tooth, and then measured the resistance to the application of that force on the "tooth". Testing was conducted on a Stable Micro Systems TA XT Plus using a flat screwdriver shaped probe. Three parameters were captured, including: (1) the peak force recorded to break the kibble; (2) the contact time that the probe was in contact with the kibble before it completely fractured; and (3) the deformation distance in which the kibble compressed before breaking. 50 representative kibbles from each diet were selected and individually tested, with the results then averaged by diet. Results of the Texture Probe Analysis are summarized in Table 30.

**Table 30**

| Texture Probe Analysis | | | | | |
|---|---|---|---|---|---|
| Texture Probe Analysis Measurement | Ex. IV | Diet D | Ex. IV % Chg from Diet D | Standard Diet F | Ex. IV Percent CHANGE from Diet F |
| Peak force (kg) | 11.9 | 3.7 | 222% | 17.4 | -32% |
| Contact time (sec) | 0.19 | 0.17 | 12% | 0.28 | -32% |
| Deformation (mm) | 1.08 | 0.75 | 44% | 1.54 | -30% |

As illustrated in Table 30, pet food composition (Ex. IV) was approximately 30% lower in the amount of force required to break the kibble, and the same amount reduced in contact time and kibble deformation before breaking. In contrast, Ex. IV required around twice as much energy from the probe before breaking than did the ketogenic diet D. At the same time, Ex. IV had a slightly longer contact time with the simulated tooth and was more deformable before breaking than the comparative ketogenic Diet D. These results indicate that the exemplary pet food composition provides a unique and novel texture combination that may be a component underpinning the high palatability. It is also apparent that there are not concerns about the pet foods being too hard for a companion animal cancer patient to eat. Since the peak force is 1/3 less than a standard canine maintenance kibble, the exemplary pet foods are expected to be readily consumable by cancer patients.

*Example 11*

The exemplary pet food compositions (Ex. 1) and (Ex. IV) were evaluated for their efficacy for providing a high energy density with an appealing texture for pets undergoing or recovering from acute care for purposes of body weight maintenance or recovery.

The efficacy of the pet food composition (Ex. 1) and (Ex. IV) were evaluated on a 3.75 year old male castrated dog diagnosed with a soft tissue sarcoma affecting the region just above the left eye. Surgical removal and subsequent histopathology revealed incomplete removal of the tumor due to its highly invasive nature. Follow up radiation therapy was recommended after the dog had recovered. However, postoperative pain and the nature of the disease led to decreased appetite and significant weight loss with the dog dropping from 74.8 pounds to 66.8 pounds in 12 days making the dog a poorer candidate for radiation therapy due to the 11% loss of body weight. The exemplary pet food compositions (Ex. 1) and (Ex. IV) were fed to this dog, and both were well received with the dog consuming all portions offered. After 10 days the dog regained 4 pounds and by day 27 the dog regained 5.7 pounds. The pet owner reported the dog's energy level was back to normal. By day 54 the dog had regained 6.1 pounds and surgical wounds had healed and the dog was ready for additional radiation therapy. The dog continued to consume the exemplary pet food compositions (Ex. 1) and (Ex. IV) during radiation therapy and maintained body condition throughout therapy. The dog is now a healthy 1 year survivor of his cancer.

It should be appreciated that pets with cancer are subject to inappetence that may arise organically from their underlying disease or secondarily from medications given to slow cancer progression (chemotherapeutic agents), post-operative pain, or radiation therapy. It is generally accepted that a majority of dogs (about 69%) diagnosed with cancer experience some weight loss prior to their diagnosis and a significant percentage (about 35%) experience loss of muscle mass. Further, up to about 91% of cats have a history of muscle/weight loss prior to their diagnosis with cancer. Additionally, those cats with the lowest body condition generally exhibit the lowest survival times.

In view of the foregoing, stimulation of and maintenance of appetite in pets with cancer is a significant factor in maintaining, elevating, and extending quality of life and extending lifespan in pets with cancer. Further, pets with cancer or other debilitating diseases may have decreased strength or ability to prehend a typical quantity of food. Therefore, the combination of a food that is energy dense, complete in protein and highly palatable and digestible would be particularly advantageous because a pet could consume their necessary daily quantity of calories, protein, fat, carbohydrates, vitamins, and minerals in a smaller, energy dense meal.

## Claims

1. A pet food composition, comprising:

   greater than 33 wt% fat;
   a protein source;
   less than 7 wt% carbohydrates; and
   greater than 10 wt% fiber;
   wherein $(0.9 F + 0.46 P) / (0.1 F + 0.58 P + C)$ is 1.5 or greater, wherein F, P, and C, are wt% of fat, protein, and carbohydrates, respectively.

2. The pet food composition according to claim 1, wherein the pet food composition is **characterized by** one or more of the following features:

   (i) the pet food composition comprises greater than 14 wt% of fiber, preferably, greater than 16 wt% of fiber;
   (ii) the pet food composition comprises less than 6 wt% carbohydrates, preferably less than 5 wt% carbohydrates, preferably less than 3 wt% carbohydrates, or more preferably less than 1.5 wt% of carbohydrates;
   (iii) the fat is present in an amount of greater than 34 wt%, greater than 35 wt%, preferably greater than 36 wt%;
   (iv) the fat comprises at least 20 wt% medium chain triglycerides (MCT), in particular from 10 wt% to 35 wt% MCT, 20 wt% to 35 wt% MCT, 20 wt% to 30 wt% MCT, or preferably 20 wt% to 25 wt% MCT;
   (v) the pet food composition further comprises from 4.5 g to 14 g of protein per 100 kcal metabolizable energy, 6 g to 12 g of protein per 100 kcal metabolizable energy, or more preferably from 7 g to 10 g of protein per 100 kcal metabolizable energy.

3. The pet food composition according to any foregoing claim, wherein the pet food composition is **characterized by** one or both of the following features:

   (i) the pet food composition further comprises ash in an amount of less than 7 wt%, preferably less than 6 wt%, or more preferably less than 3 wt%;
   (ii) the pet food composition further comprises phosphorus in an amount of 1 wt% or less, 0.8 wt% or less, or preferably 0.7 wt.% or less.

4. The pet food composition of claim 1, further comprising an optimal fiber blend comprising: cellulose, lignin, hemicellulose, pectin, alginate, and modified cellulose.

5. The pet food composition of claim 1, further comprising:

water in an amount of from 50 wt% to 60 wt%;
wherein (0.9 F + 0.46 P) / (0.1 F + 0.58 P + C) is greater than 1.5,
wherein the pet food composition is in the form of a wet loaf.

6. The pet food composition of claim 1, wherein the pet food composition is a feline pet food composition, further comprising:

protein in an amount of from 6.5 g to 14 g of protein per 100 kcal metabolizable energy;
less than 5 wt% carbohydrates; and
wherein (0.9 F + 0.46 P) / (0.1 F + 0.58 P + C) is greater than 1.5.

7. The pet food composition of any one of claims 1-6 for use in a method of treating high blood glucose, chronic inflammation, metabolic stress, or cancer in a companion animal;
optionally, wherein the companion animal is selected from a canine and a feline.

8. The pet food composition of any one of claims 1-6 for use in a method for promoting or facilitating maintenance of a ketogenic state in a companion animal by decreasing the formation of odd-chain short chain fatty acids (SCFA) in the companion animal;
optionally, wherein the odd-chain SCFA comprise one or more of propionate, valerate, or combinations thereof.

9. The pet food composition of any one of claims 1-6 for use in a method for decreasing virulence factors in a companion animal;
optionally, wherein the method is **characterized by** one or both of the following features:

(i) the virulence factors comprise one or more of: *Enterococcus faecalis* GI 48190, *Clostridium perfringens* GEN E nagK, *Clostridium perfringens* GEN E nagH, *Escherichia coli* GENE stbA, *Clostridium perfringens* GEN E cloSI, *Escherichia coli* GENE lacY, *Clostridium perfringens* GENE nagJ, *Clostridium perfringens* GENE pfoA, *Clostridium perfringens* GENE nagI, *Clostridium perfringens* GENE nagL, *Clostridium perfringens* GENE nanI, *Clostridium perfringens* GENE nanH, *Clostridium perfringens* GENE nanJ, *Escherichia coli GEN E* ompA, *Clostridium perfringens* GEN E plc, *Streptococcus pyogenes* GENE msrD, *Enterococcus faecium* GI 21886745, *Enterococcus faecium* GI 21886747, *Clostridium perfringens* GENE colA, or combinations thereof;
(ii) decreasing the virulence factors prevents or inhibits infections in the companion animal.

10. The pet food composition of any one of claims 1-6 for use in a method for inhibiting growth of pathogenic bacteria and promoting growth of beneficial bacteria in the gut of a companion animal;
optionally, wherein the method is **characterized by** one or more of the following features:

(i) the beneficial bacteria comprise one or more of *Clostridium hiranonis, Faecalibacterium prausnitzii,* butyrate producing bacterium, *Butyricicoccus pullicaecorum,* or combinations thereof;
(ii) the pathogenic bacteria comprise one or more of *Clostridium perfringens, Paeniclostridium sordellii, Clostridium baratii,* or combinations thereof;
(iii) the beneficial bacteria and the pathogenic bacteria are in the same genus;
(iv) the pathogenic bacteria comprise *C. perfringens,* and the beneficial bacteria comprise *C. hiranonis.*

11. The pet food composition of any one of claims 1-6 for use in a method for reducing levels of microbial antibiotic resistance genes (ABr) in a gut microbiome of a companion animal;
optionally, wherein the ABr are **characterized by** one or more of the following features:

(i) the ABr comprise one or more of Macrolide mefA Membrane-fusion-protein emrA, Macrolide m 2021 Branch, Sensor-protein phoQ, Sensor-protein evgS, Involved-in-polymyxin-resistance pmrB, Bacitracin-resistance bacA, polymyxin-and-cationic-antimicrobial-peptides arnA, Sensor-kinase cpxA, Macrolide InuA, Macrolide mel, Sensor-kinase baeS, Involved-in-polymyxin-resistance pmrC, Membrane-fusion-protein emrK, Regulator cpxR, Membrane-fusion-protein acre, Inner-membrane-transporter acrF, Beta-lactam pbp2 Ecoli, or combinations thereof;
(ii) the Abr does not comprise one or more of Aminoglycoside aph2 Ib, Aminoglycoside aac6' Im, Aminoglycoside aph3' III, tetW, tet40, or combinations thereof;
(iii) the ABr comprises tetM.

**12.** The pet food composition of any one of claims 1-6 for use in a method for reducing levels of multidrug resistance efflux pump genes in a gut microbiome of a companion animal;
optionally, wherein the multidrug resistance efflux pump genes comprise one or more of efflux-pump mdtB, efflux-pump mdtA, efflux-pump mdtC, efflux-pump mdtD, transporter-for-efflux-complex mdtF, efflux-pump baeR, efflux-pump emrR, efflux-pump mdtN, repressor-for-mdr-efflux-pump crp, efflux-pump mdtO, efflux-pump acrD, repressor-of-efflux-complex acrS, or combinations thereof.

**13.** The pet food composition of any one of claims 1-6 for use in a method for supporting or improving renal health of a companion animal by reducing blood circulating levels of phenols and indoles in the companion animal;

wherein the phenols preferably comprise one or more of 3-methoxycatechol sulfate (2), 3-methyl catechol sulfate (1), 4-acetylcatechol sulfate (1), 4-allylcatechol sulfate, 4-ethylcatechol sulfate, 4-hydroxycatechol sulfate, 4-methylcatechol sulfate, 4-vinylcatechol sulfate, catechol sulfate, 4-acetylphenyl sulfate, 4-aminophenol sulfate (2), 4-ethylphenyl sulfate, 4-hydroxyphenylacetate, 4-hydroxyphenylacetylglycine, 4-hydroxyphenylpyruvate, 4-methoxyphenol sulfate, 4-vinylphenol sulfate, or combinations thereof;
wherein the indoles preferably comprise one or more of 7-hydroxyindole sulfate, 5-hydroxyindole sulfate, 5-hydroxyindole glucuronide, 3-hydroxyindolin-2-one sulfate, indoxyl glucuronide, 3-formylindole, 6-hydroxyindole sulfate, indolin-2-one, 3-indoxyl sulfate, 5-hydroxyindoleacetate, indoleacetylglutamine, indoleacetate, 3-indoleglyoxylic acid, methyl indole-3-acetate, indoleacrylate, indolepropionate, indoleacetylglycine, indolelactate, 2-oxindole-3-acetate, indoleacetylalanine, or combinations thereof, and more preferably comprise one or more of indoxyl glucuronide, 3-formylindole, 6-hydroxyindole sulfate, indolin-2-one, 3-indoxyl sulfate, 5-hydroxyindoleacetate, indoleacetylglutamine, indoleacetate, 3-indoleglyoxylic acid, methyl indole-3-acetate, indoleacrylate, indolepropionate, indoleacetylglycine, indolelactate, 2-oxindole-3-acetate, indoleacetylalanine, or combinations thereof.

**14.** The pet food composition of any one of claims 1-6 for use in a method for supporting or improving renal health in a companion animal by reducing microbial proteolysis in the colon of the companion animal;
optionally, wherein the pet food composition decreases proteinogenic amino acids, dipeptides formed from the proteinogenic amino acids, or combinations thereof; wherein the dipeptides formed from the proteinogenic amino acids preferably comprise one or more of alanylleucine, glycylisoleucine, glycylleucine, glycylvaline, isoleucylglycine, leucylalanine, leucylglutamine, leucylglycine, lysylleucine, phenylalanylalanine, phenylalanylglycine, threonylphenylalanine, tryptophylglycine, tyrosylglycine, valylglutamine, valylglycine, valylleucine, or combinations thereof; wherein the proteinogenic amino acids preferably comprise one or more of alanine, arginine, asparagine, aspartate, cysteine, cystine, glutamate, glutamine, glycine, histidine, isoleucine, leucine, lysine, methionine, phenylalanine, proline, serine, taurine, threonine, tryptophan, tyrosine, valine, or combinations thereof.

**Patentansprüche**

**1.** Haustierfutterzusammensetzung, umfassend:

mehr als 33 Gew.-% Fett;
eine Proteinquelle;
weniger als 7 Gew.-% Kohlenhydrate und
mehr als 10 Gew.-% Ballaststoffe,
wobei (0,9 F + 0,46 P) / (0,1 F + 0,58 P + C) 1,5 oder größer ist, wobei F, P und C Gewichtsprozente von Fett, Protein bzw. Kohlenhydraten sind.

**2.** Haustierfutterzusammensetzung nach Anspruch 1, wobei die Haustierfutterzusammensetzung durch eines oder mehrere der folgenden Merkmale gekennzeichnet ist:

(i) die Haustierfutterzusammensetzung umfasst mehr als 14 Gew.-% Ballaststoffe, vorzugsweise mehr als 16 Gew.-% Ballaststoffe;
(ii) die Haustierfutterzusammensetzung enthält weniger als 6 Gew.-% Kohlenhydrate, vorzugsweise weniger als 5 Gew.-% Kohlenhydrate, vorzugsweise weniger als 3 Gew.-% Kohlenhydrate oder noch bevorzugter weniger als 1,5 Gew.-% Kohlenhydrate;
(iii) das Fett ist in einer Menge von mehr als 34 Gew.-%, mehr als 35 Gew.-%, vorzugsweise mehr als 36 Gew.-% vorhanden;

(iv) das Fett umfasst mindestens 20 Gew.-% mittelkettige Triglyceride (MCT), insbesondere von 10 Gew.-% bis 35 Gew.-% MCT, 20 Gew.-% bis 35 Gew.-% MCT, 20 Gew.-% bis 30 Gew.-% MCT oder vorzugsweise 20 Gew.-% bis 25 Gew.-% MCT;

(v) die Haustierfutterzusammensetzung umfasst ferner 4,5 g bis 14 g Protein pro 100 kcal metabolisierbarer Energie, 6 g bis 12 g Protein pro 100 kcal metabolisierbarer Energie oder noch bevorzugter 7 g bis 10 g Protein pro 100 kcal metabolisierbarer Energie.

3. Haustierfutterzusammensetzung nach einem der vorstehenden Ansprüche, wobei die Haustierfutterzusammensetzung durch eines oder beide der folgenden Merkmale gekennzeichnet ist:

(i) die Haustierfutterzusammensetzung umfasst ferner Asche in einer Menge von weniger als 7 Gew.-%, vorzugsweise weniger als 6 Gew.-% oder noch bevorzugter weniger als 3 Gew.-%;

(ii) die Haustierfutterzusammensetzung enthält ferner Phosphor in einer Menge von 1 Gew.-% oder weniger, 0,8 Gew.-% oder weniger, oder vorzugsweise 0,7 Gew.-% oder weniger.

4. Die Haustierfutterzusammensetzung nach Anspruch 1, welche ferner eine optimale Ballaststoffmischung umfasst, die Folgendes umfasst: Cellulose, Lignin, Hemicellulose, Pektin, Alginat und modifizierte Cellulose.

5. Haustierfutterzusammensetzung nach Anspruch 1, die ferner Folgendes umfasst:

Wasser in einer Menge von 50 Gew.-% bis 60 Gew.-%;
wobei (0,9 F + 0,46 P) / (0,1 F + 0,58 P + C) größer als 1,5 ist,
wobei die Haustierfutterzusammensetzung in Form eines feuchten Laibes vorliegt.

6. Haustierfutterzusammensetzung nach Anspruch 1, wobei die Haustierfutterzusammensetzung eine Katzenfutterzusammensetzung ist, die ferner Folgendes umfasst:

Protein in einer Menge von 6,5 g bis 14 g Protein pro 100 kcal metabolisierbarer Energie;
weniger als 5 Gew.-% Kohlenhydrate; und
wobei (0,9 F + 0,46 P) / (0,1 F + 0,58 P + C) größer als 1,5 ist.

7. Haustierfutterzusammensetzung nach einem der Ansprüche 1-6 zur Verwendung in einem Verfahren zur Behandlung von hohem Blutzucker, chronischer Entzündung, metabolischem Stress oder Krebs bei einem Heimtier; wobei das Heimtier wahlweise aus einem Hund oder einer Katze ausgewählt ist.

8. Haustierfutterzusammensetzung nach einem der Ansprüche 1 bis 6 zur Verwendung in einem Verfahren zur Förderung oder Erleichterung der Aufrechterhaltung eines ketogenen Zustands bei einem Heimtier durch Verringerung der Bildung von ungeradkettigen kurzkettigen Fettsäuren (SCFA) in dem Heimtier; wahlweise, wobei die ungeradkettigen SCFA eines oder mehrere von Propionat, Valerat oder Kombinationen davon umfassen.

9. Haustierfutterzusammensetzung nach einem der Ansprüche 1-6 zur Verwendung in einem Verfahren zur Verringerung von Virulenzfaktoren in einem Heimtier; wahlweise, wobei das Verfahren durch eines oder beide der folgenden Merkmale gekennzeichnet ist:

(i) die Virulenzfaktoren umfassen einen oder mehrere von: *Enterococcus faecalis* GI 48190, *Clostridium perfringens* GENE nagK, *Clostridium perfringens* GENE nagH, *Escherichia coli* GENE stbA, *Clostridium perfringens* GENE cloSI, *Escherichia coli* GENE lacY, *Clostridium perfringens* GENE nagJ, *Clostridium perfringens* GENE pfoA, *Clostridium perfringens* GENE nagI, *Clostridium perfringens* GENE nagL, *Clostridium perfringens* GENE nanI, *Clostridium perfringens* GENE nanH, *Clostridium perfringens* GENE nanJ, *Escherichia coli* GENE ompA, *Clostridium perfringens* GENE plc, *Streptococcus pyogenes* GENE msrD, *Enterococcus faecium* GI 21886745, *Enterococcus faecium* GI 21886747, *Clostridium perfringens* GENE colA, oder Kombinationen davon;

(ii) die Verringerung der Virulenzfaktoren verhindert oder hemmt Infektionen bei dem Heimtier.

10. Haustierfutterzusammensetzung nach einem der Ansprüche 1 bis 6 zur Verwendung in einem Verfahren zur Hemmung des Wachstums pathogener Bakterien und zur Förderung des Wachstums nützlicher Bakterien im Darm eines Heimtieres; wahlweise, wobei das Verfahren durch eines oder mehrere der folgenden Merkmale gekennzeichnet ist:

(i) die nützlichen Bakterien umfassen eines oder mehrere der Folgenden: *Clostridium hiranonis, Faecalibacterium prausnitzii,* ein Butyrat produzierendes Bakterium, Butyricicoccus pullicaecorum, oder Kombinationen davon;

(ii) die pathogenen Bakterien umfassen eines oder mehrere der Folgenden: *Clostridium perfringens, Paeniclostridium sordellii, Clostridium baratii,* oder Kombinationen davon;

(iii) die nützlichen Bakterien und die pathogenen Bakterien gehören zur selben Gattung;

(iv) die pathogenen Bakterien umfassen *C. perfringens,* und die nützlichen Bakterien umfassen *C. hiranonis.*

**11.** Haustierfutterzusammensetzung nach einem der Ansprüche 1 bis 6 zur Verwendung in einem Verfahren zur Verringerung des Gehalts an mikrobiellen Antibiotikaresistenzgenen (ABr) in einem Darmmikrobiom eines Heimtieres; wahlweise, wobei die ABr durch eines oder mehrere der folgenden Merkmale gekennzeichnet sind:

(i) die ABr umfassen eines oder mehrere der folgenden Merkmale: Makrolid mefA Membranfusionsprotein emrA, Makrolid m 2021 Branch, Sensorprotein phoQ, Sensorprotein evgS, Involved-in-Polymyxin-Resistenz pmrB, Bacitracin-Resistenz bacA, Polymyxin-und-kationischeantimikrobielle-Peptide arnA, Sensorkinase cpxA, Makrolid InuA, Makrolid mel, Sensorkinase baeS, Involved-in-Polymyxin-Resistenz pmrC, Membranfusionsprotein emrK, Regulator cpxR, Membranfusionsprotein acre, Innenmembran-Transporter acrF, Beta-Lactam pbp2 Ecoli, oder Kombinationen davon;

(ii) das Abr enthält nicht eines oder mehrere der folgenden Elemente: Aminoglycosid aph2 Ib, Aminoglycosid aac6' Im, Aminoglycosid aph3' III, tetW, tet40, oder Kombinationen davon;

(iii) das ABr umfasst tetM.

**12.** Haustierfutterzusammensetzung nach einem der Ansprüche 1 bis 6 zur Verwendung in einem Verfahren zur Verringerung des Gehalts an Multidrug-Resistenz-Effluxpumpengenen in einem Darmmikrobiom eines Heimtieres; wahlweise, wobei die Multidrug-Resistenz-Efflux-Pumpen-Gene eines oder mehrere der Folgenden umfassen: Efflux-Pumpe mdtB, Efflux-Pumpe mdtA, Efflux-Pumpe mdtC, Efflux-Pumpe mdtD, Transporter-für-Efflux-Komplex mdtF, Efflux-Pumpe baeR, Efflux-Pumpe emrR, Efflux-Pumpe mdtN, Repressor-für-mdr-Efflux-Pumpe crp, Efflux-Pumpe mdtO, Efflux-Pumpe acrD, Repressor-für-Efflux-Komplex acrS, oder Kombinationen davon.

**13.** Haustierfutterzusammensetzung nach einem der Ansprüche 1 bis 6 zur Verwendung in einem Verfahren zur Unterstützung oder Verbesserung der Nierengesundheit eines Heimtieres durch Verringerung der zirkulierenden Blutspiegel von Phenolen und Indolen in dem Heimtier;

wobei die Phenole vorzugsweise eines oder mehrere von 3-Methoxycatechinsulfat (2), 3-Methylcatechinsulfat (1), 4-Acetylcatechinsulfat (1), 4-Allylcatechinsulfat, 4-Ethylcatechinsulfat, 4-Hydroxycatechinsulfat, 4-Methylcatechinsulfat, 4-Vinylcatechinsulfat, Catechinsulfat, 4-Acetylphenylsulfat, 4-Aminophenolsulfat (2), 4-Ethylphenylsulfat, 4-Hydroxyphenylacetat, 4-Hydroxyphenylacetylglycin, 4-Hydroxyphenylpyruvat, 4-Methoxyphenolsulfat, 4-Vinylphenolsulfat, oder Kombinationen davon umfassen;

wobei die Indole vorzugsweise eines oder mehrere von 7-Hydroxyindolsulfat, 5-Hydroxyindolsulfat, 5-Hydroxyindolglucuronid, 3-Hydroxyindolin-2-on-Sulfat, Indoxylglucuronid, 3-Formylindol, 6-Hydroxyindolsulfat, Indolin-2-on, 3-Indoxylsulfat, 5-Hydroxyindolacetat, Indolacetylglutamin, Indolacetat, 3-Indoleglyoxylsäure, Methylindol-3-acetat, Indolacrylat, Indolpropionat, Indolacetylglycin, Indollactat, 2-Oxindol-3-acetat, Indolacetylalanin oder Kombinationen davon und vorzugsweise eines oder mehrere von Indoxylglucuronid, 3-Formylindol, 6-Hydroxyindolsulfat, Indolin-2-on, 3-Indoxylsulfat, 5-Hydroxyindolacetat, Indolacetylglutamin, Indolacetat, 3-Indoleglyoxylsäure, Methylindol-3-acetat, Indolacrylat, Indolpropionat, Indolacetylglycin, Indollactat, 2-Oxindol-3-acetat, Indolacetylalanin, oder Kombinationen davon umfassen.

**14.** Haustierfutterzusammensetzung nach einem der Ansprüche 1 bis 6 zur Verwendung in einem Verfahren zur Unterstützung oder Verbesserung der Nierengesundheit bei einem Heimtier durch Verringerung der mikrobiellen Proteolyse im Dickdarm des Heimtieres;

wahlweise, wobei die Haustierfutterzusammensetzung proteinogene Aminosäuren, aus den proteinogenen Aminosäuren gebildete Dipeptide oder Kombinationen davon vermindert;

wobei die aus den proteinogenen Aminosäuren gebildeten Dipeptide vorzugsweise eines oder mehrere von Alanylleucin, Glycylisoleucin, Glycylleucin, Glycylvalin, Isoleucylglycin, Leucylalanin, Leucylglutamin, Leucylglycin, Lysylleucin, Phenylalanylalanin, Phenylalanylglycin, Threonylphenylalanin, Tryptophylglycin, Tyrosylglycin, Valylglutamin, Valylglycin, Valylleucin, oder Kombinationen davon umfassen;

wobei die proteinogenen Aminosäuren vorzugsweise eine oder mehrere von Alanin, Arginin, Asparagin, Aspartat, Cystein, Cystin, Glutamat, Glutamin, Glycin, Histidin, Isoleucin, Leucin, Lysin, Methionin, Phenylalanin, Prolin, Serin, Taurin, Threonin, Tryptophan, Tyrosin, Valin, oder Kombinationen davon umfassen.

**Revendications**

1. Composition alimentaire pour animaux de compagnie, comprenant :

   plus de 33 % en poids de matière grasse ;
   une source de protéines ;
   moins de 7 % en poids de glucides ; et
   plus de 10 % en poids de fibres ;
   dans laquelle (0,9 F + 0,46 P)/(0,1 F + 0,58 P + C) est égal ou supérieur à 1,5, dans laquelle F, P et C sont, respectivement, des % en poids de matière grasse, de protéines et de glucides.

2. Composition alimentaire pour animaux de compagnie selon la revendication 1, dans laquelle la composition alimentaire pour animaux de compagnie est **caractérisée par** une ou plusieurs des caractéristiques suivantes :

   (i) la composition alimentaire pour animaux de compagnie comprend plus de 14 % en poids de fibres, de préférence plus de 16 % en poids de fibres ;
   (ii) la composition alimentaire pour animaux de compagnie comprend moins de 6 % en poids de glucides, de préférence moins de 5 % en poids de glucides, de préférence moins de 3 % en poids de glucides, ou plus préférentiellement moins de 1,5 % en poids de glucides ;
   (iii) la matière grasse est présente en une quantité supérieure à 34 % en poids, supérieure à 35 % en poids, de préférence supérieure à 36 % en poids ;
   (iv) la matière grasse comprend au moins 20 % en poids de triglycérides à chaîne moyenne (MCT), en particulier de 10 % en poids à 35 % en poids de MCT, de 20 % en poids à 35 % en poids de MCT, de 20 % en poids à 30 % en poids de MCT, ou de préférence de 20 % en poids à 25 % en poids de MCT ;
   (v) la composition alimentaire pour animaux de compagnie comprend en outre de 4,5 g à 14 g de protéines par 100 kcal d'énergie métabolisable, de 6 g à 12 g de protéines par 100 kcal d'énergie métabolisable, ou plus préférentiellement de 7 g à 10 g de protéines par 100 kcal d'énergie métabolisable.

3. Composition alimentaire pour animaux de compagnie selon l'une quelconque des revendications précédentes, dans laquelle la composition alimentaire pour animaux de compagnie est **caractérisée par** l'une ou les deux caractéristiques suivantes :

   (i) la composition alimentaire pour animaux de compagnie comprend en outre des cendres en une quantité inférieure à 7 % en poids, de préférence inférieure à 6 % en poids, ou plus préférentiellement inférieure à 3 % en poids ;
   (ii) la composition alimentaire pour animaux de compagnie comprend en outre du phosphore en une quantité de 1 % en poids ou moins, de 0,8 % en poids ou moins, ou de préférence de 0,7 % en poids ou moins.

4. Composition alimentaire pour animaux de compagnie selon la revendication 1, comprenant en outre un mélange optimal de fibres comprenant : de la cellulose, de la lignine, de l'hémicellulose, de la pectine, de l'alginate et de la cellulose modifiée.

5. Composition alimentaire pour animaux de compagnie selon la revendication 1, comprenant en outre :

   de l'eau en une quantité de 50 % en poids à 60 % en poids ;
   dans laquelle (0,9 F + 0,46 P)/(0,1 F + 0,58 P + C) est supérieur à 1,5,
   dans laquelle la composition alimentaire pour animaux de compagnie se présente sous la forme d'un pain humide.

6. Composition alimentaire pour animaux de compagnie selon la revendication 1, dans laquelle la composition alimentaire pour animaux de compagnie est une composition alimentaire pour animaux de compagnie félins, comprenant en outre :

   des protéines en quantité de 6,5 g à 14 g de protéines par 100 kcal d'énergie métabolisable ;

moins de 5 % en poids de glucides ; et

dans laquelle (0,9 F + 0,46 P)/(0,1 F + 0,58 P + C) est supérieur à 1,5.

7. Composition alimentaire pour animaux de compagnie selon l'une quelconque des revendications 1 à 6, pour une utilisation dans une méthode de traitement d'une glycémie élevée, d'une inflammation chronique, d'un stress métabolique ou d'un cancer chez un animal de compagnie ;

éventuellement, dans laquelle l'animal de compagnie est choisi parmi un chien et un félin.

8. Composition alimentaire pour animaux de compagnie selon l'une quelconque des revendications 1 à 6, pour une utilisation dans un procédé pour favoriser ou faciliter le maintien d'un état cétogène chez un animal de compagnie par diminution de la formation d'acides gras à chaîne courte (SCFA) à chaîne impaire chez l'animal de compagnie ;

éventuellement, dans laquelle les SCFA à chaîne impaire comprennent un ou plusieurs éléments parmi le propionate, le valérate ou des combinaisons de ceux-ci.

9. Composition alimentaire pour animaux de compagnie selon l'une quelconque des revendications 1 à 6, pour une utilisation dans un procédé pour diminuer des facteurs de virulence chez un animal de compagnie ;

éventuellement, dans laquelle le procédé est **caractérisé par** l'une ou les deux caractéristiques suivantes :

(i) les facteurs de virulence comprennent un ou plusieurs parmi : GI 48190 de *Enterococcus faecalis*, GENE nagK de *Clostridium perfringens*, GENE nagH de *Clostridium perfringens*, GENE stbA de *Escherichia coli*, GENE cloSI de *Clostridium perfringens*, GENE lacY de *Escherichia coli*, GENE nagJ de *Clostridium perfringens*, GENE pfoA de *Clostridium perfringens*, *GENE* nagI de *Clostridium perfringens*, GENE nagL de *Clostridium perfringens*, GENE nanI de *Clostridium perfringens*, GENE nanH de *Clostridium perfringens*, GENE nanJ de *Clostridium perfringens* , GENE ompA de *Escherichia coli*, GENE pic de *Clostridium perfringens*, GENE msrD de *Streptococcus pyogenes*, GI 21886745 de *Enterococcus faecium*, GI 21886747 de *Enterococcus faecium*, GENE colA de *Clostridium perfringens*ou des combinaisons de ceux-ci ;

(ii) la diminution des facteurs de virulence prévient ou inhibe les infections chez l'animal de compagnie.

10. Composition alimentaire pour animaux de compagnie selon l'une quelconque des revendications 1 à 6, pour une utilisation dans un procédé pour inhiber la croissance de bactéries pathogènes et favoriser la croissance de bactéries bénéfiques dans l'intestin d'un animal de compagnie ;

éventuellement, dans laquelle le procédé est **caractérisé par** une ou plusieurs des caractéristiques suivantes :

(i) les bactéries bénéfiques comprennent une ou plusieurs bactéries parmi *Clostridium hiranonis, Faecalibacterium prausnitzii,* une bactérie productrice de butyrate, *Butyricicoccus pullicaecorum* ou des combinaisons de celles-ci ;

(ii) les bactéries pathogènes comprennent une ou plusieurs bactéries parmi *Clostridium perfringens, Paeniclostridium sordellii, Clostridium baratii* ou des combinaisons de celles-ci ;

(iii) les bactéries bénéfiques et les bactéries pathogènes appartiennent au même genre ;

(iv) les bactéries pathogènes comprennent *C. perfringens,* et les bactéries bénéfiques comprennent *C. hiranonis.*

11. Composition alimentaire pour animaux de compagnie selon l'une quelconque des revendications 1 à 6, pour une utilisation dans un procédé pour réduire les niveaux de gènes microbiens de résistance aux antibiotiques (ABr) dans le microbiome intestinal d'un animal de compagnie ;

éventuellement, dans laquelle les ABr sont **caractérisés par** une ou plusieurs des caractéristiques suivantes :

(i) les ABr comprennent un ou plusieurs parmi mefA pour macrolides emrA protéine de fusion membranaire, m 2021 Branch pour macrolides, phoQ protéine de détection, evgS protéine de détection, pmrB impliqué dans la résistance à la polymyxine, bacA résistance à la Bacitracine, arnA pour peptides antimicrobiens cationiques et polymyxine, cpxA kinase de détection, lnuA pour macrolides, mel pour macrolides, baeS kinase de détection, pmrC impliqué dans la résistance à la polymyxine, emrK protéine de fusion membranaire, cpxR régulateur, acre protéine de fusion membranaire, acrF transporteur membranaire interne, pbp2 de E. coli pour bêta-lactamines ou des combinaisons de ceux-ci ;

(ii) l'Abr ne comprend pas un ou plusieurs parmi aph2 Ib pour aminoglycosides, aac6' Im pour aminoglycosides, aph3' III pour aminoglycosides, tetW, tet40 ou des combinaisons de ceux-ci ;

(iii) l'ABr comprend tetM.

12. Composition alimentaire pour animaux de compagnie selon l'une quelconque des revendications 1 à 6, pour une utilisation dans un procédé pour réduire les niveaux de gènes de pompe d'efflux de multirésistance aux médicaments dans le microbiome intestinal d'un animal de compagnie ;

éventuellement, dans laquelle les gènes de pompe d'efflux de multirésistance aux médicaments comprennent un ou plusieurs parmi la pompe d'efflux mdtB, la pompe d'efflux mdtA, la pompe d'efflux mdtC, la pompe d'efflux mdtD, le transporteur pour complexe d'efflux mdtF, la pompe d'efflux baeR, la pompe d'efflux emrR, la pompe d'efflux mdtN, le répresseur pour pompe d'efflux mdr crp, la pompe d'efflux mdtO, la pompe d'efflux acrD, le répresseur de complexe d'efflux acrS ou des combinaisons de ceux-ci.

13. Composition alimentaire pour animaux de compagnie selon l'une quelconque des revendications 1 à 6, pour une utilisation dans un procédé pour favoriser ou améliorer la santé rénale d'un animal de compagnie par réduction des niveaux circulants de phénols et d'indoles dans le sang chez l'animal de compagnie ;

dans laquelle les phénols comprennent de préférence un ou plusieurs parmi le sulfate de 3-méthoxycatéchol (2), le sulfate de 3-méthylcatéchol (1), le sulfate de 4-acétylcatéchol (1), le sulfate de 4-allylcatéchol, le sulfate de 4-éthylcatéchol, le sulfate de 4-hydroxycatéchol, le sulfate de 4-méthylcatéchol, le sulfate de 4-vinylcatéchol, le sulfate de catéchol, le sulfate de 4-acétylphényle, le sulfate de 4-aminophénol (2), le sulfate de 4-éthylphényle, le 4-hydroxyphénylacétate, la 4-hydroxyphénylacétylglycine, le 4-hydroxyphénylpyruvate, le sulfate de 4-méthoxyphénol, le sulfate de 4-vinylphénol ou des combinaisons de ceux-ci ;
dans laquelle les indoles comprennent de préférence un ou plusieurs parmi le sulfate de 7-hydroxyindole, le sulfate de 5-hydroxyindole, le glucuronide de 5-hydroxyindole, le sulfate de 3-hydroxyindolin-2-one, le glucuronide d'indoxyle, le 3-formylindole, le sulfate de 6-hydroxyindole, l'indolin-2-one, le sulfate de 3-indoxyle, le 5-hydroxyindoleacétate, l'indoleacétylglutamine, l'indoleacétate, l'acide 3-indoleglyoxylique, l'indole-3-acétate de méthyle, l'indoleacrylate, l'indolepropionate, l'indoleacétylglycine, l'indolelactate, le 2-oxindole-3-acétate, l'indoleacétylalanine ou des combinaisons de ceux-ci et comprennent plus préférentiellement, un ou plusieurs parmi le glucuronide d'indoxyle, le 3-formylindole, le sulfate de 6-hydroxyindole, l'indolin-2-one, le sulfate de 3-indoxyle, le 5-hydroxyindoleacétate, l'indoleacétyl-glutamine, l'indoleacétate, l'acide 3-indoleglyoxylique, l'indole-3-acétate de méthyle, l'indoleacrylate, l'indolepropionate, l'indoléacétylglycine, l'indolelactate, le 2-oxindole-3-acétate, l'indoléacétylalanine ou des combinaisons de ceux-ci.

14. Composition alimentaire pour animaux de compagnie selon l'une quelconque des revendications 1 à 6, pour une utilisation dans un procédé pour favoriser ou améliorer la santé rénale chez un animal de compagnie par réduction de la protéolyse microbienne dans le côlon de l'animal de compagnie ;

éventuellement, dans laquelle la composition alimentaire pour animaux de compagnie diminue les acides aminés protéinogènes, les dipeptides formés à partir des acides aminés protéinogènes ou des combinaisons de ceux-ci ;
dans laquelle les dipeptides formés à partir des acides aminés protéinogènes comprennent de préférence un ou plusieurs parmi l'alanylleucine, la glycylisoleucine, la glycylleucine, la glycylvaline, l'isoleucylglycine, la leucylalanine, la leucylglutamine, la leucylglycine, la lysylleucine, la phénylalanylalanine, la phénylalanylglycine, la thréonylphénylalanine, la tryptophylglycine, la tyrosylglycine, la valylglutamine, la valylglycine, la valylleucine ou des combinaisons de ceux-ci ;
dans laquelle les acides aminés protéinogènes comprennent de préférence un ou plusieurs parmi l'alanine, l'arginine, l'asparagine, l'aspartate, la cystéine, la cystine, le glutamate, la glutamine, la glycine, l'histidine, l'isoleucine, la leucine, la lysine, la méthionine, la phénylalanine, la proline, la sérine, la taurine, la thréonine, le tryptophane, la tyrosine, la valine ou des combinaisons de ceux-ci.

**REFERENCES CITED IN THE DESCRIPTION**

**Patent documents cited in the description**

- US 20110081443 A1
- US 20190134132
- US 20180214410, Hagihara
- US 20190373917 A1
- US 20100292330 A1
- US 2020375934 A1

**Non-patent literature cited in the description**

- **R. T. WOODYATT.** *Arch Intern Med (Chic).,* 1921, vol. 28 (2), 125-141
- The Gut Microbiota Mediates the Anti-Seizure Effects of the Ketogenic Diet. *Cell,* 14 June 2018, vol. 173 (7), 1728-1741
- **M. COHEN ; R. G. H. MORGAN ; A. F. HOFMANN.** *Gastroenterology,* 1971, vol. 60 (1), 1-15
- **A. BACH et al.** *J. Nutr.,* 1977, vol. 107 (10), 1863-1870
- **S. A. RIAL et al.** *Nutrients,* 2016, vol. 8 (5), 281
- **R. BELLINATI-PIRES.** *J. Leukoc. Biol.,* 1993, vol. 53 (4), 404-410
- **S. I. LEE ; K. S. KANG.** *Scientific Reports,* 2017, vol. 7, 16530
- **LATHAN A. CRANDALL, JR.** A Comparison Of Ketosis In Man And Dog. *J. Biol. Chem.,* 1941, vol. 138, 123-128
- *Association of American Feed Control Officials: Official Publication,* 2006, 160-165